# EUROPÄISCHE PATENTSCHRIFT

(11) **EP 3 197 889 B1**
(45) Veröffentlichungstag und Bekanntmachung des Hinweises auf die Patenterteilung: **01.08.2018**
(21) Anmeldenummer: 15766508.4
(22) Anmeldetag: 22.09.2015
(51) Int. Cl.: C07D 413/14, C07D 409/12, C07D 409/14, A61K 31/4436, A61P 1/00, A61P 27/02, A61P 29/00

(54) **SUBSTITUIERTE OXOPYRIDIN-DERIVATE**
SUBSTITUTED OXOPYRIDINE DERIVATIVES
DÉRIVÉS D'OXOPYRIDINE SUBSTITUÉS

(30) Priorität: 24.09.2014 EP 14186086
(43) Veröffentlichungstag der Anmeldung: 02.08.2017
(73) Patentinhaber: Bayer Pharma Aktiengesellschaft, 13353 Berlin (DE)
(72) Erfinder: RÖHRIG, Susanne, 40724 Hilden (DE); JIMENEZ NUNEZ, Eloisa, 42117 Wuppertal (DE); SCHLEMMER, Karl-Heinz, 42113 Wuppertal (DE); TERSTEEGEN, Adrian, 42111 Wuppertal (DE); TELLER, Henrik, 18258 Schwaan (DE); HILLISCH, Alexander, 42651 Solingen (DE); HEITMEIER, Stefan, 42489 Wülfrath (DE); SCHMIDT, Martina Victoria, 51063 Köln (DE); STAMPFUß, Jan, 40470 Düsseldorf (DE)
(74) Vertreter: BIP Patents
(86) Internationale Anmeldenummer: PCT/EP2015/071643
(87) Internationale Veröffentlichungsnummer: WO 2016/046156

(56) Entgegenhaltungen:
- WO-A2-02/42273
- GB-A- 2 497 806

## Beschreibung

Die Erfindung betrifft substituierte Oxopyridin-Derivate und Verfahren zu ihrer Herstellung sowie ihre Verwendung zur Herstellung von Arzneimitteln zur Behandlung und/oder Prophylaxe von Krankheiten, insbesondere von Herz-Kreislauf-Erkrankungen, vorzugsweise von thrombotischen beziehungsweise thromboembolischen Erkrankungen sowie von Ödemen, als auch von ophthalmologischen Erkrankungen.

Die Blutgerinnung ist ein Schutzmechanismus des Organismus, mit dessen Hilfe Defekte in der Gefäßwand rasch und zuverlässig "abgedichtet" werden können. So kann ein Blutverlust vermieden beziehungsweise minimiert werden. Die Blutstillung nach Gefäßverletzung erfolgt im Wesentlichen durch das Gerinnungssystem, bei dem eine enzymatische Kaskade komplexer Reaktionen von Plasmaproteinen ausgelöst wird. Hierbei sind zahlreiche Blutgerinnungsfaktoren beteiligt, von denen jeder, sobald aktiviert, die jeweils nächste inaktive Vorstufe in ihre aktive Form überführt. Am Ende der Kaskade steht die Umwandlung des löslichen Fibrinogens in das unlösliche Fibrin, so dass es zu einem Blutgerinnsel kommt. Traditionell unterscheidet man bei der Blutgerinnung zwischen dem intrinsischen und dem extrinsischen System, die in einem abschließenden gemeinsamen Reaktionsweg münden. Hierbei kommen den Faktoren Xa und IIa (Thrombin) Schlüsselrollen zu: Faktor Xa bündelt die Signale der beiden Gerinnungswege, da er sowohl über Faktor VIIa/Tissue Factor (extrinsischer Weg) wie auch den Tenase Komplex (intrisischer Weg) durch Umsetzung von Faktor X entsteht. Die aktivierte Serinprotease Xa spaltet Prothrombin zu Thrombin, das über eine Reihe von Umsetzungen die Impulse aus der Kaskade auf den Gerinnungsstatus des Blutes überträgt.

In der jüngeren Vergangenheit ist die traditionelle Theorie der zwei getrennten Bereiche der Koagulationskaskade (extrinsischer beziehungsweise intrinsischer Pfad) aufgrund neuer Erkenntnisse modifiziert worden: In diesen Modellen wird die Koagulation durch Bindung von aktiviertem Faktor VIIa an Tissue Faktor (TF) initiiert. Der entstandene Komplex aktiviert Faktor X, was wiederum zur Thrombin-Generierung mit anschließender Herstellung von Fibrin und Thrombozyten-Aktivierung (via PAR-1) als verletzungsverschließende Endprodukte der Hämostase führt. Im Vergleich zur anschließenden Amplifikations-/Propagationsphase ist die Geschwindigkeit der Thrombinherstellung in dieser ersten Phase klein und durch das Auftreten von TFPI als Hemmer des TF-FVIIa-FX-Komplexes zeitlich begrenzt.

Ein zentraler Bestandteil des Übergangs von der Initiation zur Amplifikation und Propagation der Koagulation ist Faktor XIa: Thrombin aktiviert in positiven Rückkopplungsschleifen neben Faktor V und Faktor VIII auch Faktor XI zu Faktor XIa, der Faktor IX zu Faktor IXa umsetzt und über den so generierten Faktor IXa/Faktor VIIIa-Komplex die Faktor X-Aktivierung und damit wiederum die Thrombinbildung stark stimuliert, was zu starkem Thrombuswachstum führt und den Thrombus stabilisiert.

Darüber hinaus ist in den Blickpunkt gerückt, dass neben der Stimulation über Tissue Faktor die Aktivierung des Gerinnungssystems an insbesondere negativ geladenen Oberflächen erfolgen kann, zu denen neben Oberflächenstrukturen körperfremder Zellen (z.B. Bakterien) auch artifizielle Oberflächen wie Gefäßprothesen, Stents und extrakorporale Kreisläufe gehören. Auf der Oberfläche findet zunächst die Aktivierung von Faktor XII (FXII) zu Faktor Xlla statt, der im Folgenden an Zelloberflächen gebundenen Faktor XI zu Faktor XIa aktiviert. Dieser führt wie zuvor beschrieben zur weiteren Aktivierung der Gerinnungskaskade. Daneben aktiviert Faktor XIIa ebenfalls gebundenes Plasmaprokallikrein zu Plasmakallikrein (PK), das zum einen zu weiterer Faktor XII-Aktivierung im Rahmen einer Potentierungsschleife führt, was insgesamt eine Verstärkung der Initiation der Gerinnungskaskade zur Folge hat. Zusätzlich stellt PK eine wichtige Bradikinin-freisetzende Protease dar, die somit unter anderem zum Anstieg der endothelialen Permeabilität führt. Als weitere Substrate wurden Prorenin und Prourokinase beschrieben, deren Aktivierung die regulatorischen Prozesse des Renin-Angiotensin-Systems und der Fibrinolyse beeinflussen kann. Damit stellt die Aktivierung von PK ein wichtiges Bindeglied zwischen koagulativen und inflammatorischen Prozessen dar.

Eine unkontrollierte Aktivierung des Gerinnungssystems oder eine defekte Hemmung der Aktivierungsprozesse kann die Bildung von lokalen Thrombosen oder Embolien in Gefäßen (Arterien, Venen, Lymphgefäßen) oder Herzhöhlen bewirken. Darüber hinaus kann eine systemische Hyperkoagulabilität zur systemweiten Bildung von Thromben und schließlich zu einer Verbrauchskoagulopathie im Rahmen einer disseminierten intravasalen Gerinnung führen. Thromboembolische Komplikationen können ferner in extrakorporalen Blutkreisläufen, wie z. B. während einer Hämodialyse, sowie in Gefäß- beziehungsweise Herzklappenprothesen und Stents auftreten.

Im Verlauf vieler Herzkreislauf- und Stoffwechselerkrankungen kommt es infolge systemischer Faktoren, wie z.B. Hyperlipidämie, Diabetes oder Rauchen, infolge von Blutflußveränderungen mit Stase, wie z.B. beim Vorhofflimmern, oder infolge pathologischer Gefäßwandveränderungen, z.B. endothelialer Dysfunktionen oder Atherosklerose, zu einer erhöhten Neigung von Gerinnungs- und Thrombozytenaktivierung. Diese unerwünschte und überschießende Aktivierung der Gerinnung kann durch Bildung fibrin- und plättchenreicher Thromben zu thromboembolischen Erkrankungen und thrombotischen Komplikationen mit lebensbedrohlichen Zuständen führen. Hierbei können auch entzündliche Prozesse involviert sein. Thromboembolische Erkrankungen gehören daher nach wie vor zu den häufigsten Ursachen von Morbidität und Mortalität in den meisten industrialisierten Ländern.

Die aus dem Stand der Technik bekannten Antikoagulantien, d.h. Stoffe zur Hemmung oder Verhinderung der Blutgerinnung, weisen verschiedene, Nachteile auf. Eine effiziente Behandlungsmethode beziehungsweise Prophylaxe von thrombotischen/thromboembolischen Erkrankungen erweist sich in der Praxis deshalb als sehr schwierig und unbefriedigend.

In der Therapie und Prophylaxe von thromboembolischen Erkrankungen findet zum einen Heparin Verwendung, das parenteral oder subkutan appliziert wird. Aufgrund günstigerer pharmakokinetischer Eigenschaften wird zwar heutzutage zunehmend niedermolekulares Heparin bevorzugt; allerdings können auch hierdurch die im Folgenden geschilderten bekannten Nachteile nicht vermieden werden, die bei der Therapierung mit Heparin bestehen. So ist Heparin oral unwirksam und besitzt nur eine vergleichsweise geringe Halbwertszeit. Darüber hinaus besteht ein hohes Blutungsrisiko, insbesondere können Hirnblutungen und Blutungen im Gastrointestinaltrakt auftreten, und es kann zu Thrombopenie, Alopecia medicomentosa oder Osteoporose kommen. Niedermolekulare Heparine besitzen zwar eine geringere Wahrscheinlichkeit zur Ausbildung einer Heparin-induzierten Thrombocytopenie, sind aber auch nur subkutan applizierbar. Dies gilt auch für Fondaparinux, einem synthetisch hergestellten, selektiven Faktor Xa Inhibitor mit einer langen Halbwertszeit.

Eine zweite Klasse von Antikoagulantien stellen die Vitamin K-Antagonisten dar. Hierzu gehören beispielsweise 1,3-Indandione, vor allem aber Verbindungen wie Warfarin, Phenprocoumon, Dicumarol und andere Cumarin-Derivate, die unselektiv die Synthese verschiedener Produkte bestimmter Vitamin K-abhängiger Gerinnungsfaktoren in der Leber hemmen. Durch den Wirkmechanismus bedingt, setzt die Wirkung nur sehr langsam ein (Latenzzeit bis zum Wirkeintritt 36 bis 48 Stunden). Die Verbindungen können zwar oral appliziert werden, aufgrund des hohen Blutungsrisikos und des engen therapeutischen Indexes ist aber eine aufwendige individuelle Einstellung und Beobachtung des Patienten notwendig. Darüber hinaus sind weitere Nebenwirkungen wie gastrointestinale Störungen, Haarausfall und Hautnekrosen beschrieben.

Neuere Ansätze für orale Antikoagulantien befinden sich in verschiedenen Phasen der klinischen Erprobung beziehungsweise im klinischen Einsatz und haben ihre Wirksamkeit in verschiedenen Studien unter Beweis gestellt. Allerdings kann es auch unter der Einnahme dieser Arzneimittel insbesondere bei prädisponierten Patienten zu Blutungskomplikationen kommen. Daher ist bei antithrombotischen Arzneimitteln ist die therapeutische Breite von zentraler Bedeutung: Der Abstand zwischen der therapeutisch wirksamen Dosis zur Gerinnungshemmung und der Dosis, bei der Blutungen auftreten können, sollte möglichst groß sein, so dass eine maximale therapeutische Wirksamkeit bei minimalem Risikoprofil erreicht wird.

In verschiedenen in-vitro und in-vivo Modellen mit beispielsweise Antikörpern als Faktor XIa Inhibitoren, aber auch in Faktor XIa-Knock-out-Modellen, wurde der anti-thrombotische Effekt bei geringer/keiner Verlängerung der Blutungszeit oder Vergrößerung des Blutvolumens belegt. In klinischen Studien waren erhöhte Faktor XIa-Spiegel mit einer gesteigerten Ereignisrate assoziiert. Dagegen führte Faktor XI-Defizienz (Hämophilie C) nicht zu spontanen Blutungen und fiel nur im Rahmen von Operationen und Traumen auf, zeigte aber eine Protektion gegenüber bestimmten thromboembolischen Ereignissen.

Daneben ist Plasmakallikrein (PK) mit weiteren Erkrankungen assoziiert, die mit erhöhten Gefäßpermeabilitäten oder chronisch-entzündlichen Erkrankungen einhergehen, wie es z.B. bei der diabetischen Retinopathie, dem Makulaödem und dem hereditären Angioödem beziehungsweise chronisch-entzündlichen Darmerkrankungen der Fall ist. Der diabetischen Retinopathie liegt in erster Linie eine Mikrogefäßschwäche zu Grunde, in deren Folge es zu einer Basalmembranverdickung der Gefäße und zum Verlust von gefäßummantelnden Perizyten, später zum Gefäßverschluss mit retinaler Ischämie kommt, welche aufgrund der hervorgerufenen retinalen Hypoxie zu verstärkter Gefäßpermeabilität mit nachfolgender Ausbildung eines Makulaödems und aufgrund aller vorliegender Prozesse zur Erblindung des Patienten führen kann. Beim Hereditären Angioödem (HAE) kommt es durch verminderte Bildung des physiologischen Kallikrein-Inhibitors C1-Esterase-Inhibitors zur unkontrollierten Plasmakallikrein-Aktivierung und damit zu Entzündungen mit fulminanter Ödembildung und starken Schmerzen. Aus tierexperimentellen Ansätzen gibt es Hinweise, dass die Inhibition von Plasmakallikrein die erhöhte Gefäßpermeabilität inhibiert und somit die Ausbildung eines Makulaödems beziehungsweise der diabetischen Retinopathie verhindern beziehungsweise die akute Symptomatik des HAE verbessern kann. Orale Plasmakallikrein-Inhibitoren könnten ebenfalls zur Prophylaxe des HAE eingesetzt werden.

Bei der Progression von chronisch-entzündlichen Darmerkrankungen (CED) haben vor allem die mittels Plasmakallikrein generierten Kinine eine tragende Rolle. Deren pro-inflammatorische Wirkung über Aktivierung von Bradykinin-Rezeptoren induziert und potenziert den Krankheitsverlauf. Studien an Morbus Crohn-Patienten zeigen eine Korrelation zwischen der Kallikrein-Konzentration im Darmepithel und dem Grad der Darmentzündung. Eine Aktivierung des Kallikrein-Kinin-Systems wurde ebenfalls in tierexperimentellen Studien beobachtet. Eine Hemmung der Bradykinin-Synthese durch Kallikrein-Inhibitoren könnte demnach auch zur Prophylaxe und/oder Therapie von chronisch-entzündlichen Darmerkrankungen eingesetzt werden.

Weiterhin kann auch die Kombination von antithrombotischen und antiinflammtorischen Prinzipien für viele Erkrankungen besonders attraktiv sein, um die wechselseitige Verstärkung von Koagulation und Inflammation zu unterbinden.

Eine Aufgabe der vorliegenden Erfindung ist daher die Bereitstellung neuer Verbindungen zur Behandlung von Herz-Kreislauf-Erkrankungen, insbesondere von thrombotischen beziehungsweise thromboembolischen Erkrankungen, und/oder ödematösen Erkrankungen, und/oder ophthalmologischen Erkrankungen, insbesondere von diabetischer Retinopathie beziehungsweise des Makulaödems, bei Menschen und Tieren, die eine große therapeutische Bandbreite aufweisen.

WO 2006/030032 beschreibt unter anderem substituierte Pyridinone als allosterische Modulatoren des mGluR2 Rezeptors und WO 2008/079787 beschreibt substituierte Pyridin-2-one und ihre Verwendung als Glucokinase Aktivatoren. WO 2014/154794, WO 2014/160592, WO 2015/011087 und WO 2015/063093 beschreiben substituierte Pyridin-2-one und ihre Verwendung als Faktor XIa Inhibitoren.

Gegenstand der Erfindung sind Verbindungen der Formel 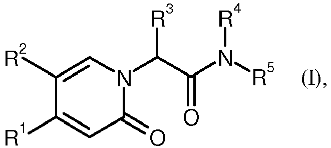 in welcher
- R¹: für eine Gruppe der Formel 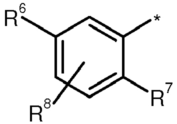 steht,
wobei * die Anknüpfstelle an den Oxopyridinring ist,
R⁶ für Brom, Chlor, Fluor, Methyl, Difluormethyl, Trifluormethyl, Methoxy, Difluormethoxy oder Trifluormethoxy steht,
R⁷ für Brom, Chlor, Fluor, Cyano, Nitro, Hydroxy, Methyl, Difluormethyl, Trifluormethyl, Methoxy, Ethoxy, Difluormethoxy, Trifluormethoxy, Ethinyl, 3,3,3-Trifluorprop-1-in-1-yl oder Cyclopropyl steht,
R⁸ für Wasserstoff, Chlor oder Fluor steht,
- R²: für Wasserstoff, Brom, Chlor, Fluor, Cyano, C₁-C₃-Alkyl, Difluormethyl, Trifluormethyl, 1,1-Difluorethyl, 2,2-Difluorethyl, 2,2,2-Trifluorethyl, C₁-C₃-Alkoxy, Difluormethoxy, Trifluormethoxy, 1,1-Difluorethoxy, 2,2-Difluorethoxy, 2,2,2-Trifluorethoxy, Methylcarbonyl oder Cyclopropyl steht,
- R³: für Wasserstoff, C₁-C₅-Alkyl, C₁-C₄-Alkoxy, Difluormethyl, Trifluormethyl, 1,1-Difluorethyl, 1,1,2,2,2-Pentadeuteroethyl, 3,3,3-Trifluor-2-hydroxyprop-1-yl, 3,3,3-Trifluor-2-methoxyprop-1-yl, 3,3,3-Trifluor-2-ethoxyprop-1-yl, Prop-2-in-1-yl, Cyclopropyloxy oder Cyclobutyloxy steht,
wobei Alkyl substituiert sein kann mit einem Substituenten ausgewählt aus der Gruppe bestehend aus Fluor, Cyano, Hydroxy, Difluormethyl, Trifluormethyl, Methoxy, Ethoxy, Difluormethoxy, Trifluormethoxy, C₃-C₆-Cycloalkyl, 4- bis 6-gliedriges Oxo-Heterocyclyl, 1,4-Dioxanyl, Oxazolyl, Pyrazolyl, Phenyl und Pyridyl,
worin Cycloalkyl substituiert sein kann mit 1 bis 2 Substituenten unabhängig voneinander ausgewählt aus der Gruppe bestehend aus Fluor, Hydroxy, Methyl, Ethyl, Methoxy, Ethoxy, Difluormethyl, Trifluormethyl, Difluormethoxy und Trifluormethoxy,
   und
worin Oxo-Heterocyclyl substituiert sein kann mit 1 bis 2 Substituenten unabhängig voneinander ausgewählt aus der Gruppe bestehend aus Oxo, Fluor, Methyl, Ethyl, Difluormethyl und Trifluormethyl,
   und
worin Oxazolyl und Pyrazolyl substituiert sein können mit 1 bis 2 Substituenten unabhängig voneinander ausgewählt aus der Gruppe bestehend aus Methyl und Ethyl,
- R⁴: für Wasserstoff steht,
- R⁵: für eine Gruppe der Formel 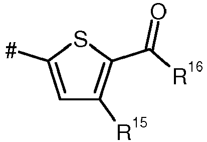 steht,
wobei # die Anknüpfstelle an das Stickstoffatom ist,
R¹⁵ für Wasserstoff oder Fluor steht,
R¹⁶ für Hydroxy oder -NHR¹⁷ steht,
worin
R¹⁷ für Wasserstoff oder C₁₋C₄-Alkyl steht,
und ihre Salze, ihre Solvate und die Solvate ihrer Salze.

Erfindungsgemäße Verbindungen sind die Verbindungen der Formel (I) und deren Salze, Solvate und Solvate der Salze, sowie die von Formel (I) umfassten, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler beziehungsweise chiraler Phase.

Sofern die erfindungsgemäßen Verbindungen in tautomeren Formen vorkommen können, umfasst die vorliegenden Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagenzien und/oder Ausgangsverbindungen eingesetzt werden.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin, N-Methylpiperidin und Cholin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Außerdem offenbart die vorliegende Erfindung auch Prodrugs der erfindungsgemäßen Verbindungen.

Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Im Rahmen der vorliegenden Erfindung haben die Substituenten, soweit nicht anders spezifiziert, die folgende Bedeutung:
Alkyl steht für einen linearen oder verzweigten Alkylrest mit 1 bis 5 Kohlenstoffatomen, bevorzugt 1 bis 4 Kohlenstoffatomen, besonders bevorzugt 1 bis 3 Kohlenstoffatomen, beispielhaft und vorzugsweise für Methyl, Ethyl, n-Propyl, iso-Propyl, 2-Methyl-prop-1-yl, n-Butyl, *tert*.-Butyl und 2,2-Dimethylprop-1-yl.

Alkoxy steht für einen linearen oder verzweigten Alkoxyrest mit 1 bis 4 Kohlenstoffatomen, bevorzugt 1 bis 3 Kohlenstoffatomen, beispielhaft und vorzugsweise für Methoxy, Ethoxy, n-Propoxy, iso-Propoxy, 2-Methyl-prop-1-oxy, n-Butoxy und *tert*.-Butoxy.

Cycloalkyl steht für eine monocyclische Cycloalkylgruppe mit 3 bis 6 Kohlenstoffatomen, beispielhaft und vorzugsweise für Cycloalkyl seien genannt Cyclopropyl, Cyclobutyl, Cyclopentyl und Cyclohexyl.

4- bis 6-gliedriges Oxo-Heterocyclyl in der Definition des Restes R³ steht für einen gesättigten monocyclischen Rest mit 4 bis 6 Ringatomen, in dem ein Ringatom ein Sauerstoffatom ist, beispielhaft und vorzugsweise für Oxetanyl, Tetrahydrofuranyl und Tetrahydro-2H-pyranyl.

4- bis 6-gliedriges Thio-Heterocyclyl in der Definition des Restes R³ steht für einen gesättigten monocyclischen Rest mit 4 bis 6 Ringatomen, in dem ein Ringatom ein Schwefelatom ist, beispielhaft und vorzugsweise für Thientanyl, Tetrahydrothienyl und Tetrahydro-2H-thiopyranyl.

In den Formeln der Gruppe, die für R¹ stehen kann, steht der Endpunkt der Linie, neben der jeweils ein * steht, nicht für ein Kohlenstoffatom beziehungsweise eine CH₂-Gruppe sondern ist Bestandteil der Bindung zu dem Atom, an das R¹ gebunden ist.

In den Formeln der Gruppe, die für R⁵ stehen kann, steht der Endpunkt der Linie, neben der jeweils ein # steht, nicht für ein Kohlenstoffatom beziehungsweise eine CH₂-Gruppe sondern ist Bestandteil der Bindung zu dem Atom, an das R⁵ gebunden ist.

Bevorzugt sind Verbindungen der Formel (I), in welcher
- R¹: für eine Gruppe der Formel 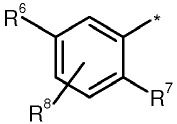 steht,
wobei * die Anknüpfstelle an den Oxopyridinring ist,
R⁶ für Chlor steht,
R⁷ für Fluor, Cyano, Difluormethyl oder Difluormethoxy steht,
R⁸ für Wasserstoff steht,
- R²: für Chlor, Cyano, Methoxy oder Difluormethoxy steht,
- R³: für Methyl, Ethyl, n-Propyl oder n-Butyl steht,
wobei Methyl substituiert sein kann mit einem Substituenten ausgewählt aus der Gruppe bestehend aus Cyclopropyl, Cyclobutyl, Cyclohexyl, Tetrahydro-2H-pyranyl, Oxazolyl, Pyrazolyl und Pyridyl,
worin Cyclobutyl und Cyclohexyl substituiert sein können mit 1 bis 2 Substituenten unabhängig voneinander ausgewählt aus der Gruppe bestehend aus Hydroxy und Methoxy,
   und
worin Oxazolyl und Pyrazolyl substituiert sein können mit einem Substituenten Methyl,
und
wobei Ethyl, n-Propyl und n-Butyl substituiert sein können mit einem Substituenten ausgewählt aus der Gruppe bestehend aus Methoxy und Trifluormethoxy,
- R⁴: für Wasserstoff steht,
- R⁵: für eine Gruppe der Formel 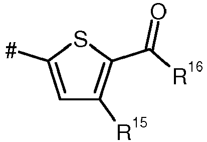 steht,
wobei # die Anknüpfstelle an das Stickstoffatom ist,
R¹⁵ für Wasserstoff oder Fluor steht,
R¹⁶ für Hydroxy oder -NHR¹⁷ steht,
worin
R¹⁷ für Wasserstoff, Methyl oder Ethyl steht,
und ihre Salze, ihre Solvate und die Solvate ihrer Salze.

Bevorzugt sind auch Verbindungen der Formel (I), in welcher
- R¹: für eine Gruppe der Formel 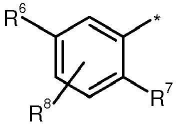 steht,
wobei * die Anknüpfstelle an den Oxopyridinring ist,
R⁶ für Chlor steht,
R⁷ für Fluor oder Cyano steht,
R⁸ für Wasserstoff steht,
- R²: für Chlor, Methoxy oder Difluormethoxy steht,
- R³: für Methyl oder Ethyl steht,
wobei Methyl substituiert sein kann mit einem Substituenten ausgewählt aus der Gruppe bestehend aus Tetrahydro-2H-pyranyl, Oxazolyl, Pyrazolyl und Pyridyl,
worin Oxazolyl und Pyrazolyl substituiert sein können mit einem Substituenten Methyl,
und
wobei Ethyl substituiert sein kann mit einem Substituenten Methoxy,
- R⁴: für Wasserstoff steht,
- R⁵: für eine Gruppe der Formel 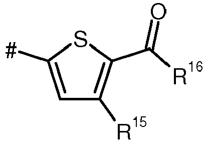 steht,
- wobei: # die Anknüpfstelle an das Stickstoffatom ist,
- R¹⁵: für Wasserstoff oder Fluor steht,
- R¹⁶: für Hydroxy oder -NHR¹⁷ steht, worin
- R¹⁷: für Wasserstoff oder Methyl steht,
und ihre Salze, ihre Solvate und die Solvate ihrer Salze.

Bevorzugt sind auch Verbindungen der Formel (I), in welcher
- R¹: für eine Gruppe der Formel 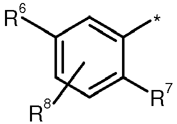 steht,
- wobei: * die Anknüpfstelle an den Oxopyridinring ist,
- R⁶: für Brom, Chlor, Fluor, Methyl, Difluormethyl, Trifluormethyl, Methoxy, Difluormethoxy oder Trifluormethoxy steht,
- R⁷: für Brom, Chlor, Fluor, Cyano, Nitro, Hydroxy, Methyl, Difluormethyl, Trifluormethyl, Methoxy, Ethoxy, Difluormethoxy, Trifluormethoxy, Ethinyl, 3,3,3-Trifluorprop-1-in-1-yl oder Cyclopropyl steht,
- R⁸: für Wasserstoff, Chlor oder Fluor steht,
- R²: für Wasserstoff, Brom, Chlor, Fluor, Cyano, C₁₋C₃-Alkyl, Difluormethyl, Trifluormethyl, 1,1-Difluorethyl, 2,2-Difluorethyl, 2,2,2-Trifluorethyl, C₁-C₃-Alkoxy, Difluormethoxy, Trifluormethoxy, 1,1-Difluorethoxy, 2,2-Difluorethoxy, 2,2,2-Trifluorethoxy, Methylcarbonyl oder Cyclopropyl steht,
- R³: für Wasserstoff, C₁-C₅-Alkyl, C₁-C₄-Alkoxy, Difluormethyl, Trifluormethyl, 1,1-Difluorethyl, 1,1,2,2,2-Pentadeuteroethyl, 3,3,3-Trifluor-2-hydroxyprop-1-yl, 3,3,3-Trifluor-2-methoxyprop-1-yl, 3,3,3-Trifluor-2-ethoxyprop-1-yl, Prop-2-in-1-yl, Cyclopropyloxy oder Cyclobutyloxy steht,
wobei Alkyl substituiert sein kann mit einem Substituenten ausgewählt aus der Gruppe bestehend aus Fluor, Cyano, Hydroxy, Difluormethyl, Trifluormethyl, Methoxy, Ethoxy, Difluormethoxy, Trifluormethoxy, C₃-C₆-Cycloalkyl, 4- bis 6-gliedriges Oxo-Heterocyclyl, 1,4-Dioxanyl, Oxazolyl, Phenyl und Pyridyl,
worin Cycloalkyl substituiert sein kann mit 1 bis 2 Substituenten unabhängig voneinander ausgewählt aus der Gruppe bestehend aus Fluor, Hydroxy, Methyl, Ethyl, Methoxy, Ethoxy, Difluormethyl, Trifluormethyl, Difluormethoxy und Trifluormethoxy,
   und
worin Oxo-Heterocyclyl substituiert sein kann mit 1 bis 2 Substituenten unabhängig voneinander ausgewählt aus der Gruppe bestehend aus Oxo, Fluor, Methyl, Ethyl, Difluormethyl und Trifluormethyl,
   und
worin Oxazolyl substituiert sein kann mit 1 bis 2 Substituenten unabhängig voneinander ausgewählt aus der Gruppe bestehend aus Methyl und Ethyl,
- R⁴: für Wasserstoff steht,
- R⁵: für eine Gruppe der Formel 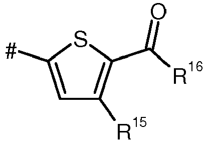 steht,
- wobei: # die Anknüpfstelle an das Stickstoffatom ist,
- R¹⁵: für Wasserstoff oder Fluor steht,
- R¹⁶: für Hydroxy oder -NHR¹⁷ steht,
worin
R¹⁷ für Wasserstoff oder C₁₋C₄-Alkyl steht,
und ihre Salze, ihre Solvate und die Solvate ihrer Salze.

Bevorzugt sind auch Verbindungen der Formel (I), in welcher
- R¹: für eine Gruppe der Formel 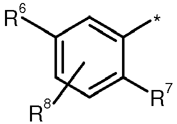 steht,
wobei * die Anknüpfstelle an den Oxopyridinring ist,
R⁶ für Chlor steht,
R⁷ für Fluor, Cyano, Difluormethyl oder Difluormethoxy steht,
R⁸ für Wasserstoff steht,
- R²: für Chlor, Cyano, Methoxy oder Difluormethoxy steht,
- R³: für Methyl, Ethyl, n-Propyl oder n-Butyl steht,
wobei Methyl substituiert sein kann mit einem Substituenten ausgewählt aus der Gruppe bestehend aus Cyclopropyl, Cyclobutyl, Cyclohexyl, Tetrahydro-2H-pyranyl, Oxazolyl und Pyridyl,
worin Cyclobutyl und Cyclohexyl substituiert sein können mit 1 bis 2 Substituenten unabhängig voneinander ausgewählt aus der Gruppe bestehend aus Hydroxy und Methoxy,
   und
worin Oxazolyl substituiert sein kann mit einem Substituenten Methyl,
und
wobei Ethyl, n-Propyl und n-Butyl substituiert sein können mit einem Substituenten ausgewählt aus der Gruppe bestehend aus Methoxy und Trifluormethoxy,
- R⁴: für Wasserstoff steht,
- R⁵: für eine Gruppe der Formel 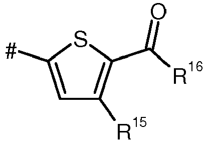 steht,
- wobei: # die Anknüpfstelle an das Stickstoffatom ist,
- R¹⁵: für Wasserstoff oder Fluor steht,
- R¹⁶: für Hydroxy oder -NHR¹⁷ steht,
worin
- R¹⁷: für Wasserstoff, Methyl oder Ethyl steht,
und ihre Salze, ihre Solvate und die Solvate ihrer Salze.

Bevorzugt sind auch Verbindungen der Formel (I), in welcher
- R¹: für eine Gruppe der Formel 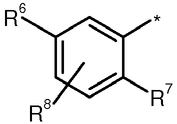 steht,
wobei * die Anknüpfstelle an den Oxopyridinring ist,
R⁶ für Chlor steht,
R⁷ für Fluor oder Cyano steht,
R⁸ für Wasserstoff steht,
- R²: für Chlor, Methoxy oder Difluormethoxy steht,
- R³: für Methyl oder Ethyl steht,
wobei Methyl substituiert sein kann mit einem Substituenten ausgewählt aus der Gruppe bestehend aus Tetrahydro-2H-pyranyl, Oxazolyl und Pyridyl,
worin Oxazolyl substituiert sein kann mit einem Substituenten Methyl,
und
wobei Ethyl substituiert sein kann mit einem Substituenten Methoxy,
- R⁴: für Wasserstoff steht,
- R⁵: für eine Gruppe der Formel 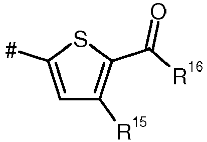 steht,
- wobei: /# die Anknüpfstelle an das Stickstoffatom ist,
- R¹⁵: für Wasserstoff oder Fluor steht,
- R¹⁶: für Hydroxy oder -NHR¹⁷ steht,
worin
- R¹⁷: für Wasserstoff oder Methyl steht,
und ihre Salze, ihre Solvate und die Solvate ihrer Salze.

Bevorzugt sind auch Verbindungen der Formel (I), in welcher
- R¹: für eine Gruppe der Formel 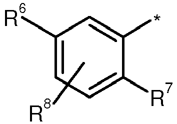 steht,
wobei * die Anknüpfstelle an den Oxopyridinring ist,
R⁶ für Chlor steht,
R⁷ für Cyano steht,
R⁸ für Wasserstoff steht,
- R²: für Chlor oder Methoxy steht,
- R³: für Methyl oder Ethyl steht,
wobei Methyl substituiert ist mit einem Substituenten ausgewählt aus der Gruppe bestehend aus Tetrahydro-2H-pyranyl, Oxazolyl und Pyridyl,
worin Oxazolyl substituiert sein kann mit einem Substituenten Methyl,
und
wobei Ethyl substituiert sein kann mit einem Substituenten Methoxy,
- R⁴: für Wasserstoff steht,
- R⁵: für eine Gruppe der Formel 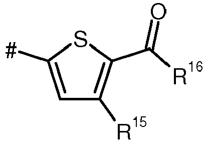 steht,
- wobei: # die Anknüpfstelle an das Stickstoffatom ist,
- R¹⁵: für Wasserstoff oder Fluor steht,
- R¹⁶: für Hydroxy oder -NHR¹⁷ steht,
worin
- R¹⁷: für Wasserstoff oder Methyl steht,
und ihre Salze, ihre Solvate und die Solvate ihrer Salze.

Bevorzugt sind auch Verbindungen der Formel (I), in welcher
- R¹: für eine Gruppe der Formel 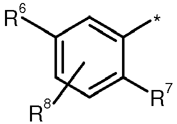 steht,
- wobei: * die Anknüpfstelle an den Oxopyridinring ist,
- R⁶: für Chlor steht,
- R⁷: für Cyano steht,
- R⁸: für Wasserstoff steht.

Bevorzugt sind auch Verbindungen der Formel (I), in welcher R² für Chlor oder Methoxy steht.

Bevorzugt sind auch Verbindungen der Formel (I), in welcher
- R³: für Methyl oder Ethyl steht,
wobei Methyl substituiert ist mit einem Substituenten ausgewählt aus der Gruppe bestehend aus Tetrahydro-2H-pyranyl, Oxazolyl und Pyridyl,
worin Oxazolyl substituiert sein kann mit einem Substituenten Methyl,
und
wobei Ethyl substituiert sein kann mit einem Substituenten Methoxy.

Bevorzugt sind auch Verbindungen der Formel (I), in welcher
- R⁵: für eine Gruppe der Formel 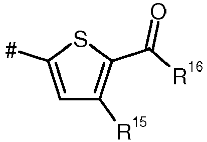 steht,
wobei # die Anknüpfstelle an das Stickstoffatom ist,
R¹⁵ für Wasserstoff oder Fluor steht,
R¹⁶ für Hydroxy oder -NHR¹⁷ steht,
worin
R¹⁷ für Wasserstoff oder Methyl steht.

Bevorzugt sind auch Verbindungen, welche die Formel (Ia) aufweisen 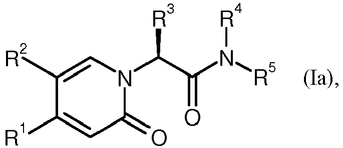 worin R¹, R², R³, R⁴ und R⁵ wie oben definiert sind.

Gegenstand der Erfindung ist weiterhin ein Verfahren zur Herstellung der Verbindungen der Formel (I), oder ihrer Salze, ihrer Solvate oder der Solvate ihrer Salze, wobei
[A] die Verbindungen der Formel 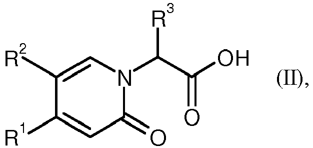 in welcher
   R¹, R² und R³ die oben angegebene Bedeutung haben,
   in der ersten Stufe mit Verbindungen der Formel 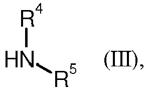 in welcher
   R⁴ und R⁵ die oben angegebene Bedeutung haben,
   in Gegenwart eines Dehydratisierungsreagenzes umgesetzt werden, und
   gegebenfalls in einer zweiten Stufe durch saure oder basische Esterspaltung zu Verbindungen der Formel (I) umgesetzt werden,
   oder
[B] die Verbindungen der Formel 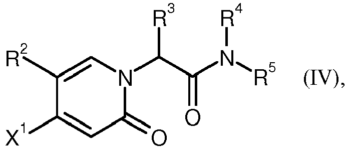 in welcher
   R², R³, R⁴ und R⁵ die oben angegebene Bedeutung haben, und
   X¹ für Chlor, Brom oder Iod steht, mit Verbindungen der Formel 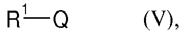 in welcher
   R¹ die oben angegebene Bedeutung hat, und
   Q für -B(OH)₂, einen Boronsäure-Ester, bevorzugt Boronsäurepinakolester, oder -BF₃⁻K⁺ steht,
unter Suzuki-Kupplungsbedingungen zu Verbindungen der Formel (I) umgesetzt werden.

Die Umsetzung der ersten Stufe nach Verfahren [A] erfolgt im Allgemeinen in inerten Lösungsmitteln, gegebenenfalls in Gegenwart einer Base, bevorzugt in einem Temperaturbereich von 0°C bis Raumtemperatur bei Normaldruck.

Als Dehydratisierungsreagenzien eignen sich hierbei beispielsweise Carbodiimide wie z.B. *N,N'-*Diethyl-, *N,N'*-Dipropyl-, *N,N*'-Diisopropyl-, *N,N*'-Dicyclohexylcarbodiimid, *N*-(3-Dimethylamino-isopropyl)-*N'*-ethylcarbodiimid-Hydrochlorid (EDC) (gegebenenfalls in Gegenwart von Pentafluorphenol (PFP)), *N*-Cyclohexylcarbodiimid-*N'*-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder *O*-(Benzotriazol-1-yl)-*N,N,N',N'*-tetra-methyluronium-hexafluorophosphat (HBTU), 2-(2-Oxo-1-(2H)-pyridyl)-1,1,3,3-tetramethyluroniumtetrafluoroborat (TPTU), (Benzotriazol-1-yloxy)bisdimethylamino-methyliumfluoroborat (TBTU) oder *O*-(7-Azabenzotriazol-1-yl)-*N,N,N',N'*-tetramethyl-uronium-hexafluorophosphat (HATU), oder 1-Hydroxybenztriazol (HOBt), oder Benzotriazol-1-yloxy-tris(dimethylamino)-phosphoniumhexafluorophosphat (BOP), oder Ethyl-cyan(hydroxy-imino)acetat (Oxyma), oder (1-Cyano-2-ethoxy-2-oxoethylidenaminooxy)dimethylamino-morpholino-carbenium hexafluorophosphat (COMU), oder N-[(Dimethylamino)(3*H-*[1,2,3]triazolo[4,5-b]pyridin-3-yloxy)methyliden]-N-methylmethanaminium-hexafluorophosphat, oder 2,4,6-Tripropyl-1,3,5,2,4,6-trioxatriphosphinan-2,4,6-trioxid (T3P), oder Mischungen aus diesen, mit Basen. Vorzugsweise wird die Kondensation mit HATU oder mit T3P durchgeführt.

Basen sind beispielsweise Alkalicarbonate, wie z.B. Natrium- oder Kaliumcarbonat, oder -hydrogencarbonat, oder organische Basen wie Trialkylamine, z.B. Triethylamin, *N*-Methylmorpholin, *N*-Methylpiperidin, 4-Dimethylaminopyridin oder Diisopropylethylamin, oder Pyridin. Vorzugsweise wird die Kondensation mit Diisopropylethylamin oder Pyridin durchgeführt.

Inerte Lösungsmittel sind beispielsweise Halogenkohlenwasserstoffe wie Dichlormethan oder Trichlormethan, Kohlenwasserstoffe wie Benzol, oder andere Lösungsmittel wie Nitromethan, Dioxan, Dimethylformamid, Dimethylsulfoxid oder Acetonitril. Ebenso ist es möglich, Gemische der Lösemittel einzusetzen. Besonders bevorzugt ist Dimethylformamid.

Die Verbindungen der Formel (III) sind bekannt, lassen sich nach bekannten Verfahren aus den entsprechenden Ausgangsverbindungen synthetisieren oder können analog den im Beispielteil beschriebenen Verfahren hergestellt werden.

Die Umsetzung der zweiten Stufe nach Verfahren [A] erfolgt bei einer sauren Esterspaltung im Allgemeinen in inerten Lösungsmitteln, bevorzugt in einem Temperaturbereich von Raumtemperatur bis 60°C bei Normaldruck.

Inerte Lösungsmittel sind beispielsweise Halogenkohlenwasserstoffe wie Dichlormethan, Trichlormethan, Tetrachlormethan oder 1,2-Dichlorethan, oder Ether wie Tetrahydrofuran oder Dioxan, bevorzugt ist Dichlormethan.

Säuren sind beispielsweise Trifluoressigsäure oder Chlorwasserstoff in Dioxan, bevorzugt ist Trifluoressigsäure.

Die Umsetzung der zweiten Stufe nach Verfahren [A] erfolgt bei einer basischen Esterspaltung im Allgemeinen in inerten Lösungsmitteln, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Dichlormethan, Trichlormethan, Tetrachlormethan oder 1,2-Dichlorethan, Alkohole wie Methanol oder Ethanol, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist ein Gemisch aus Tetrahydrofuran und Wasser.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, oder Alkoholate wie Kalium- oder Natrium-tert-butylat, bevorzugt ist Lithiumhydroxid.

Die Umsetzung nach Verfahren [B] erfolgt im Allgemeinen in inerten Lösungsmitteln, in Gegenwart eines Katalysators, gegebenenfalls in Gegenwart eines Zusatzreagenzes, gegebenenfalls in einer Mikrowelle, bevorzugt in einem Temperaturbereich von Raumtemperatur bis 150°C bei Normaldruck bis 3 bar.

Katalysatoren sind beispielsweise für Suzuki-Reaktionsbedingungen übliche Palladium-Katalysatoren, bevorzugt sind Katalysatoren wie z.B. Dichlorbis(triphenylphosphin)-palladium, Tetrakistriphenylphosphinpalladium(0), Palladium(II)acetat/Triscyclohexylphosphin, Tris(dibenzylidenaceton)dipalladium, Bis-(diphenylphosphanferrocenyl)-palladium-(II)-chlorid, 1,3-Bis(2,6-diisopropylphenyl)imidazol-2-yliden(1,4-napthtochinon)palladiumdimer, Allyl(chlor)-(1,3-dimesityl-1,3-dihydro-2H-imidazol-2-yliden)palladium, Palladium(II)acetat/Dicyclohexyl-(2',4',6'-triisopropyl-biphenyl-2-yl)-phosphin, [1,1-Bis-(diphenylphosphino)-ferrocen]-palladium(II)chlorid-Monodichlormethan-addukt oder XPhos Präkatalysator [(2'-Aminobiphenyl-2-yl)(chlor)palladium-Dicyclohexyl(2',4',6'-triisopropylbiphenyl-2-yl)phosphan (1:1)], bevorzugt ist Tetrakistriphenylphosphin-palladium(0), [1,1-Bis-(diphenylphosphino)-ferrocen]-palladium(II)chlorid-Monodichlormethan-addukt oder XPhos Präkatalysator [(2'-Aminobiphenyl-2-yl)(chlor)palladium-Dicyclohexyl(2',4',6'-triisopropylbiphenyl-2-yl)phosphan (1:1)].

Zusatzreagenzien sind beispielsweise Kaliumacetat, Cäsium-, Kalium- oder Natriumcarbonat, Kalium-tert.-butylat, Cäsiumfluorid oder Kaliumphosphat, wobei diese in wässriger Lösung vorliegen können, bevorzugt sind Zusatzreagenzien wie Kaliumcarbonat oder wässrige Kaliumphosphat-Lösung.

Inerte Lösungsmittel sind beispielsweise Ether wie Dioxan, Tetrahydrofuran oder 1,2-Dimethoxyethan, Kohlenwasserstoffe wie Benzol, Xylol oder Toluol, oder Carbonsäureamide wie Dimethylformamid oder Dimethylacetamid, Alkylsulfoxide wie Dimethylsulfoxid, oder N-Methylpyrrolidon oder Acetonitril, oder Gemische der Lösungsmittel mit Alkoholen wie Methanol oder Ethanol und/oder Wasser, bevorzugt ist Tetrahydrofuran, Dioxan oder Acetonitril.

Die Verbindungen der Formel (V) sind bekannt oder lassen sich nach bekannten Verfahren aus den entsprechenden Ausgangsverbindungen synthetisieren.

Die Verbindungen der Formel (II) sind bekannt oder können hergestellt werden, indem
[C] Verbindungen der Formel 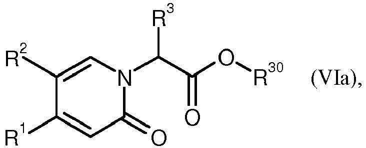 in welcher
   R¹, R² und R³ die oben angegebene Bedeutung haben, und
   R³⁰ für tert-Butyl steht,
   mit einer Säure umgesetzt werden,
   oder
[D] Verbindungen der Formel 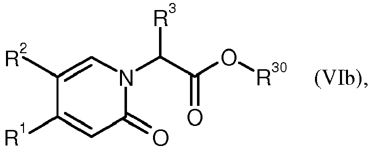 in welcher
   R¹, R² und R³ die oben angegebene Bedeutung haben, und
   R³⁰ für Methyl oder Ethyl steht,
mit einer Base umgesetzt werden.

Die Verbindungen der Formeln (VIa) und (VIb) bilden zusammen die Menge der Verbindungen der Formel (VI).

Die Umsetzung nach Verfahren [C] erfolgt im Allgemeinen in inerten Lösungsmitteln, bevorzugt in einem Temperaturbereich von Raumtemperatur bis 60°C bei Normaldruck.

Inerte Lösungsmittel sind beispielsweise Halogenkohlenwasserstoffe wie Dichlormethan, Trichlormethan, Tetrachlormethan oder 1,2-Dichlorethan, oder Ether wie Tetrahydrofuran oder Dioxan, bevorzugt ist Dichlormethan.

Säuren sind beispielsweise Trifluoressigsäure oder Chlorwasserstoff in Dioxan, bevorzugt ist Trifluoressigsäure.

Die Umsetzung nach Verfahren [D] erfolgt im Allgemeinen in inerten Lösungsmitteln, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Dichlormethan, Trichlormethan, Tetrachlormethan oder 1,2-Dichlorethan, Alkohole wie Methanol oder Ethanol, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist ein Gemisch aus Tetrahydrofuran und Wasser.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, oder Alkoholate wie Kalium- oder Natrium-tert-butylat, bevorzugt ist Lithiumhydroxid.

Die Verbindungen der Formel (VI) sind bekannt oder können hergestellt werden, indem
[E] Verbindungen der Formel 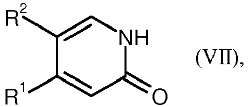 in welcher
   R¹ und R² die oben angegebene Bedeutung haben,
   mit Verbindungen der Formel 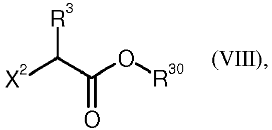 in welcher
   R³ die oben angegebene Bedeutung hat,
   R³⁰ für Methyl, Ethyl oder tert-Butyl steht, und
   X² für Chlor, Brom, Iod, Methansulfonyloxy oder Trifluormethansulfonyloxy steht, umgesetzt werden,
      oder
[F] Verbindungen der Formel 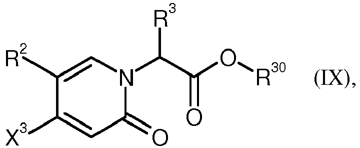 in welcher
   R² und R³ die oben angegebene Bedeutung haben,
   R³⁰ für Methyl, Ethyl oder tert-Butyl steht, und
   X³ für Chlor, Brom oder Iod steht,
mit Verbindungen der Formel (V) unter Suzuki-Kupplungsbedingungen umgesetzt werden.

Die Umsetzung nach Verfahren [E] erfolgt im Allgemeinen in inerten Lösungsmitteln, gegebenenfalls in Gegenwart einer Base, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Dichlormethan, Trichlormethan, Tetrachlormethan oder 1,2-Dichlorethan, Alkohole wie Methanol oder Ethanol, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist Dimethylformamid.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, oder Kalium- oder Natrium-tert-butylat, Natriumhydrid oder eine Mischung aus diesen Basen oder eine Mischung aus Natriumhydrid und Lithiumbromid, bevorzugt ist Kaliumcarbonat oder Natriumhydrid.

Die Verbindungen der Formel (VIII) sind bekannt oder lassen sich nach bekannten Verfahren aus den entsprechenden Ausgangsverbindungen synthetisieren.

Die Umsetzung nach Verfahren [F] erfolgt wie für Verfahren [B] beschrieben.

Die Verbindungen der Formel (VII) sind bekannt oder können hergestellt werden, indem Verbindungen der Formel 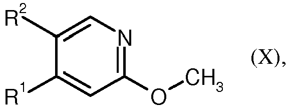 in welcher
R¹ und R² die oben angegebene Bedeutung haben,
mit Pyridinium-Hydrochlorid oder Pyridinium-Hydrobromid umgesetzt werden.

Die Umsetzung erfolgt im Allgemeinen in inerten Lösungsmitteln, bevorzugt in einem Temperaturbereich von 80°C bis 120°C bei Normaldruck.

Inerte Lösungsmittel sind beispielsweise Kohlenwasserstoffe wie Benzol, oder andere Lösungsmittel wie Nitromethan, Dioxan, Dimethylformamid, Dimethylsulfoxid oder Acetonitril. Ebenso ist es möglich, Gemische der Lösemittel einzusetzen. Besonders bevorzugt ist Dimethylformamid.

Die Verbindungen der Formel (X) sind bekannt oder können hergestellt werden, indem Verbindungen der Formel 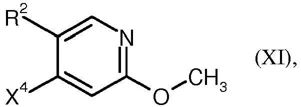 in welcher
R² die oben angegebene Bedeutung hat, und
X⁴ für Chlor, Brom oder Iod steht,
mit Verbindungen der Formel (V) unter Suzuki-Kupplungsbedingungen umgesetzt werden.

Die Umsetzung erfolgt wie für Verfahren [B] beschrieben.

Die Verbindungen der Formel (XI) sind bekannt oder lassen sich nach bekannten Verfahren aus den entsprechenden Ausgangsverbindungen synthetisieren.

Die Verbindungen der Formel (IX) sind bekannt oder können hergestellt werden, indem Verbindungen der Formel 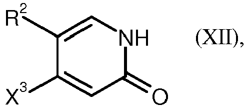 in welcher
R² die oben angegebene Bedeutung hat, und
X³ für Chlor, Brom oder Iod steht,
mit Verbindungen der Formel (VIII) umgesetzt werden.

Die Umsetzung erfolgt wie für Verfahren [E] beschrieben.

Die Verbindungen der Formel (XII) sind bekannt oder lassen sich nach bekannten Verfahren aus den entsprechenden Ausgangsverbindungen synthetisieren.

Die Verbindungen der Formel (IV) sind bekannt oder können hergestellt werden, indem Verbindungen der Formel 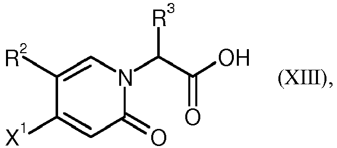 in welcher
R² und R³ die oben angegebene Bedeutung haben, und
X¹ für Chlor, Brom oder Iod steht,
mit Verbindungen der Formel (III) in Gegenwart eines Dehydratisierungsreagenzes umgesetzt werden.

Die Umsetzung erfolgt wie für Verfahren [A] beschrieben.

Die Verbindungen der Formel (XIII) sind bekannt oder können hergestellt werden, indem
[G] Verbindungen der Formel 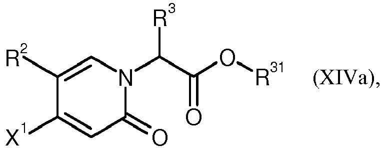 in welcher
   R² und R³ die oben angegebene Bedeutung haben,
   R³¹ für tert-Butyl steht, und
   X¹ für Chlor, Brom oder Iod steht,
   mit einer Säure umgesetzt werden,
   oder
[H] Verbindungen der Formel 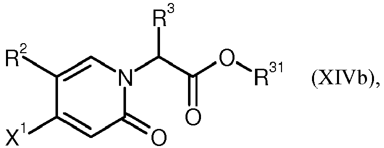 in welcher
   R² und R³ die oben angegebene Bedeutung haben,
   R³¹ für Methyl oder Ethyl steht, und
   X¹ für Chlor, Brom oder Iod steht,
mit einer Base umgesetzt werden.

Die Verbindungen der Formeln (XIVa) und (XIVb) bilden zusammen die Menge der Verbindungen der Formel (XIV).

Die Umsetzung nach Verfahren [G] erfolgt wie für Verfahren [C] beschrieben.

Die Umsetzung nach Verfahren [H] erfolgt wie für Verfahren [D] beschrieben.

Die Verbindungen der Formel (XIV) sind bekannt oder können hergestellt werden, indem Verbindungen der Formel 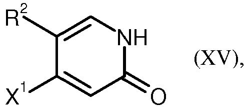 in welcher
R² die oben angegebene Bedeutung habt, und
X¹ für Chlor, Brom oder Iod steht,
mit Verbindungen der Formel 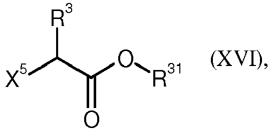 in welcher
R³ die oben angegebene Bedeutung hat,
R³¹ für Methyl, Ethyl oder tert-Butyl steht, und
X⁵ für Chlor, Brom, Iod, Methansulfonyloxy oder Trifluormethansulfonyloxy steht,
umgesetzt werden.

Die Umsetzung erfolgt wie für Verfahren [E] beschrieben.

Die Verbindungen der Formel (XV) und (XVI) sind bekannt oder lassen sich nach bekannten Verfahren aus den entsprechenden Ausgangsverbindungen synthetisieren.

In einem alternativen Verfahren können die Verbindungen der Formel (VI) hergestellt werden, indem Verbindungen der Formel 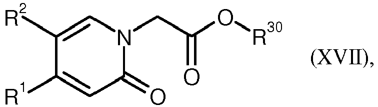 in welcher
R¹ und R² die oben angegebene Bedeutung haben, und
R³⁰ für Methyl, Ethyl oder tert-Butyl steht,
mit Verbindungen der Formel 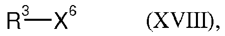 in welcher
R³ die oben angegebene Bedeutung hat, und
X⁶ für Chlor, Brom, Iod, Methansulfonyloxy, Trifluormethansulfonyloxy oder para-Toluolsulfonyloxy steht,
umgesetzt werden.

Die Umsetzung erfolgt im Allgemeinen in inerten Lösungsmitteln, gegebenenfalls in Gegenwart einer Base, bevorzugt in einem Temperaturbereich von -78°C bis Raumtemperatur bei Normaldruck.

Inerte Lösungsmittel sind beispielsweise Halogenkohlenwasserstoffe wie Dichlormethan, Trichlormethan, Tetrachlormethan oder 1,2-Dichlorethan, Alkohole wie Methanol oder Ethanol, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist Tetrahydrofuran.

Basen sind beispielsweise Kalium- oder Natrium-tert-butylat, Natriumhydrid, N-Butyllithium oder Bis-(trimethylsilyl)-lithiumamid, bevorzugt ist Bis-(trimethylsilyl)-lithiumamid.

Die Verbindungen der Formel (XVII) sind bekannt oder lassen sich nach den oben beschriebenen Verfahren, z.B. Verfahren [E], aus den entsprechenden Ausgangsverbindungen synthetisieren.

Die Verbindungen der Formel (XVIII) sind bekannt oder lassen sich nach bekannten Verfahren aus den entsprechenden Ausgangsverbindungen synthetisieren.

In einem alternativen Verfahren können die Verbindungen der Formel (II) hergestellt werden, indem Verbindungen der Formel 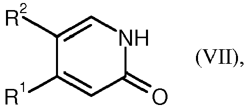 in welcher
R¹ und R² die oben angegebene Bedeutung haben,
mit Verbindungen der Formel 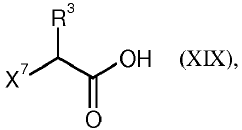 in welcher
R³ die oben angegebene Bedeutung hat, und
X⁷ für Chlor, Brom oder Iod steht,
umgesetzt werden.

Die Umsetzung erfolgt im Allgemeinen in inerten Lösungsmitteln, gegebenenfalls in Gegenwart einer Base, bevorzugt in einem Temperaturbereich von -10°C bis 90°C bei Normaldruck.

Inerte Lösungsmittel sind beispielsweise Halogenkohlenwasserstoffe wie Dichlormethan, Trichlormethan, Tetrachlormethan oder 1,2-Dichlorethan, Alkohole wie Methanol oder Ethanol, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist Tetrahydrofuran.

Basen sind beispielsweise Kalium- oder Natrium-tert-butylat, Natriumhydrid oder Bis-(trimethylsilyl)-lithiumamid oder ein Gemisch aus Magnesiumdi-tert-butylat und Kalium-tert-butylat, bevorzugt ist ein Gemisch aus Magnesiumdi-tert-butylat und Kalium-tert-butylat.

Die Verbindungen der Formel (XIX) sind bekannt oder lassen sich nach bekannten Verfahren aus den entsprechenden Ausgangsverbindungen synthetisieren.

In einem alternativen Verfahren können die Verbindungen der Formel (XIII) hergestellt werden, indem Verbindungen der Formel 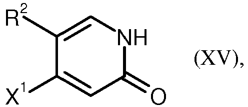 in welcher
R² die oben angegebene Bedeutung habt, und
X¹ für Chlor, Brom oder Iod steht,
mit Verbindungen der Formel 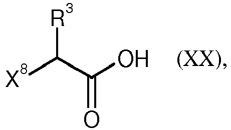 in welcher
R³ die oben angegebene Bedeutung hat, und
X⁸ für Chlor, Brom oder Iod steht,
umgesetzt werden.

Die Umsetzung erfolgt wie für die Umsetzung von Verbindungen der Formel (VII) mit Verbindungen der Formel (XIX) beschrieben.

Die Verbindungen der Formel (XX) sind bekannt oder lassen sich nach bekannten Verfahren aus den entsprechenden Ausgangsverbindungen synthetisieren.

Die Herstellung der Ausgangsverbindungen und der Verbindungen der Formel (I) kann durch das folgende Syntheseschema verdeutlicht werden.

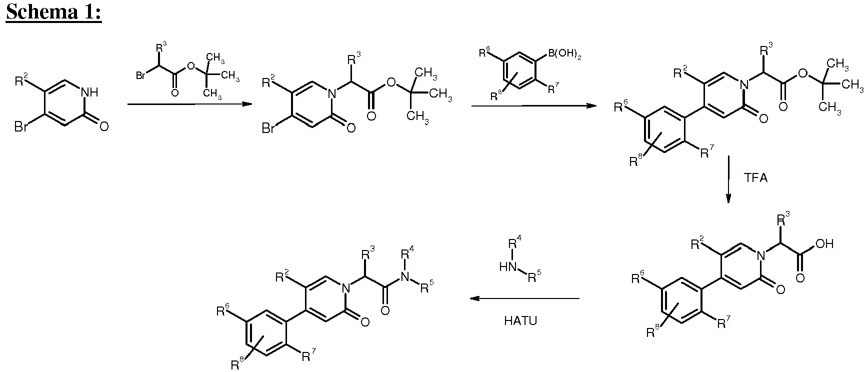

Die erfindungsgemäßen Verbindungen zeigen ein nicht vorhersehbares, wertvolles pharmakologisches Wirkspektrum und ein gutes pharmakokinetisches Verhalten. Es handelt sich dabei um Verbindungen, die die proteolytische Aktivität der Serinprotease Faktor XIa (FXIa) und/oder der Serinprotease Plasmakallikrein (PK) beeinflussen. Die erfindungsgemäßen Verbindungen hemmen die von FXIa und/oder PK katalysierte enzymatische Spaltung von Substraten, die wesentliche Rollen in der Aktivierung der Blutgerinnung, in der Aggregation von Blutplättchen via Reduktion des für die PAR-1-Aktivierung der Plättchen notwendigen Thrombins, und in inflammatorischen Prozessen einnehmen, die insbesondere eine Steigerung der Gefäßpermeabilität miteinschließen. Sie eigenen sich daher zur Verwendung als Arzneimittel zur Behandlung und/oder Prophylaxe von Krankheiten bei Menschen und Tieren.

Weiterer Gegenstand der vorliegenden Erfindung sind die erfindungsgemäßen Verbindungen zur Behandlung und/oder Prophylaxe von Erkrankungen, insbesondere von Herz-Kreislauf-Erkrankungen, vorzugsweise von thrombotischen beziehungsweise thromboembolischen Erkrankungen und/oder thrombotischen beziehungsweise thromboembolischen Komplikationen, und/oder ophthalmologischen Erkrankungen, insbesondere von diabetischer Retinopathie beziehungsweise des Makulaödems, und/oder entzündlichen Erkrankungen, insbesondere diejenigen, die mit übermäßiger Plasmakallikrein-Aktivität in Zusammenhang stehen, wie z.B. das hereditäre Angioödem (HAE) oder chronisch-entzündliche Erkrankungen, insbesondere des Darms, wie z. B. Morbus Crohn.

Faktor XIa (FXIa) ist ein wichtiges Enzym im Rahmen der Koagulation, das sowohl durch Thrombin als auch Faktor XIIa (FXIIa) aktiviert werden kann und somit in zwei wesentlichen Prozessen der Koagulation involviert ist: Es ist ein zentraler Bestandteil des Übergangs von der Initiation zur Amplifikation und Propagation der Koagulation: Thrombin aktiviert in positiven Rückkopplungsschleifen neben Faktor V und Faktor VIII auch Faktor XI zu Faktor XIa, der Faktor IX zu Faktor IXa umsetzt und über den so generierten Faktor IXa/ Faktor VIIIa-Komplex die Faktor X-Aktivierung und damit wiederum die Thrombinbildung stark stimuliert, was zu starkem Thrombuswachstum führt und den Thrombus stabilisiert.

Darüber hinaus ist Faktor XIa wichtiger Bestandteil der intrinsischen Initiation der Gerinnung: Neben der Stimulation über Gewebefaktor (tissue factor, TF) kann die Aktivierung des Gerinnungssystems auch auf insbesondere negativ geladenen Oberflächen erfolgen, zu denen neben Oberflächenstrukturen körperfremder Zellen (z.B. Bakterien) auch artifizielle Oberflächen wie Gefäßprothesen, Stents und extrakorporale Kreisläufe gehören. Auf der Oberfläche findet zunächst die Aktivierung von Faktor XII (FXII) zu Faktor XIIa (FXIIa) statt, der im Folgenden an Zelloberflächen gebundenen FXI zu FXIa aktiviert. Dieser führt wie zuvor beschrieben zur weiteren Aktivierung der Gerinnungskaskade.

Dagegen bleibt die Thrombingenerierung in der Initiationsphase über TF/Faktor VIIa und Faktor X-Aktivierung und letztlich Thrombinbildung, die physiologische Reaktion auf Gefäßverletzungen, unbeeinflußt. Dies könnte erklären, warum keine Verlängerungen der Blutungszeiten in FXIa-Knock- out-Mäusen, wie auch in Kaninchen und anderen Spezies unter FXIa-Inhibitor-Gabe gefunden wurde. Diese niedrige durch die Substanz hervorgerufene Blutungsneigung ist für die Anwendung am Menschen insbesondere bei Patienten mit erhöhtem Blutungsrisiko von großem Vorteil.

Daneben aktiviert Faktor XIIa im Rahmen der intrinsischen Aktivierung ebenfalls Plasmaprokallikrein zu Plasmakallikrein (PK), das unter anderem zu weiterer Faktor XII-Aktivierung im Rahmen einer Potentierungsschleife führt, was insgesamt eine Verstärkung der Initiation der Gerinnungskaskade an Oberflächen zur Folge hat. Eine PK-hemmende Aktivität einer erfindungsgemäßen Verbindung wird also die Gerinnung via Oberflächenaktivierung reduzieren und somit antikoagulatorisch wirken. Ein Vorteil könnte in der Kombination von Faktor XIa- und PK-inhibitorischer Aktivität liegen, die eine balancierte antithrombotische Wirkung zuläßt.

Die erfindungsgemäßen Verbindungen eignen sich daher zur Behandlung und/oder Prophylaxe von Erkrankungen oder Komplikationen, die durch Gerinnselbildung entstehen können.

Zu den "thrombotischen beziehungsweise thromboembolischen Erkrankungen" im Sinne der vorliegenden Erfindung zählen Erkrankungen, die sowohl im arteriellen als auch im venösen Gefäßbett auftreten und mit den erfindungsgemäßen Verbindungen behandelt werden können, insbesondere Erkrankungen in den Koronararterien des Herzens, wie das akute Koronarsyndrom (ACS), Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie, Stentimplantation oder aortokoronarem Bypass, aber auch thrombotische beziehungsweise thromboembolische Erkrankungen in weiteren Gefäßen, die zu peripheren arteriellen Verschlusskrankheiten, Lungenembolien, venösen Thromboembolien, venösen Thrombosen, insbesondere in tiefen Beinvenen und Nierenvenen, transitorische ischämische Attacken sowie thrombotischer Hirnschlag und thromboembolischer Hirnschlag führen.

Die Stimulation des Gerinnungssystems kann durch verschiedene Ursachen beziehungsweise Begleiterkrankungen erfolgen. Unter anderem kann im Rahmen von chirurgischen Eingriffen, Immobilität, Bettlägerigkeit, Infektionen, Inflammation oder einer Krebserkrankung beziehungsweise Krebstherapie das Gerinnungssystem stark angeregt sein und es zu thrombotischen Komplikationen, insbesondere venösen Thrombosen, kommen. Die erfindungsgemäßen Verbindungen eignen sich daher zur Thromboseprophylaxe im Rahmen von chirurgischen Eingriffen bei Patienten, die eine Krebserkrankung haben. Die erfindungsgemäßen Verbindungen eignen sich daher auch zur Thromboseprophylaxe in Patienten mit aktiviertem Gerinnungssystem, beispielsweise unter den beschriebenen Stimulationssituationen.

Die erfindungsgemäßen Verbindungen eignen sich daher auch zur Prävention und Behandlung von kardiogenen Thromboembolien, wie beispielsweise Hirn-Ischämien, Schlaganfall und systemischen Thromboembolien und Ischämien, bei Patienten mit akuten, intermittierenden oder persistierenden Herzarrhythmien, wie beispielsweise Vorhofflimmern, und bei Patienten, die sich einer Kardioversion unterziehen, ferner bei Patienten mit Herzklappen-Erkrankungen oder mit künstlichen Herzklappen.

Darüber hinaus sind die erfindungsgemäßen Verbindungen zur Behandlung und Prävention einer disseminierten intravasalen Gerinnung (DIC) geeignet, die unter anderem im Rahmen einer Sepsis, aber auch infolge von Operationen, Tumorerkrankungen, Verbrennungen oder anderen Verletzungen auftreten und durch Mikrothrombosierungen zu schweren Organschäden führen kann.

Thromboembolische Komplikationen treten ferner auf bei mikroangiopathischen hämolytischen Anämien und durch Kontakt des Blutes mit körperfremden Oberflächen im Rahmen von extrakorporalen Blutkreisläufen, wie zum Beispiel Hämodialyse, ECMO ("extracorporal membrane oxygenation"), LVAD ("left ventricular assist device") und ähnlichen Verfahren, AV-Fisteln, Gefäß- sowie Herzklappenprothesen.

Außerdem kommen die erfindungsgemäßen Verbindungen zur Behandlung und/oder Prophylaxe von Erkrankungen in Betracht, bei denen Mikrogerinnselbildungen beziehungsweise Fibrinablagerungen in Gehirngefäßen auftreten, die zu Demenzerkrankungen, wie beispielsweise vaskulärer Demenz oder Morbus Alzheimer führen können. Hierbei kann das Gerinnsel sowohl über Okklusionen als auch über die Bindung weiterer krankheitsrelevanter Faktoren zur Erkrankung beitragen.

Außerdem kommen die erfindungsgemäßen Verbindungen insbesondere zur Behandlung und/oder Prophylaxe von Erkrankungen in Betracht, bei denen neben der prokoagulanten auch die proinflammatorische Komponente eine wesentliche Rolle spielt. Insbesondere die gegenseitige Verstärkung von Koagulation und Inflammation kann durch die erfindungsgemäßen Verbindungen unterbunden und daher die Wahrscheinlichkeit für eine thrombotische Komplikation entscheidend gesenkt werden. Dabei können sowohl die Faktor XIa-inhibitorische Komponente (über Hemmung der Thrombinproduktion) als auch die PK-inhibitorische Komponente zur antikoagulanten und antiinflammatorischen Wirkung (z.B. via Bradikinin) beitragen. Daher kommen unter anderem die Behandlung und/oder Prophylaxe im Rahmen von atherosklerotischen Gefäßerkrankungen, Entzündungen im Rahmen von rheumatischen Erkrankungen des Bewegungsapparates, entzündlichen Erkrankungen der Lunge, wie beispielsweise Lungenfibrosen, entzündliche Erkrankungen der Niere, wie beispielsweise Glomerulonephritiden, entzündliche Erkrankungen des Darms, wie beispielsweise Morbus Crohn oder Colitis ulcerosa, oder Erkrankungen, die im Rahmen einer diabetischen Grunderkrankung vorliegen können, wie beispielsweise diabetische Retinopathie beziehungsweise Nephropathie in Betracht.

Bei der Progression von chronisch-entzündlichen Darmerkrankungen (CED) haben unter anderem mittels Plasmakallikrein generierten Kinine eine tragende Rolle. Deren pro-inflammatorische Wirkung über Aktivierung von Bradykinin-Rezeptoren induziert und potenziert den Krankheitsverlauf. Studien an Morbus Crohn-Patienten zeigen eine Korrelation zwischen der Kallikrein-Konzentration im Darmepithel und dem Grad der Darmentzündung. Eine Aktivierung des Kallikrein-Kinin-Systems wurde ebenfalls in tierexperimentellen Studien beobachtet. Eine Hemmung der Bradykinin-Synthese durch Kallikrein-Inhibitoren könnte demnach auch zur Prophylaxe und/oder Therapie von chronisch-entzündlichen Darmerkrankungen eingesetzt werden.

Außerdem können die erfindungsgemäßen Verbindungen zur Inhibition des Tumorwachstums und der Metastasenbildung, sowie zur Prophylaxe und/oder Behandlung thromboembolischer Komplikationen, wie beispielsweise venöser Thromboembolien, bei Tumorpatienten, insbesondere solchen, die sich größeren chirurgischen Eingriffen oder einer Chemo- oder Radiotherapie unterziehen, eingesetzt werden.

Außerdem kommen die erfindungsgemäßen Verbindungen auch für die Prophylaxe und/oder Behandlung von pulmonaler Hypertonie in Betracht.

Der Begriff "pulmonale Hypertonie" umfasst im Rahmen der vorliegenden Erfindung die pulmonale arterielle Hypertonie, die pulmonale Hypertonie bei Erkrankungen des linken Herzens, die pulmonale Hypertonie bei Lungenerkrankung und/oder Hypoxie und die Pulmonale Hypertonie aufgrund chronischer Thrombembolien (CTEPH).

Die "pulmonale arterielle Hypertonie" beinhaltet die Idiopathische Pulmonale Arterielle Hypertonie (IPAH, früher auch als primäre pulmonale Hypertonie bezeichnet), die Familiär bedingte Pulmonale Arterielle Hypertonie (FPAH) und die Assoziierte Pulmonal-Arterielle Hypertonie (APAH), die assoziiert ist mit Kollagenosen, kongenitalen systemisch-pulmonalen Shuntvitien, portaler Hypertension, HIV-Infektionen, der Einnahme bestimmter Drogen und Medikamente, mit anderen Erkrankungen (Schilddrüsenerkrankungen, Glykogenspeicherkrankheiten, Morbus Gaucher, hereditäre Teleangiektasie, Hämoglobinopathien, myeloproliferative Erkrankungen, Splenektomie), mit Erkrankungen mit einer signifikanten venösen/kapillären Beteiligung wie der pulmonal-venookklusiven Erkrankung und der pulmonal-kapillären Hämangiomatose, sowie die persistierende pulmonale Hypertonie der Neugeborenen.

Die pulmonale Hypertonie bei Erkrankungen des linken Herzens beinhaltet die Erkrankung des linken Vorhofes oder Ventrikels und Mitral- oder Aortenklappenfehler.

Die pulmonale Hypertonie bei Lungenerkrankung und/oder Hypoxie beinhaltet chronisch obstruktive Lungenerkrankungen, interstitielle Lungenerkrankung, Schlafapnoe-Syndrom, alveolärer Hypoventilation, chronische Höhenkrankheit und anlagebedingte Fehlbildungen.

Die Pulmonale Hypertonie aufgrund chronischer Thrombembolien (CTEPH) beinhaltet den thrombembolischen Verschluss proximaler Lungenarterien, den thrombembolischen Verschluss distaler Lungenarterien und nicht-thrombotische Lungenembolien (Tumor, Parasiten, Fremdkörper).

Weiterer Gegenstand der vorliegenden Erfindung ist die Verwendung der erfindungsgemäßen Verbindungen zur Herstellung von Arzneimitteln zur Behandlung und/oder Prophylaxe von pulmonaler Hypertonie bei Sarkoidose, Histiozytose X und Lymphangiomatosis.

Außerdem kommen die erfindungsgemäßen Substanzen auch zur Behandlung von pulmonalen und hepatischen Fibrosen in Betracht.

Außerdem kommen die erfindungsgemäßen Verbindungen auch für die Behandlung und/oder Prophylaxe einer Disseminierten intravasalen Koagulation im Rahmen einer Infektionserkrankung und/oder von Systemic Inflammatory Syndrome (SIRS), septischer Organdysfunktion, septischem Organversagen und Multiorganversagen, Acute Respiratory Distress Syndrome (ARDS), Acute Lung Injury (ALI), Septischer Schock und/oder des septischen Organversagens in Betracht.

Im Verlauf einer Infektion kann es zur generalisierten Aktivierung des Gerinnungssystems kommen ("Disseminated Intravascular coagulation", oder "Verbrauchskoagulopathie", nachfolgend als "DIC" bezeichnet) mit Mikrothrombosierung in verschiedenen Organen und sekundärer Blutungskomplikationen. Außerdem kann es zur endothelialen Schädigung mit Erhöhung der Gefäßpermeabilität und Austritt von Flüssigkeit und Proteinen in den Extravasalraum kommen. Im weiteren Verlauf kann ein Versagen eines Organs (z.B. Nierenversagen, Leberversagen, Atemversagen, zentralnervöse Defizite und Herz-/Kreislaufversagen) oder zum Multiorganversagen kommen.

Bei der DIC kommt es an der Oberfläche von geschädigten Endothelzellen, Fremdkörperoberflächen oder vernetztem extravaskulärem Gewebe zur massiven Aktivierung des Gerinnungssystems. Als Folge kommt es zur Gerinnung in kleinen Gefäßen verschiedener Organe mit Hypoxie und anschließender Organdysfunktion. Sekundär kommt es zum Verbrauch von Gerinnungsfaktoren (z.B. Faktor X, Prothrombin und Fibrinogen) und Plättchen, wodurch die Gerinnungsfähigkeit des Blutes herabgesetzt wird und schwere Blutungen auftreten können.

Erfindungsgemäße Verbindungen, die Plasmakallikrein alleine oder in Kombination mit Faktor XIa hemmen, kommen zusätzlich zur Behandlung und/oder Prophylaxe von Erkrankungen in Betracht, in deren Verlauf Plasmakallikrein involviert ist. Zusätzlich zur antikoagulanten Aktivität stellt Plasmakallikrein eine wichtige Bradikinin-freisetzende Protease dar, die somit unter anderem zum Anstieg der endothelialen Permeabilität führt. Die Verbindungen können somit zur Behandlung und/oder Prophylaxe von Erkrankungen eingesetzt werden, die mit Ödembildungen einhergehen, wie zum Beispiel ophthalmologische Erkrankungen, insbesondere die diabetische Retinopathie oder das Makulaödem, oder das hereditäre Angioödem.

Zu den "ophthalmologischen Erkrankungen" im Sinne der vorliegenden Erfindung zählen insbesondere Erkrankungen wie diabetische Retinopathie, diabetisches Makulaödem (diabetic macular edema, DME), Makulaödem, Makulaödem assoziiert mit retinalem Venenverschluss, altersbedingte Makula-Degeneration (AMD), choroidale Neovaskularisierung (CNV), choroidale neovaskuläre Membranen (CNVM), zystoides Makulaödem (cystoid macula edema, CME), epiretinale Membranen (ERM) und Makula-Perforationen, Myopie-assoziierte choroidale Neovaskularisierung, angioide beziehungsweise vaskuläre Streifen (angioid streaks, vascular streaks), Retina-Ablösung, atrophische Veränderungen des retinalen Pigmentepithels, hypertrophische Veränderungen des retinalen Pigmentepithels, retinaler Venenverschluss, choroidaler retinaler Venenverschluss, Retinitis pigmentosa, Stargardt'sche Erkrankung, Frühgeborenen-Retinopathie, Glaukom, entzündliche Erkrankungen des Auges wie z.B. Uveitis, Skleritis oder Endophthalmitis, Katarakt, Refraktionsanomalien wie z.B. Myopie, Hyperopie oder Astigmatismus und Keratokonus, Erkrankungen des vorderen Auges wie z.B. korneale Angiogenese als Folge von z.B. Keratitis, Hornhaut-Transplantation oder Keratoplastie, korneale Angiogenese als Folge von Hypoxie (z.B. durch zu langes Tragen von Kontaktlinsen), Pterygium conjunctivae, subkorneales Ödem und intrakorneales Ödem.

Weiterhin kommen die erfindungsgemäßen Verbindungen zur Primärprophylaxe thrombotischer oder thromboembolischer Erkrankungen und/oder inflammatorischer Erkrankungen und/oder Erkrankungen mit erhöhter Gefäßpermeabilität in Patienten in Frage, in denen Genmutationen zu verstärkter Aktivität der Enzyme oder erhöhten Spiegeln der Zymogene führen und diese durch entsprechende Tests/Messungen der Enzymaktivität bzw. Zymogenkonzentrationen festgestellt werden.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung sind die erfindungsgemäßen Verbindungen zur Verwendung in einem Verfahren zur Behandlung und/oder Prophylaxe von Erkrankungen, insbesondere der zuvor genannten Erkrankungen, unter Verwendung einer therapeutisch wirksamen Menge einer erfindungsgemäßen Verbindung.

Weiterer Gegenstand der vorliegenden Erfindung sind Arzneimittel, enthaltend eine erfindungsgemäße Verbindung und einen oder mehrere weitere Wirkstoffe.

Die erfindungsgemäßen Verbindungen können darüber hinaus auch zur Verhinderung von Koagulation *ex vivo* eingesetzt werden, z.B. zum Schutz von zu transplantierenden Organen vor durch Gerinnselbildung verursachten Organschäden und zum Schutz des Organ-Empfängers vor Thromboemboli aus dem transplantierten Organ, zur Konservierung von Blut- und Plasmaprodukten, zur Reinigung/Vorbehandlung von Kathetern und anderen medizinischen Hilfsmitteln und Geräten, zur Beschichtung künstlicher Oberflächen von *in vivo* oder *ex vivo* eingesetzten medizinischen Hilfsmitteln und Geräten oder bei biologischen Proben, die Faktor XIa oder Plasmakallikrein enthalten könnten.

Weiterer Gegenstand der vorliegenden Erfindung ist ein Verfahren zur Verhinderung der Blutkoagulation *in vitro,* insbesondere bei Blutkonserven oder biologischen Proben, die Faktor XIa oder Plasmakallikrein oder beide Enzyme enthalten könnten, das dadurch gekennzeichnet ist, dass eine antikoagulatorisch wirksame Menge der erfindungsgemäßen Verbindung zugegeben wird.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- Lipidsenker, insbesondere HMG-CoA-(3-Hydroxy-3-methylglutaryl-Coenzym A)-Reduktase-Inhibitoren wie beispielsweise Lovastatin (Mevacor), Simvastatin (Zocor), Pravastatin (Pravachol), Fluvastatin (Lescol) und Atorvastatin (Lipitor);
- Koronartherapeutika/Vasodilatatoren, insbesondere ACE-(Angiotensin-Converting-Enzyme)-Inhibitoren wie beispielsweise Captopril, Lisinopril, Enalapril, Ramipril, Cilazapril, Benazepril, Fosinopril, Quinapril und Perindopril, oder AII-(Angiotensin II)-Rezeptor-Antagonisten wie beispielsweise Embusartan, Losartan, Valsartan, Irbesartan, Candesartan, Eprosartan und Temisarta, oder β-Adrenozeptor-Antagonisten wie beispielsweise Carvedilol, Alprenolol, Bisoprolol, Acebutolol, Atenolol, Betaxolol, Carteolol, Metoprolol, Nadolol, Penbutolol, Pindolol, Propanolol und Timolol, oder alpha-1-Adrenozeptor-Antagonisten wie beispielsweise Prazosin, Bunazosin, Doxazosin und Terazosin, oder Diuretika wie beispielsweise Hydrochlorothiazid, Furosemid, Bumetanid, Piretanid, Torasemid, Amilorid und Dihydralazin, oder Calciumkanal-Blocker wie beispielsweise Verapamil und Diltiazem, oder Dihydropyridin-Derivate wie beispielsweise Nifedipin (Adalat) und Nitrendipin (Bayotensin), oder Nitropräparate wie beispielsweise Isosorbid-5-mononitrat, Isosorbiddinitrat und Glyceroltrinitrat, oder Substanzen, die eine Erhöhung von cyclischem Guanosinmonophosphat (cGMP) bewirken, wie beispielsweise Stimulatoren der löslichen Guanylatcyclase, wie beispielsweise Riociguat;
- Plasminogen-Aktivatoren (Thrombolytika/Fibrinolytika) und die Thrombolyse/Fibrinolyse steigernde Verbindungen wie Inhibitoren des Plasminogen-Aktivator-Inhibitors (PAI-Inhibitoren) oder Inhibitoren des Thrombin-aktivierten Fibrinolyse-Inhibitors (TAFI-Inhibitoren) wie beispielsweise Gewebsplasminogen-Aktivator (t-PA, beispielsweise Actilyse®), Streptokinase, Reteplase und Urokinase oder Plasminogen-modulierende Substanzen, die zu verstärkter Plasmin-Bildung führen;
- antikoagulatorisch wirksame Substanzen (Antikoagulantien) wie beispielsweise Heparin (UFH), niedermolekulare Heparine (NMH) wie beispielsweise Tinzaparin, Certoparin, Parnaparin, Nadroparin, Ardeparin, Enoxaparin, Reviparin, Dalteparin, Danaparoid, Semuloparin (AVE 5026), Adomiparin (M118) und EP-42675/ORG42675;
- direkte Thrombin Inhibitoren (DTI) wie beispielsweise Pradaxa (Dabigatran), Atecegatran (AZD-0837), DP-4088, SSR-182289A, Argatroban, Bivalirudin und Tanogitran (BIBT-986 und prodrug BIBT-1011), Hirudin;
- direkte Faktor Xa-Inhibitoren wie beispielsweise Rivaroxaban, Apixaban, Edoxaban (DU-176b), Betrixaban (PRT-54021), R-1663, Darexaban (YM-150), Otamixaban (FXV-673/RPR-130673), Letaxaban (TAK-442), Razaxaban (DPC-906), DX-9065a, LY-517717, Tanogitran (BIBT-986, prodrug: BIBT-1011), Idraparinux und Fondaparinux,
- plättchenaggregationshemmende Substanzen (Plättchenaggregationshemmer, Thrombozytenaggregationshemmer) wie beispielsweise Acetylsalicylsäure (wie beispielsweise Aspirin), P2Y12-Antagonisten wie beispielsweise Ticlopidin (Ticlid), Clopidogrel (Plavix), Prasugrel, Ticagrelor, Cangrelor, Elinogrel, PAR-1-Antagonisten wie beispielsweise Vorapaxar, PAR-4 Antagonisten, EP3-Antagonisten wie beispielsweise DG041;
- Plättchenadhäsionshemmern wie GPVI- und/oder GPIb-Antagonisten wie beispielsweise Revacept oder Caplacizumab;
- Fibrinogen-Rezeptor-Antagonisten (Glycoprotein-IIb/IIIa-Antagonisten) wie beispielsweise Abciximab, Eptifibatide, Tirofiban, Lamifiban, Lefradafiban und Fradafiban;
- rekombinantes humanes aktiviertes Protein C wie beispielsweise Xigris oder rekombinantes Thrombomodulin;
- sowie Antiarrhythmika;
- Inhibitoren der VEGF- und/oder PDGF Signalwege wie beispielsweise Aflibercept, Ranibizumab, Bevacizumab, KH-902, Pegaptanib, Ramucirumab, Squalamin oder Bevasiranib, Apatinib, Axitinib, Brivanib, Cediranib, Dovitinib, Lenvatinib, Linifanib, Motesanib, Pazopanib, Regorafenib, Sorafenib, Sunitinib, Tivozanib, Vandetanib, Vatalanib, Vargatef und E-10030;
- Hemmer der Angiopoietin-Tie Signalwege wie beispielsweise AMG386;
- Inhibitoren der Tie2 Rezeptortyrosinkinase;
- Hemmer der Integrin-Signalwege wie beispielsweise Volociximab, Cilengitid und ALG1001;
- Hemmer der PI3K-Akt-mTor Signalwege wie beispielsweise XL-147, Perifosin, MK2206, Sirolimus, Temsirolimus und Everolimus;
- Corticosteroid wie beispielsweise Anecortave, Betamethason, Dexamethason, Triamcinolon, Fluocinolon und Fluocinolonacetonid;
- Inhibitoren des ALK1-Smad1/5 Signalweges wie beispielsweise ACE041;
- Cyclooxygenaseinhibitoren wie beispielsweise Bromfenac und Nepafenac;
- Hemmer des Kallikrein-Kinin-Systems wie beispielsweise Safotibant und Ecallantid;
- Inhibitoren der Sphingosin-1-phosphat-Signalwege wie beispielsweise Sonepcizumab;
- Inhibitoren des Komplement-C5a Rezeptors wie beispielsweise Eculizumab;
- Inhibitoren des 5HTla Rezeptors wie beispielsweise Tandospiron;
- Hemmer des Ras-Raf-Mek-Erk Signalwegs; Hemmer der MAPK Signalwege; Hemmer der FGF-Signalwege; Hemmer der Endothelzellproliferation; Apoptose-induzierende Wirkstoffe;
- Photodynamische Therapie, bestehend aus einem Wirkstoff und der Einwirkung von Licht, wobei der Wirkstoff beispielsweise Verteporfin ist.

Unter "Kombinationen" im Sinne der Erfindung werden nicht nur Darreichungsformen, die alle Komponenten enthalten (sog. Fixkombinationen) und Kombinationspackungen, die die Komponenten voneinander getrennt enthalten, verstanden, sondern auch gleichzeitig oder zeitlich versetzt applizierte Komponenten, sofern sie zur Prophylaxe und/oder Behandlung derselben Krankheit eingesetzt werden. Ebenso ist es möglich, zwei oder mehr Wirkstoffe miteinander zu kombinieren, es handelt sich dabei also jeweils um zwei- oder mehrfach-Kombinationen.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat beziehungsweise Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nicht überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die extraoculare (topische) Applikation eignen sich nach dem Stand der Technik funktionierende schnell und/oder modifiziert beziehungsweise kontrolliert den Wirkstoff abgebende Applikationsformen, die den Wirkstoff in kristalliner und/oder amorphisierter und/oder gelöster Form enthalten, wie z.B. Augentropfen, Sprays und Lotionen (z.B. Lösungen, Suspensionen, vesikuläre/kolloidale Systeme, Emulsionen, Aerosole), Pulver für Augentropfen, Sprays und Lotionen (z.B. gemahlener Wirkstoff, Mischungen, Lyophilisate, gefällter Wirkstoff), halbfeste Augenzubereitungen (z.B. Hydrogele, in-situ Hydrogele, Cremes und Salben), Augeninserte (feste und halbfeste Zubereitungen, z.B. Bioadhesives, Filme/Wafer, Tabletten, Kontaktlinsen).

Die intraoculare Applikation umfasst z.B. die intravitreale subretinale, subsclerale, intrachoroidale, subconjunctivale, retrobulbare und subtenone Applikation. Für die intraoculare Applikation eignen sich nach dem Stand der Technik funktionierende schnell und/oder modifiziert beziehungsweise kontrolliert den Wirkstoff abgebende Applikationsformen, die den Wirkstoff in kristalliner und/oder amorphisierter und/oder gelöster Form enthalten, wie z.B. Injektionen und Konzentrate für Injektionen (z.B. Lösungen, Suspensionen, vesikuläre/kolloidale Systeme, Emulsionen, Pulver für Injektionen (z.B. gemahlener Wirkstoff, Mischungen, Lyophilisate, gefällter Wirkstoff), Gele für Injektionen (halbfeste Zubereitungen, z.B. Hydrogele, in-situ Hydrogele) und Implantate (feste Zubereitungen, z.B. bioabbaubare und nicht-bioabbaubare Implantate, implantierbare Pumpen).

Bevorzugt ist die orale Applikation beziehungsweise bei ophthalmologischen Erkrankungen die extraokulare und die intraokulare Applikation.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, vorzugsweise zusammen mit einem oder mehreren inerten nichttoxischen, pharmazeutisch geeigneten Hilfsstoff enthalten, sowie deren Verwendung zu den zuvor genannten Zwecken.

Im Allgemeinen hat es sich als vorteilhaft erwiesen, bei parenteraler Applikation Mengen von etwa 5 bis 250 mg je 24 Stunden zur Erzielung wirksamer Ergebnisse zu verabreichen. Bei oraler Applikation beträgt die Menge etwa 5 bis 500 mg je 24 Stunden.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt beziehungsweise Intervall, zu welchem die Applikation erfolgt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10% w/v": 100 ml Lösung oder Suspension enthalten 10 g Substanz.

### A) Beispiele

### Abkürzungen:

- Boc: *tert.*-Butyloxycarbonyl
- Bsp.: Beispiel
- ca.: circa
- d: Tag(e), Dublett (bei NMR)
- DC: Dünnschicht-Chromatographie
- DCM: Dichlormethan
- DCI: direkte chemische Ionisation (bei MS)
- dd: Doppeltes Dublett (bei NMR)
- DIC: *N,N'*-Diisopropylcarbodiimid
- DIEA: *N,N*-Diisopropylethylamin
- DMAP: 4-Dimethylaminopyridin
- DMF: *N,N*-Dimethylformamid
- DMSO: Dimethylsulfoxid
- d. Th.: der Theorie (bei Ausbeute)
- eq.: Äquivalent(e)
- ESI: Elektrospray-Ionisation (bei MS)
- h: Stunde(n)
- HATU: *O*-(7-Azabenzotriazol-1-yl)*-N,N,N',N'*-tetramethyluronium-Hexafluorphosphat
- HPLC: Hochdruck-, Hochleistungsflüssigchromatographie
- HV: Hochvakuum
- LC-MS: Flüssigchromatographie-gekoppelte Massenspektroskopie
- LDA: Lithiumdiisopropylamid
- m: Multiplett (bei NMR)
- min: Minute(n)
- MS: Massenspektroskopie
- NMR: Kernresonanzspektroskopie
- Oxima: Hydroxyiminocyanoessigsaeureethylester
- q: Quartett oder Quadruplett (bei NMR)
- quant.: quantitativ
- quin: Quintett (bei NMR)
- RP: reverse phase (bei HPLC)
- RT: Raumtemperatur
- Rₜ: Retentionszeit (bei HPLC)
- s: Singulett (bei NMR)
- sxt: Sextett (bei NMR)
- SFC: superkritische Flüssigkeitschromatographie (mit überkritischem Kohlendioxid als mobile Phase)
- t: Triplett (bei NMR)
- THF: Tetrahydrofuran
- TFA: Trifluoressigsäure
- T3P: 2,4,6-Tripropyl-1,3,5,2,4,6-trioxatriphosphinan-2,4,6-trioxid
- Xantphos: 4,5-Bis(diphenylphosphino)-9,9-dimethylxanthen
- XPhos Präkatalysator: [(2'-Aminobiphenyl-2-yl)(chlor)palladium-dicyclohexyl(2',4',6'-triisopropylbiphenyl-2-yl)phosphan (1:1)], J. Am. Chem. Soc. 2010, 132, 14073-14075

### HPLC-. LC/MS- und GC-Methoden:

Methode 1: Instrument: Waters ACQUITY SQD UPLC System; Säule: Waters Acquity UPLC HSS T3 1.8 µ 50 mm x 1 mm; Eluent A: 1 l Wasser + 0.25 ml 99%ige Ameisensäure, Eluent B: 1 l Acetonitril + 0.25 ml 99%ige Ameisensäure; Gradient: 0.0 min 90% A → 1.2 min 5% A → 2.0 min 5% A; Ofen: 50°C; Fluss: 0.40 ml/min; UV-Detektion: 208-400 nm.
Methode 2: Instrument: Waters ACQUITY SQD UPLC System; Säule: Waters Acquity UPLC HSS T3 1.8 µ 50 mm x 1 mm; Eluent A: 1 l Wasser + 0.25 ml 99%ige Ameisensäure, Eluent B: 1 l Acetonitril + 0.25 ml 99%ige Ameisensäure; Gradient: 0.0 min 95% A → 6.0 min 5% A → 7.5 min 5% A; Ofen: 50°C; Fluss: 0.35 ml/min; UV-Detektion: 210-400 nm.
Methode 3: Instrument: Micromass Quattro Premier mit Waters UPLC Acquity; Säule: Thermo Hypersil GOLD 1.9 µ 50 mm x 1 mm; Eluent A: 1 l Wasser + 0.5 ml 50%ige Ameisensäure, Eluent B: 1 l Acetonitril + 0.5 ml 50%ige Ameisensäure; Gradient: 0.0 min 97% A → 0.5 min 97% A → 3.2 min 5% A → 4.0 min 5% A; Ofen: 50°C; Fluss: 0.3 ml/min; UV-Detektion: 210 nm.
Methode 4: Instrument MS: Waters (Micromass) Quattro Micro; Instrument HPLC: Agilent 1100 Serie; Säule: YMC-Triart C18 3µ 50 mm x 3 mm; Eluent A: 1 l Wasser + 0.01 mol Ammoniumcarbonat, Eluent B: 1 l Acetonitril; Gradient: 0.0 min 100% A → 2.75 min 5% A → 4.5 min 5% A; Ofen: 40°C; Fluss: 1.25 ml/min; UV-Detektion: 210 nm.
Methode 5: Instrument MS: Waters (Micromass) QM; Instrument HPLC: Agilent 1100 Serie; Säule: Agient ZORBAX Extend-C18 3.0 mm x 50 mm 3.5-Micron; Eluent A: 1 l Wasser + 0.01 mol Ammoniumcarbonat, Eluent B: 1 l Acetonitril; Gradient: 0.0 min 98% A → 0.2 min 98% A → 3.0 min 5% A → 4.5 min 5% A; Ofen: 40°C; Fluss: 1.75 ml/min; UV-Detektion: 210 nm.
Methode 6: Instrument MS: Waters (Micromass) ZQ; Instrument HPLC: Agilent 1100 Serie; Säule: Agient ZORBAX Extend-C18 3.0 mm x 50 mm 3.5-Micron; Eluent A: 1 l Wasser + 0.01 mol Ammoniumcarbonat, Eluent B: 1 l Acetonitril; Gradient: 0.0 min 98% A → 0.2 min 98% A → 3.0 min 5% A → 4.5 min 5% A; Ofen: 40°C; Fluss: 1.75 ml/min; UV-Detektion: 210 nm.
Methode 7: Instrument: Thermo DFS, Trace GC Ultra; Säule: Restek RTX-35, 15 m x 200 µm x 0.33 µm; konstanter Fluss mit Helium: 1.20 ml/min; Ofen: 60°C; Inlet: 220°C; Gradient: 60°C, 30°C/min → 300°C (3.33 min halten).
Methode 8: Instrument: Agilent MS Quad 6150; HPLC: Agilent 1290; Säule: Waters Acquity UPLC HSS T3 1.8 µ 50 mm x 2.1 mm; Eluent A: 1 l Wasser + 0.25 ml 99%ige Ameisensäure, Eluent B: 1 l Acetonitril + 0.25 ml 99%ige Ameisensäure; Gradient: 0.0 min 90% A → 0.3 min 90% A → 1.7 min 5% A → 3.0 min 5% A; Ofen: 50°C; Fluss: 1.20 ml/min; UV-Detektion: 205-305 nm.
Methode 9: Instrument: Thermo Scientific DSQII, Thermo Scientific Trace GC Ultra; Säule: Restek RTX-35MS, 15 m x 200 µm x 0.33 µm; konstanter Fluss mit Helium: 1.20 ml/min; Ofen: 60°C; Inlet: 220°C; Gradient: 60°C, 30°C/min → 300°C (3.33 min halten).
Methode 10: Gerätetyp MS: Thermo Scientific FT-MS; Gerätetyp UHPLC+: Thermo Scientific UltiMate 3000; Säule: Waters, HSST3, 2.1 mm x 75 mm, C18 1.8 µm; Eluent A: 1 l Wasser + 0.01% Ameisensäure; Eluent B: 1 l Acetonitril + 0.01% Ameisensäure; Gradient: 0.0 min 10% B → 2.5 min 95% B → 3.5 min 95% B; Ofen: 50°C; Fluss: 0.90 ml/min; UV-Detektion: 210 nm/ Optimum Integration Path 210-300 nm.
Methode 11: Instrument MS: Waters (Micromass) Quattro Micro; Instrument Waters UPLC Acquity; Säule: Waters BEH C18 1.7 µ 50 mm x 2.1 mm; Eluent A: 1 l Wasser + 0.01 mol Ammoniumformiat, Eluent B: 1 l Acetonitril; Gradient: 0.0 min 95% A → 0.1 min 95% A → 2.0 min 15% A → 2.5 min 15% A → 2.51 min 10% A → 3.0 min 10% A; Ofen: 40°C; Fluss: 0.5 ml/min; UV-Detektion: 210 nm.

Mikrowelle: Als Mikrowellenreaktor wurde ein "single mode" Gerät vom Typ Emrys™ Optimizer verwendet.

Bei Aufreinigungen von erfindungsgemäßen Verbindungen per präparativer HPLC nach den oben beschriebenen Methoden, in denen die Elutionsmittel Zusatzstoffe wie beispielsweise Trifluoressigsäure, Ameisensäure oder Ammoniak enthalten, können die erfindungsgemäßen Verbindungen in Salz-Form, beispielsweise als Trifluoracetat, Formiat oder Ammonium-Salz anfallen, sofern die erfindungsgemäßen Verbindungen eine ausreichend basische beziehungsweise saure Funktionalität enthalten. Ein solches Salz kann durch verschiedene dem Fachmann bekannte Methoden in die entsprechende freie Base beziehungsweise Säure überführt werden.

Wenn bei den im Folgenden beschriebenen Synthese-Intermediaten und Ausführungsbeispielen der Erfindung eine Verbindung in der Form eines Salzes der korrespondierenden Base beziehungsweise Säure aufgeführt ist, so ist die exakte stöchiometrische Zusammensetzung eines solchen Salzes, wie es nach dem jeweiligen Herstell- und/oder Reinigungsverfahren erhalten wurde, in der Regel nicht bekannt. Sofern nicht genauer spezifiziert, sind daher Namens- und Strukturformel-Zusätze wie beispielsweise "Hydrochlorid", "Trifluoracetat", "Natrium-Salz" bzw. "x HCl", "x CF₃COOH", "x Na⁺" bei solchen Salzen nicht stöchiometrisch zu verstehen, sondern haben allein deskriptiven Charakter bezüglich der enthaltenen salzbildenden Komponenten.

Sinngemäß gleiches gilt für den Fall, dass Synthese-Intermediate oder Ausführungsbeispiele oder Salze hiervon nach den beschriebenen Herstell- und/oder Reinigungsverfahren in Form von Solvaten, wie beispielsweise Hydraten, erhalten wurden, deren stöchiometrische Zusammensetzung (sofern definierter Art) nicht bekannt ist.

### Ausgangsverbindungen

### Allgemeine Methode 1A: Darstellung einer Boronsäure

Eine Lösung des entsprechenden Pyridinderivates in Tetrahydrofuran (ca. 3 ml/mmol) wurde bei -78°C mit Lithiumdiisopropylamid (2M in Tetrahydrofuran/Heptan/Ethylbenzol) versetzt, 2 bis 4 h gerührt und anschließend zügig mit Triisopropylborat versetzt. Das Reaktionsgemisch wurde für weitere 2 bis 3 h bei -78°C gehalten und anschließend langsam über Nacht auf RT aufgetaut. Nach Zugabe von Wasser wurde das Tetrahydrofuran im Vakuum entfernt und die wässrige Phase zweimal mit Essigsäureethylester extrahiert. Die wässrige Phase wurde mit wässriger Salzsäure (2M) angesäuert, wobei in der Regel ein Niederschlag ausfiel, der filtriert, mit Wasser gewaschen und getrocknet wurde. Die wässrige Phase wurde dreimal mit Essigsäureethylester extrahiert. Die vereinigten, organischen Phasen wurden getrocknet (Natrium- oder Magnesiumsulfat), filtriert und im Vakuum eingeengt.

### Allgemeine Methode 2A: Suzuki-Kupplung

In einem ausgeheizten, mit Argon gespülten Kolben wurden 1.0 eq. der entsprechenden Boronsäuren, 1.0 eq. des Arylbromides oder Aryliodides, 3.0 eq. Kaliumcarbonat und 0.1 eq. [1,1-Bis-(diphenylphosphino)-ferrocen]-palladium(II)chlorid-Monodichlormethan-addukt oder Tetrakis(triphenylphosphin)palladium(0) vorgelegt. Der Kolben wurde anschließend dreimal evakuiert und immer wieder mit Argon belüftet. Das Reaktionsgemisch wurde mit Dioxan (ca. 6 ml/mmol) versetzt und für mehrere Stunden bis zur weitgehend vollständigen Umsetzung bei 110°C gerührt. Das Reaktionsgemisch wurde anschließend über Celite filtriert, das Filtrat im Vakuum eingeengt. Der Rückstand wurde mit Wasser versetzt. Nach Zugabe von Essigsäureethylester und Phasentrennung wurde die organische Phase einmal mit Wasser und einmal mit gesättigter, wässriger Natriumchlorid-Lösung gewaschen, getrocknet (Natrium- oder Magnesiumsulfat), filtriert und im Vakuum eingeengt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 3A: Methoxypyridin Spaltung

Eine Lösung des entsprechenden Methoxypyridins in Dimethylformamid (10-12.5 ml/mmol) wurde mit 20 eq. Pyridinium-Hydrochlorid oder Pyridinium-Hydrobromid versetzt und bei 100°C für mehrere Stunden bis Tage gerührt, eventuell mit weiterem Pyridinium-Hydrochlorid oder Pyridinium-Hydrobromid versetzt, bis zur weitgehend vollständigen Umsetzung. Anschließend wurde die Reaktionslösung im Vakuum eingeengt und der Rückstand mit Wasser verrührt. Der anfallende Niederschlag wurde filtriert, mit Wasser gewaschen und im Vakuum getrocknet.

### Allgemeine Methode 4A: N-Alkylierung von 2-Pyridinon-Derivaten mit den entsprechenden 2-Brom- oder 2-Chlorpropansäureesterderivaten in Gegenwart von Kaliumcarbonat

Eine Lösung von 1.0 eq. des entsprechenden 2-Pyridinon-Derivates in Dimethylformamid (5-10 ml/mmol) wurde unter Argon bei RT mit 1.2 eq. des entsprechenden 2-Brom- oder 2-Chlorpropansäureesterderivates und 1.5 eq. Kaliumcarbonat versetzt und bei 100°C gerührt. Nach Entfernen des Dimethylformamids und Zugabe von Wasser/Essigsäureethylester und Phasentrennung wurde die organische Phase mit Wasser und mit gesättigter, wässriger Natriumchlorid-Lösung gewaschen, getrocknet (Natrium- oder Magnesiumsulfat), filtriert und im Vakuum eingeengt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 5B: Amid-Kupplung mit T3P/Pyridin

Eine Lösung der entsprechenden Carbonsäure oder Carbonsäurehydrochlorid (1 eq.) und des entsprechenden Amins oder Aminhydrochlorid (1.1-1.9 eq.) in Pyridin (ca. 0.1M) wurde auf 60°C erwärmt und tropfenweise mit T3P (50%ig in Essigsäureethylester, 1.5-15 eq.) versetzt. Alternativ wurde T3P bei RT hinzugefügt und danach bei RT gerührt oder auf 50 bis 90°C erhitzt. Nach 1 bis 20 h wurde das Reaktionsgemisch auf RT gekühlt und entweder direkt mittels präparativer RP-HPLC (Wasser-Acetonitril-Gradient oder Wasser-Methanol-Gradient) aufgereinigt, oder mit Wasser und Essigsäureethylester versetzt. Die wässrige Phase wurde mit Essigsäureethylester extrahiert. Die vereinigten organischen Phasen wurden mit wässriger Puffer-Lösung (pH=5), mit gesättigter, wässriger Natriumhydrogencarbonat-Lösung und mit gesättigter, wässriger Natriumchlorid-Lösung gewaschen, über Natriumsulfat getrocknet und im Vakuum eingeengt. Das Rohprodukt wurde gegebenenfalls anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 6A: Verseifung eines tert.-Butylesters oder eines Boc-geschützten Amins mittels TFA

Eine Lösung von 1.0 eq. des entsprechenden *tert*.-Butylesterderivates in Dichlormethan (ca. 5-10 ml/mmol) wurde bei RT mit 20 eq. TFA versetzt und bei RT für 1 bis 8 h gerührt. Anschließend wurde das Reaktionsgemisch im Vakuum eingeengt, der Rückstand mehrmals mit Dichlormethan und Toluol coevaporiert und im Vakuum getrocknet. Das Rohprodukt wurde gegebenenfalls anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 6B: Verseifung eines tert.-Butylesters mittels Chlorwasserstoff in Dioxan

Eine Lösung von 1.0 eq. des entsprechenden *tert*.-Butylesterderivates in 4M Chlorwasserstoff in Dioxan (Konzentration des *tert*.-Butylesterderivates ca. 0.1M) wurde entweder bei RT für 2 bis 48 h gerührt oder in ein Ultraschallbad für 2 bis 5 h behandelt. Anschließend wurde das Reaktionsgemisch im Vakuum eingeengt, der Rückstand mehrmals mit Tetrahydrofuran coevaporiert und im Vakuum getrocknet. Das Rohprodukt wurde ohne weitere Reinigung umgesetzt.

### Allgemeine Methode 6C: Verseifung eines Methyl-/Ethyl- oder Benzylesters mit Lithiumhydroxid

Eine Lösung von 1.0 eq. des entsprechenden Methyl- oder Ethylesters in Tetrahydrofuran/Wasser (3:1, ca. 7-15 ml/mmol) wurde bei RT mit Lithiumhydroxid (2-4 eq.) versetzt. Das Reaktionsgemisch wurde bei RT bis 60°C gerührt und anschließend mit wässriger Salzsäure (1N) auf pH 1 gestellt. Nach Zugabe von Wasser/Essigsäureethylester und Phasentrennung wurde die wässrige Phase dreimal mit Essigsäureethylester extrahiert. Die vereinigten, organischen Phasen wurden getrocknet (Natriumsulfat oder Magnesiumsulfat), filtriert und im Vakuum eingeengt. Das Rohprodukt wurde anschließend entweder mittels Normalphasen-Chromatographie (Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 7A: Darstellung von Triflaten

Eine Lösung des entsprechenden Alkohols (1 eq.) wurde in Dichlormethan (0.1M) vorgelegt und bei -20°C nacheinander mit Lutidin (1.1-1.5 eq.) oder Triethylamin (1.1-1.5 eq.) und mit Trifluormethansulfonsäureanhydrid (1.05-1.5 eq.) versetzt. Das Reaktionsgemisch wurde für 1 h bei -20°C nachgerührt und anschließend mit der dreifachen Menge (bezogen aufs Reaktionsvolumen) Methyl-*tert*.-butylether verdünnt. Die organische Phase wurde dreimal mit einer 3:1-Mischung aus gesättigter, wässriger Natriumchlorid-Lösung/IN Salzsäure und abschließend mit gesättigter, wässriger Natriumhydrogencarbonat-Lösung gewaschen, getrocknet (Natrium- oder Magnesiumsulfat), filtriert und das Lösungsmittel unter reduziertem Druck entfernt. Das Rohprodukt wurde ohne weitere Aufreinigung im nächsten Schritt eingesetzt.

### Allgemeine Methode 8B: Alkylierung von Essigsäureestern mit Triflaten

Eine Lösung des entsprechenden Essigsäureesters (1 eq.) in Tetrahydrofuran (0.1-0.2M) wurde unter Argon bei -78°C tropfenweise mit Bis-(trimethylsilyl)-lithiumamid (1.0M in THF, 1.1-1.3 eq.) versetzt und 15 min gerührt. Anschließend wurde das entsprechende Alkyltriflat (1.5-2.0 eq.) als Reinsubstanz oder Lösung in THF hinzugegeben. Die resultierende Reaktionsmischung wurde 15 min bei -78°C und 1 h bei RT nachgerührt. Das Reaktionsgemisch wurde mit gesättigter, wässriger Ammoniumchlorid-Lösung versetzt. Nach Phasentrennung wurde die wässrige Phase mit Essigsäureethylester extrahiert. Die vereinigten, organischen Phasen wurden getrocknet (Natriumsulfat oder Magnesiumsulfat), filtriert und unter reduziertem Druck eingeengt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 9A: Nitro-Reduktion mit Eisen

Die entsprechende Nitroverbindung wurde in einem Ethanol/Wasser-Gemisch (5:1) gelöst (ca. 2-3M) und mit konzentrierter Salzsäure (0.5-1 eq.) und Eisenpulver (3-8 eq.) versetzt. Die Reaktionsmischung wurde auf 80 bis 100°C erhitzt bis zur vollständigen Umsetzung (ca. 1 bis 6 h). Das Reaktionsgemisch wurde anschließend heiß über Kieselgur filtriert. Der Filterkuchen wurde mit Methanol gewaschen und das Filtrat wurde im Vakuum eingeengt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Beispiel 1.1A

### 5-Nitrothiophen-2-carboxamid

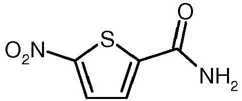

Nach der allgemeinen Methode 5B wurden 250 mg (1.44 mmol) 5-Nitrothiophen-2-carbonsäure und 232 mg (4.33 mmol, 1.5 eq.) Ammoniumchlorid umgesetzt. Nach Aufarbeitung, wurde das Rohprodukt ohne weitere Reinigung in die nächste Stufe eingesetzt. Ausbeute: 184 mg (90% Reinheit, 67% d. Th.)
LC/MS [Methode 8]: Rₜ = 0.70 min; MS (ESIpos): m/z = 173 (M+H)⁺,
¹H-NMR (400 MHz, DMSO-d₆): δ [ppm] = 8.42 (bs, 1H), 8.13 (d, 1H), 7.95 (bs, 1H), 7.78 (d, 1H).

### Beispiel 1.1B

### 5-Aminothiophen-2-carboxamid

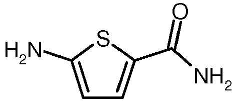

Nach der allgemeinen Methode 9A wurden 166 mg (90% Reinheit, 0.87 mmol) 5-Nitrothiophen-2-carboxamid umgesetzt. Das Rohprodukt wurde mittels Normalphasen-Chromatographie (Eluent: Dichlormethan/Methanol 5-10%) aufgereinigt. Ausbeute: 59 mg (87% Reinheit, 42% d. Th.)
LC/MS [Methode 5]: Rₜ = 0.43 min; MS (ESIpos): m/z = 143 (M+H)⁺

### Beispiel 1.2A

### 3-Fluorthiophen-2-carbonsäure

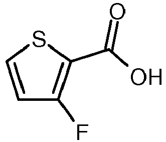

Eine Lösung von 5.0 g (39.02 mmol) Thiophen-2-carbonsäure in 175 ml Tetrahydrofuran wurde unter Argon bei -78°C tropfenweise mit 53.6 ml (1.6M in Hexan, 2.2 eq.) n-Butyllithium versetzt und 30 min gerührt. Anschließend wurde eine Lösung von 15.16 g (46.63 mmol, 1.2 eq.) N-Fluor-N-(phenylsulfonyl)benzolsulfonamid in 87 ml Tetrahydrofuran hinzugegeben. Die resultierende Reaktionsmischung wurde 16 h nachgerührt, während die Reaktionsmischung bis RT erwärmt wurde. Das Reaktionsgemisch wurde mit 43 ml Salzsäure (1M in Wasser) versetzt. Nach Phasentrennung wurde die wässrige Phase mit Diethylether extrahiert. Die vereinigten, organischen Phasen wurden mit gesättigter, wässriger Natriumchlorid-Lösung gewaschen. Die organische Phase wurde getrocknet (Natriumsulfat), filtriert und unter reduziertem Druck eingeengt. Das Rohprodukt wurde anschließend mittels Normalphasen-Chromatographie (Eluent: Cyclohexan-Essigsäureethylester (10-50%) Gemische) aufgereinigt. Ausbeute 2.6g (70% Reinheit, 32% d. Th.).
LC/MS [Methode 1]: Rₜ = 0.46 min; MS (ESIneg): m/z = 145 (M-H)⁻,
¹H-NMR (400 MHz, DMSO-d₆): δ [ppm] = 13.21 (bs, 1H), 7.92-7.84 (m, 1H), 7.11 (d, 1H).

### Beispiel 1.2B

### 3-Fluorthiophen-2-carboxamid

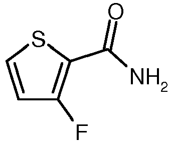

Eine Lösung von 2.60 g (70% Reinheit, 12.45 mmol) 3-Fluorthiophen-2-carbonsäure in 18 ml Dichlormethan und ein Tropfen N,N-Dimethylformamid wurde bei RT tropfenweise mit 1.58 ml (18.68 mmol, 1.5 eq.) Oxalylchlorid versetzt und 1 h gerührt. Das Lösungsmittel wurde unter reduziertem Druck eingeengt und der Rückstand wurde in 18 ml Dichlormethan gelöst. Eine Lösung von 40 ml Ammoniak (40% in Wasser, 37.4 mmol, 3 eq.) wurde tropfenweise bei RT hinzugegeben. Die resultierende Reaktionsmischung wurde 1 h nachgerührt. Nach Phasentrennung wurde die wässrige Phase mit Dichlormethan extrahiert. Die vereinigten, organischen Phasen wurden getrocknet (Natriumsulfat), filtriert und unter reduziertem Druck eingeengt. Das Rohprodukt wurde anschließend mittels Normalphasen-Chromatographie (Eluent: Cyclohexan-Essigsäureethylester (50%) Gemische) aufgereinigt. Ausbeute 1.5 g (83% d. Th.).
LC/MS [Methode 5]: Rₜ = 1.18 min; MS (ESIpos): m/z = 146 (M+H)⁺,
¹H-NMR (400 MHz, DMSO-d₆): δ [ppm] 7.76 (dd, 1H), 7.65 (bs, 1H), 7.28 (bs, 1H), 7.07 (d, 1H).

### Beispiel 1.2C

### 3-Fluor-5-nitrothiophen-2-carboxamid

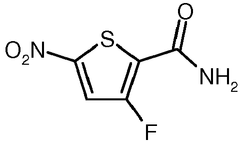

Zu einer Lösung von 1.50 g (10.33 mmol, 1 eq.) 3-Fluorthiophen-2-carboxamid in 96.4 ml konzentrierter Schwefelsäure wurde bei 0°C 6.27 g (62.0 mmol, 6 eq.) Kaliumitrat in drei Teilen dazugegeben. Nach 5 min wurde die Reaktionsmischung bei RT 1 h gerührt. Anschließend wurde die Reaktionsmischung mit Eis versetzt. Diese Mischung wurde mit Essigsäureethylester extrahiert. Die vereinigten, organischen Phasen wurden mit gesättigter, wässriger Natriumhydrogencarbonat-Lösung und mit gesättigter, wässriger Natriumchlorid-Lösung gewaschen. Die organische Phase wurde getrocknet (Natriumsulfat), filtriert und unter reduziertem Druck eingeengt. Das Rohprodukt wurde anschließend mittels Normalphasen-Chromatographie (Eluent: Cyclohexan-Essigsäureethylester (10-50%) Gemische) aufgereinigt. Ausbeute: 820 mg (41% d. Th.)
¹H-NMR (400 MHz, DMSO-d₆): δ [ppm] = 8.96 (d, 1H), 8.03 (bs, 1H), 7.69 (bs, 1H).

### Beispiel 1.2D

### 5-Amino-3-fluorthiophen-2-carboxamid

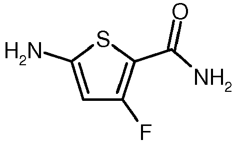

Nach der allgemeinen Methode 9A wurden 800 mg (4.21 mmol) 3-Fluor-5-nitrothiophen-2-carboxamid umgesetzt. Ausbeute: 700 mg (83% Reinheit, 86% d. Th.)
LC/MS [Methode 1]: Rₜ = 0.19 min; MS (ESIpos): m/z = 161 (M+H)⁺,
¹H-NMR (400 MHz, DMSO-d₆): δ [ppm] = 7.53 (bs, 1H), 7.15 (bs, 1H), 6.30 (d, 1H), 4.98 (s, 2H).

### Beispiel 1.3A

### N-Methyl-5-nitrothiophen-2-carboxamid

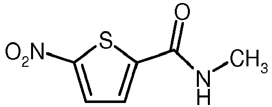

Nach der allgemeinen Methode 5B wurden 1.0 g (5.77 mmol) 5-Nitrothiophen-2-carbonsäure und 1.17 g (17.3 mmol, 3 eq.) Methylamin-Hydrochlorid umgesetzt. Nach Aufarbeitung, wurde das Rohprodukt ohne weitere Reinigung in die nächste Stufe eingesetzt. Ausbeute: 980 mg (91% d. Th.)
LC/MS [Methode 11]: Rₜ = 1.13 min; MS (ESIpos): m/z = 187 (M+H)⁺,
¹H-NMR (400 MHz, DMSO-d₆): δ [ppm] = 8.94 (bs, 1H), 8.13 (d, 1H), 7.73 (d, 1H), 2.80 (d, 3H).

### Beispiel 1.3B

### 5-Amino-N-methylthiophen-2-carboxamid

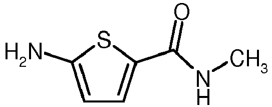

Nach der allgemeinen Methode 9A wurden 980 mg (5.26 mmol) *N*-Methyl-5-nitrothiophen-2-carboxamid umgesetzt. Das Rohprodukt wurde mittels Normalphasen-Chromatographie (Eluent: Dichlormethan/Methanol 0-10%) aufgereinigt. Ausbeute: 500 mg (60% d. Th.)
LC/MS [Methode 11]: Rₜ = 0.61 min; MS (ESIpos): m/z = 157 (M+H)⁺
¹H-NMR (400 MHz, DMSO-d₆): δ [ppm] = 7.85-7.72 (m, 1H), 7.20 (d, 1H), 6.13 (s, 2H), 5.79 (d, 1H), 2.67 (d, 3H).

### Beispiel 2.1A

### 2,5-Dimethoxypyridin-4-ylboronsäure

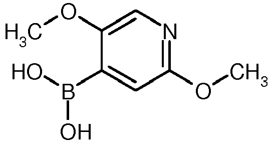

Nach der allgemeinen Methode 1A wurden 11.53 g (82.9 mmol) 2,5-Dimethoxypyridin umgesetzt. Nach Ansäuern der wässrigen Phase fiel das gewünschte Produkt als Niederschlag aus. Ausbeute: 9.53 g (61% d. Th.)
LC/MS [Methode 1]: Rₜ = 0.47 min; MS (ESIpos): m/z = 184 (M+H)⁺

### Beispiel 2.1B

### 4-Chlor-2-(2,5-dimethoxypyridin-4-yl)benzonitril

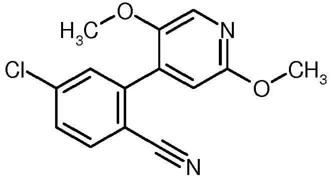

Nach der allgemeinen Methode 2A wurden 7.87 g (95% Reinheit, 40.86 mmol) 2,5-Dimethoxypyridin-4-ylboronsäure mit 8.85 g (40.86 mmol) 2-Brom-4-chlorbenzonitril in Gegenwart von [1,1-Bis-(diphenylphosphino)-ferrocen]-palladium(II)chlorid-dichlormethan-Monoaddukt umgesetzt. Ausbeute: 6.23 g (92% Reinheit, 51% d. Th.)
LC/MS [Methode 1]: Rₜ = 1.08 min; MS (ESIpos): m/z = 275 (M+H)⁺

### Beispiel 2.1C

### 4-Chlor-2-(5-methoxy-2-oxo-1,2-dihydropyridin-4-yl)benzonitril

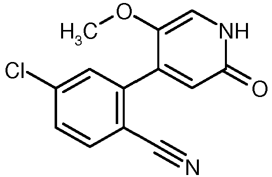

Nach der allgemeinen Methode 3A wurden 7.23 g (92% Reinheit, 24.21 mmol) 4-Chlor-2-(2,5-dimethoxypyridin-4-yl)benzonitril mit Pyridinium-Hydrochlorid umgesetzt. Ausbeute: 6.66 g (91% Reinheit, 96% d. Th.)
LC/MS [Methode 1]: Rₜ = 0.76 min; MS (ESIpos): m/z = 261 (M+H)⁺
¹H-NMR (400 MHz, DMSO-d₆): δ [ppm] = 11.45 (br. s, 1H), 7.98 (d, 1H), 7.75-7.67 (m, 2H), 7.29 (br. s, 1H), 6.43 (s, 1H), 3.64 (s, 3H).

### Beispiel 3.1A

### 2-Methoxyethyl-trifluormethansulfonat

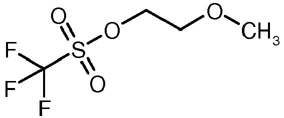

Eine Lösung von 4.0 g (52.56 mmol, 1 eq.) 2-Methoxyethanol und 8.79 ml (63.1 mmol, 1.2 eq.) Triethylamin in 40 ml Diethylether wurde unter Argon bei -78°C tropfenweise mit 9.29 ml (55.19 mmol, 1.05 eq.) Trifluormethansulfonsäureanhydrid versetzt und 45 min gerührt. Die resultierende Suspension wurde bei -78°C durch eine Kanüle filtriert. Das Filtrat wurde mit einem Gemisch aus gesättigter, wässriger Ammoniumchlorid-Lösung und 1M Salzsäure (3:1) gewaschen. Nach Phasentrennung wurde die wässrige Phase mit Diethylether extrahiert. Die vereinigten, organischen Phasen wurden getrocknet (Natriumsulfat), filtriert und unter reduziertem Druck eingeengt. Das Rohprodukt wurde ohne weitere Reinigung eingesetzt. Ausbeute: 9.50 g (95% Reinheit, 82% d. Th.)
¹H-NMR (400 MHz, DMSO-d₆): δ [ppm] = 4.46-4.37 (m, 2H), 3.66-3.54 (m, 2H), 3.33 (s, 3H).

### Beispiel 3.1B

### 2-[4-(5-Chlor-2-cyanophenyl)-5-methoxy-2-oxopyridin-1(2H)-yl]-4-methoxybutansäure-tert.-butylester (Racemat)

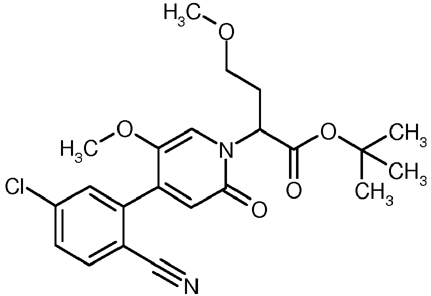

8.09 g (21.6 mmol) [4-(5-Chlor-2-cyanophenyl)-5-methoxy-2-oxopyridin-1(2*H*)-yl]essigsäure-*tert*.-butylester wurden in 180 ml THF vorgelegt und auf -78°C gekühlt. Tropfenweise wurden 23.7 ml Bis-(trimethylsilyl)-lithiumamid (1M in THF) zugegeben, und man ließ 15 min weiter rühren. Dann tropfte man 8.99 g (43.2 mmol) 2-Methoxyethyl-trifluormethansulfonat zu und ließ 15 min bei -78°C und weitere 45 min bei RT rühren. Man fügte dann gesättigte, wässrige Ammoniumchlorid-Lösung zu und extrahierte mehrfach mit Essigsäureethylester. Die vereinten organischen Phasen wurden über Natriumsulfat getrocknet und im Vakuum eingeengt. Man reinigte den Rückstand mittels Flash-Chromatographie (Kieselgel-50, Cyclohexan-Essigsäureethylester-Gradient). Ausbeute: 7.87 g (95% Reinheit, 80% d. Th.)
LC/MS [Methode 1]: Rₜ = 1.02 min; MS (ESIpos): m/z = 433 (M+H)⁺,
1H-NMR (400 MHz, DMSO-d₆): δ [ppm] = 8.01-7.96 (m, 1H), 7.76-7.69 (m, 2H), 7.37 (s, 1H), 6.48 (s, 1H), 5.11 (dd, 1H), 3.64 (s, 3H), 3.43-3.35 (m, 1H), 3.20 (s, 3H), 3.19-3.13 (m, 1H), 2.39-2.28 (m, 2H), 1.40 (s, 9H).

### Beispiel 3.1C

### 2-[4-(5-Chlor-2-cyanophenyl)-5-methoxy-2-oxopyridin-1(2H)-yl]-4-methoxybutansäure (Racemat)

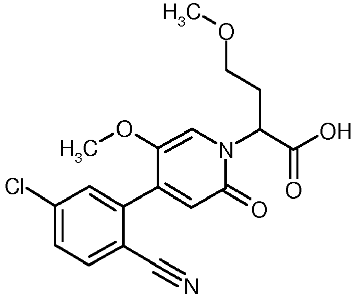

7.87 g (95% Reinheit, 17.3 mmol) 2-[4-(5-Chlor-2-cyanophenyl)-5-methoxy-2-oxopyridin-1(2*H*)-yl]-4-methoxybutansäure-*tert*.-butylester (Racemat) wurden in 175 ml Dichlormethan vorgelegt. Man setzte 42 ml (545 mmol) Trifluoressigsäure hinzu und ließ 3 h bei RT rühren. Man engte die Reaktionsmischung im Vakuum ein, nahm den Rückstand mehrmals in Dichlormethan auf und engte wieder ein. Zweimal wurde dann Toluol hinzugefügt und wieder eingeengt. Der Rückstand wurde mit Acetonitril verrührt und abgesaugt. Ausbeute 5.81 g (95% Reinheit, 84% d. Th.)
LC/MS [Methode 1]: Rₜ = 0.78 min; MS (ESIpos): m/z = 377 (M+H)⁺,
¹H-NMR (500 MHz, DMSO-d₆): δ [ppm] = 13.40-12.67 (m, 1H), 7.99 (d, 1H), 7.75 (d, 1H), 7.73 (dd, 1H), 7.43 (s, 1H), 6.48 (s, 1H), 5.14 (t, 1H), 3.64 (s, 3H), 3.41-3.36 (m, 1H), 3.19 (s, 3H), 3.13 (dt, 1H), 2.40-2.31 (m, 2H).

### Beispiel 4.1A

### [4-(5-Chlor-2-cyanophenyl)-5-methoxy-2-oxopyridin-1(2H)-yl]essigsäure-tert.-butylester

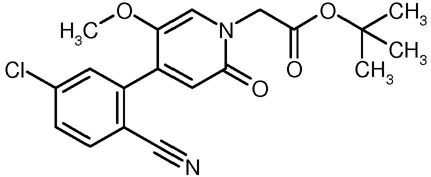

Nach der allgemeinen Methode 4A wurden 516 mg (91% Reinheit, 1.8 mmol) 4-Chlor-2-(5-methoxy-2-oxo-1,2-dihydropyridin-4-yl)benzonitril mit 1.2 eq. Bromessigsäure-*tert*.-butylester bei 100°C umgesetzt. Ausbeute: 464 mg (68% d. Th.)
LC/MS [Methode 1]: Rₜ = 1.00 min; MS (ESIpos): m/z = 375 (M+H)⁺

### Beispiel 5.1A

### Pyridin-2-ylmethyl-methansulfonat

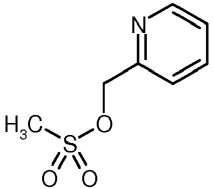

Eine Lösung von 4.00 g (36.65 mmol) Pyridin-2-ylmethanol und 11.24 ml (80.64 mmol, 2.2 eq.) Triethylamin in 122 ml Tetrahydrofuran wurde unter Argon bei 0°C tropfenweise mit einer Lösung von 2.84 ml (36.65 mmol, 1 eq.) Methansulfonsäurechlorid in 24 ml Tetrahydrofuran versetzt und 3 h gerührt. Tetrahydrofuran wurde unter reduziertem Druck entfernt. Das Rohprodukt wurde anschließend in Dichlormethan gelöst und die resultierende Mischung mit gesättigter, wässriger Natriumchlorid-Lösung gewaschen. Die organische Phase wurde getrocknet (Natriumsulfat), filtriert und unter reduziertem Druck eingeengt. Das Rohprodukt wurde anschließend mittels Normalphasen-Chromatographie (Eluent: Cyclohexan-Essigsäureethylester (20-50%) Gemische) aufgereinigt. Ausbeute 4.72 g (68% d. Th.)
LC/MS [Methode 3]: Rₜ = 0.98 min; MS (ESIpos): m/z = 188 (M+H)⁺,
¹H-NMR (400 MHz, DMSO-d₆): δ [ppm] = 8.67-8.48 (m, 1H), 7.89 (td, 1H), 7.54 (d, 1H), 7.42 (ddd, 1H), 5.30 (s, 2H), 3.28 (s, 3H).

### Beispiel 5.1B

### 2-[4-(5-Chlor-2-cyanophenyl)-5-methoxy-2-oxopyridin-1(2H)-yl]-3-(pyridin-2-yl)propansäure-tert.-butylester (Racemat)

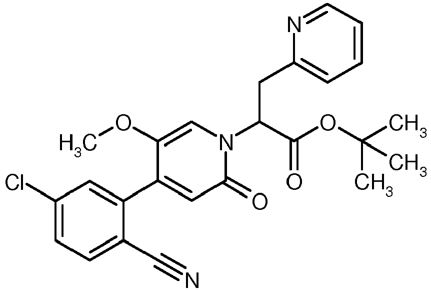

Eine Lösung von 1.50 g (4.00 mmol) [4-(5-Chlor-2-cyanophenyl)-5-methoxy-2-oxopyridin-1(2*H*)-yl]essigsäure-*tert*.-butylester in 30 ml Tetrahydrofuran wurde unter Argon bei -78°C tropfenweise mit 4.60 ml (1.0M in THF, 1.15 eq.) Bis-(trimethylsilyl)-lithiumamid versetzt und 15 min gerührt. Anschließend wurden 1.06 g (5.6 mmol, 1.4 eq.) Pyridin-2-ylmethyl-methansulfonat als Reinsubstanz hinzugegeben. Die resultierende Reaktionsmischung wurde 30 min bei -78°C und 1.5 h bei RT nachgerührt. Das Reaktionsgemisch wurde mit gesättigter, wässriger Ammoniumchlorid-Lösung versetzt. Nach Phasentrennung wurde die wässrige Phase mit Essigsäureethylester extrahiert. Die vereinigten, organischen Phasen wurden mit gesättigter, wässriger Natriumchlorid-Lösung gewaschen. Die organische Phase wurde getrocknet (Natriumsulfat), filtriert und unter reduziertem Druck eingeengt. Das Rohprodukt wurde anschließend mittels Normalphasen-Chromatographie (Eluent: Dichlormethan-Methanol (2-5%) Gemische) aufgereinigt. Ausbeute 1.99 g (93% Reinheit, 99% d. Th.)
LC/MS [Methode 1]: Rₜ = 0.97 min; MS (ESIpos): m/z = 466 (M+H)⁺.

### Beispiel 5.1C

### 2-[4-(5-Chlor-2-cyanophenyl)-5-methoxy-2-oxopyridin-1(2H)-yl]-3-(pyridin-2-yl)propansäure (Racemat)

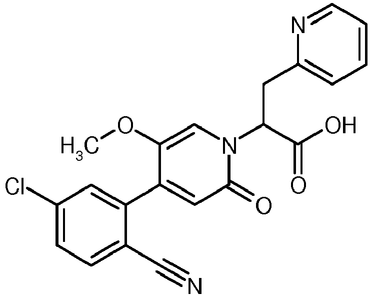

Gemäß allgemeiner Methode 6A wurden 1.99 g (93% Reinheit, 3.98 mmol) 2-[4-(5-Chlor-2-cyanophenyl)-5-methoxy-2-oxopyridin-1(*2H*)-yl]-3-(pyridin-2-yl)propansäure-*tert*.-butylester (Racemat) in 40 ml Dichlormethan mit 20 ml (259.6 mmol) TFA umgesetzt. Ausbeute: 220 mg (93% Reinheit, 13% d. Th.)
LC/MS [Methode 1]: Rₜ = 0.64 min; MS (ESIpos): m/z = 410 (M+H)⁺,
¹H-NMR (400 MHz, DMSO-d₆): δ [ppm] = 13.08 (br. s, 1H), 8.48 (d, 1H), 7.95 (d, 1H), 7.73-7.60 (m, 3H), 7.27 (s, 1H), 7.24-7.11 (m, 2H), 6.40 (s, 1H), 5.55 (t, 1H), 3.66-3.57 (m, 2H), 3.49 (s, 3H).

### Beispiel 5.1D

### Methyl-5-({2-[4-(5-chlor-2-cyanphenyl)-5-methoxy-2-oxopyridin-1(2H)-yl]-3-(pyridin-2-yl)propanoyl}amino)thiophen-2-carboxylat (Racemat)

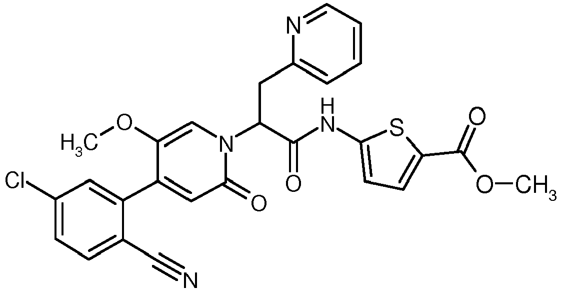

Gemäß allgemeiner Methode 5B wurden 100 mg (0.22 mmol) 2-[4-(5-Chlor-2-cyanphenyl)-5-methoxy-2-oxopyridin-1(2H)-yl]-3-(pyridin-2-yl)propansäure (Racemat) und 53 mg (0.34 mmol) Methyl-5-aminothiophen-2-carboxylat in 1 ml Pyridin umgesetzt. Ausbeute: 40 mg (32% d. Th.).
LC/MS [Methode 1]: Rₜ = 0.97 min; MS (ESIpos): m/z = 549 (M+H)⁺,
¹H-NMR (400 MHz, DMSO-d₆): δ [ppm] = 12.19 (s, 1H), 8.51-8.47 (m, 1H), 7.96 (d, 1H), 7.73-7.61 (m, 4H), 7.49 (s, 1H), 7.31 (d, 1H), 7.25-7.19 (m, 1H), 6.83 (d, 1H), 6.43 (s, 1H), 6.07 (dd, 1H), 3.78 (s, 3H), 3.74-3.64 (m, 2H), 3.61 (s, 3H).

### Beispiel 6.1A

### tert.-Butyl-2-[4-(5-chlor-2-cyanophenyl)-5-methoxy-2-oxopyridin-1(2H)-yl]-3-(pyridin-3-yl)propanoat (Racemat)

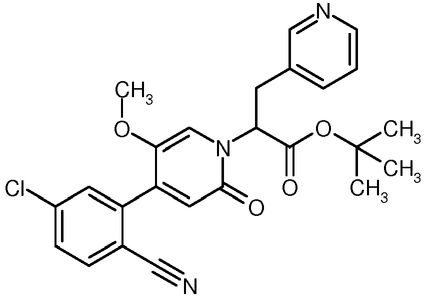

Eine Lösung von 2.40 g (6.40 mmol) [4-(5-Chlor-2-cyanophenyl)-5-methoxy-2-oxopyridin-1(2*H*)-yl]essigsäure-*tert*.-butylester in 48 ml Tetrahydrofuran wurde unter Argon bei -78°C tropfenweise mit 16.01 ml (1.0M in THF, 2.5 eq.) Bis-(trimethylsilyl)-lithiumamid versetzt und 20 min gerührt. Anschließend wurden 2.27 g (8.96 mmol, 1.4 eq.) 3-(Brommethyl)pyridin-Hydrobromid hinzugegeben. Die resultierende Reaktionsmischung wurde 30 min bei -78°C und 1.5 h bei RT nachgerührt. Das Reaktionsgemisch wurde mit gesättigter, wässriger Ammoniumchlorid-Lösung versetzt. Nach Phasentrennung wurde die wässrige Phase mit Essigsäureethylester extrahiert. Die vereinigten, organischen Phasen wurden mit gesättigter, wässriger Natriumchlorid-Lösung gewaschen. Die organische Phase wurde getrocknet (Natriumsulfat), filtriert und unter reduziertem Druck eingeengt. Das Rohprodukt wurde anschließend mittels Normalphasen-Chromatographie (Eluent: Cyclohexan/Essigsäureethylester (0-100%) Gemische) aufgereinigt. Ausbeute 2.0 g (90% Reinheit, 62% d. Th.)
LC/MS [Methode 1]: Rₜ = 0.84 min; MS (ESIpos): m/z = 466 (M+H)⁺,
¹H-NMR (400 MHz, DMSO-d₆): δ [ppm] = ppm 8.43 (d, 1H), 8.39 (dd, 1H), 7.96 (d, 1H), 7.71 (dd, 1H), 7.65 (d, 1H), 7.55-7.48 (m, 1H), 7.25 (dd, 1H), 7.21 (s, 1H), 6.46 (s, 1H), 5.32 (dd, 1H), 3.54-3.38 (m, 5H), 1.40 (s, 9H).

### Beispiel 6.1B

### 2-[4-(5-Chlor-2-cyanophenyl)-5-methoxy-2-oxopyridin-1(2H)-yl]-3-(pyridin-3-yl)propansäure-Hydrochlorid (Racemat)

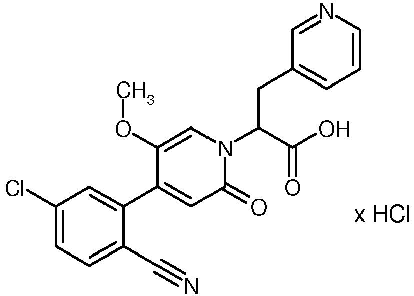

Gemäß allgemeiner Methode 6B wurden 2.0 g (3.86 mmol) tert-Butyl-2-[4-(5-chlor-2-cyanophenyl)-5-methoxy-2-oxopyridin-1(2H)-yl]-3-(pyridin-3-yl)propanoat (Racemat) und 39 ml einer Chlorwasserstoff in Dioxan (4M) Lösung umgesetzt. Ausbeute: 1.8 g (88% Reinheit, 92% d. Th.)
LC/MS [Methode 1]: Rₜ = 0.57 min; MS (ESIpos): m/z = 410 (M+H)⁺,
¹H-NMR (400 MHz, DMSO-d₆): δ [ppm] = 8.89 (s, 1H), 8.77 (d, 1H), 8.29 (d, 1H), 7.97 (d, 1H), 7.91 (dd, 1H), 7.77-7.63 (m, 2H), 7.40 (s, 1H), 6.44 (s, 1H), 5.50 (dd, 1H), 3.76 (dd, 1H), 3.65 (dd, 1H), 3.52 (s, 3H).

### Beispiel 7.1A

### tert.-Butyl-2-[4-(5-chlor-2-cyanophenyl)-5-methoxy-2-oxopyridin-1(2H)-yl]-3-(pyridin-4-yl)propanoat (Racemat)

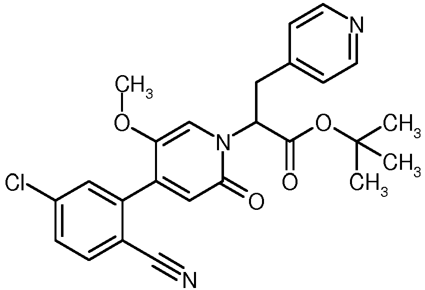

Eine Lösung von 2.25 g (6.00 mmol) [4-(5-Chlor-2-cyanophenyl)-5-methoxy-2-oxopyridin-1(2*H*)-yl]essigsäure-*tert*.-butylester in 48 ml Tetrahydrofuran wurde unter Argon bei -78°C tropfenweise mit 15.01 ml (1.0M in THF, 2.5 eq.) Bis-(trimethylsilyl)-lithiumamid versetzt und 20 min gerührt. Anschließend wurden 2.13 g (8.40 mmol, 1.4 eq.) 4-(Brommethyl)pyridin-Hydrobromid hinzugegeben. Die resultierende Reaktionsmischung wurde 30 min bei -78°C und 1.5 h bei RT nachgerührt. Das Reaktionsgemisch wurde mit gesättigter, wässriger Ammoniumchlorid-Lösung versetzt. Nach Phasentrennung wurde die wässrige Phase mit Essigsäureethylester extrahiert. Die vereinigten, organischen Phasen wurden mit gesättigter, wässriger Natriumchlorid-Lösung gewaschen. Die organische Phase wurde getrocknet (Natriumsulfat), filtriert und unter reduziertem Druck eingeengt. Das Rohprodukt wurde anschließend mittels Normalphasen-Chromatographie (Eluent: Cyclohexan/Essigsäureethylester (0-100%) Gemische) aufgereinigt. Ausbeute 2.0 g (87% Reinheit, 62% d. Th.)
LC/MS [Methode 1]: Rₜ = 0.76 min; MS (ESIpos): m/z = 466 (M+H)⁺,
¹H-NMR (400 MHz, DMSO-d₆): δ [ppm] = 8.46-8.37 (m, 2H), 7.97 (d, 1H), 7.71 (dd, 1H), 7.66 (d, 1H), 7.26-7.13 (m, 3H), 6.45 (s, 1H), 5.36 (t, 1H), 3.53-3.40 (m, 5H), 1.40 (s, 9H).

### Beispiel 7.1B

### 2-[4-(5-Chlor-2-cyanophenyl)-5-methoxy-2-oxopyridin-1(2H)-yl]-3-(pyridin-4-yl)propansäure-Hydrochlorid (Racemat)

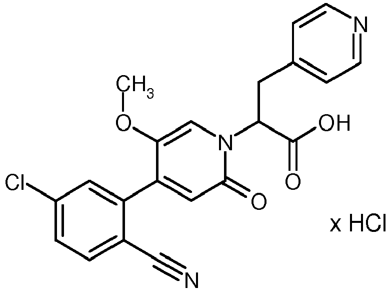

Gemäß allgemeiner Methode 6B wurden 2.1 g (3.97 mmol) tert.-Butyl-2-[4-(5-chlor-2-cyanophenyl)-5-methoxy-2-oxopyridin-1(2H)-yl]-3-(pyridin-4-yl)propanoat (Racemat) und 40 ml einer Chlorwasserstoff in Dioxan (4M) Lösung umgesetzt. Ausbeute: 1.9 g (93% Reinheit, 100% d. Th.)
LC/MS [Methode 1]: Rₜ = 0.58 min; MS (ESIpos): m/z = 410 (M+H)⁺,
¹H-NMR (400 MHz, DMSO-d₆): δ [ppm] = 8.78 (d, 2H), 7.97 (d, 1H), 7.92 (d, 2H), 7.75-7.66 (m, 2H), 7.42 (s, 1H), 6.44 (s, 1H), 5.63 (dd, 1H), 3.87 - 3.73 (m, 2H), 3.54 (s, 3H).

### Beispiel 8.1A

### 4-(5-Chlor-2-fluorphenyl)-2,5-dimethoxypyridin

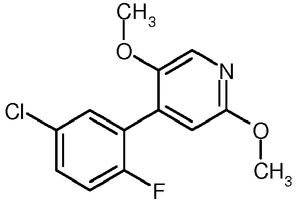

Nach der allgemeinen Methode 2A wurden 200 mg (1.09 mmol) (2,5-Dimethoxypyridin-4-yl)borsäure mit 274 mg (1.31 mmol) 2-Brom-4-chlor-1-fluorbenzol in Gegenwart von [1,1-Bis-(diphenylphosphino)-ferrocen]-palladium(II)chlorid-dichlormethan-Monoaddukt umgesetzt. Nach Aufarbeitung wurde das Rohprodukt anschließend mittels Flash-Chromatographie (Kieselgel-60, Cyclohexan-Dichlormethan-0-20% Gemische) aufgereinigt. Ausbeute: 150 mg (50% d. Th.)
LC/MS [Methode 1]: Rₜ = 1.11 min; MS (ESIpos): m/z = 268 (M+H)⁺,
¹H-NMR (400 MHz, DMSO-d₆): δ [ppm] = 8.00 (s, 1H), 7.53 (dd, 1H), 7.49 (dd, 1H), 7.40-7.32 (m, 1H), 6.80 (s, 1H), 3.84 (s, 3H), 3.78 (s, 3H)

### Beispiel 8.1B

### 4-(5-Chlor-2-fluorphenyl)-5-methoxypyridin-2(1H)-on

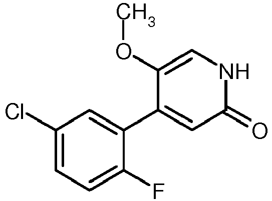

Nach der allgemeinen Methode 3A wurden 4.45 g (16.46 mmol) 4-(5-Chlor-2-fluorphenyl)-2,5-dimethoxypyridin mit Pyridinium-Hydrochlorid umgesetzt. Ausbeute: 4.00 g (80% Reinheit, 77% d. Th.)
LC/MS [Methode 1]: Rₜ = 0.75 min; MS (ESIpos): m/z = 254 (M+H)⁺.
¹H-NMR (400 MHz, DMSO-d₆): δ [ppm] = 11.32 (bs, 1H), 7.53 (ddd, 1H), 7.49-7.42 (m, 1H), 7.34 (t, 1H), 7.21 (s, 1H), 6.36 (s, 1H), 3.61 (s, 3H).

### Beispiel 8.1C

### tert.-Butyl-[4-(5-chlor-2-fluorphenyl)-5-methoxy-2-oxopyridin-1(2H)-yl]acetat

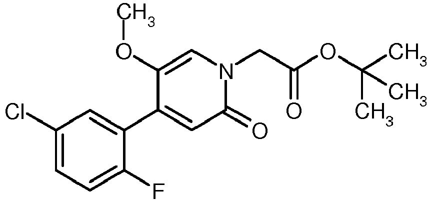

Nach der allgemeinen Methode 4A wurden 4.0 g (80% Reinheit, 12.62 mmol) 4-(5-Chlor-2-fluorphenyl)-5-methoxypyridin-2(1H)-on mit 1.2 eq. Bromessigsäure-*tert*.-butylester bei 100°C umgesetzt. Ausbeute: 3.85 g (83% d. Th.)
LC/MS [Methode 1]: Rₜ = 0.96 min, MS (ESIpos): m/z = 368 (M+H)⁺,
¹H-NMR (400 MHz, DMSO-d₆): δ [ppm] = 87.58-7.51 (m, 1H), 7.51-7.42 (m, 2H), 7.36 (dd, 1H), 6.42 (s, 1H), 4.58 (s, 2H), 3.59 (s, 3H), 1.44 (s, 9 H).

### Beispiel 8.1D

### tert.-Butyl-2-[4-(5-chlor-2-fluorphenyl)-5-methoxy-2-oxopyridin-1(2H)-yl]-3-(pyridin-2-yl)propanoat (Racemat)

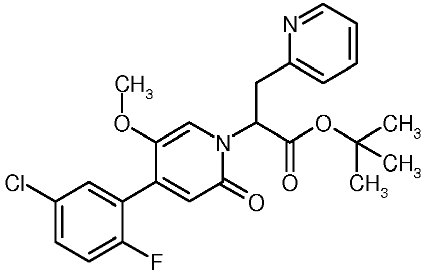

Eine Lösung von 1.00 g (2.72 mmol) *tert*.-Butyl-[4-(5-chlor-2-fluorphenyl)-5-methoxy-2-oxopyridin-1(2H)-yl]acetat in 20 ml Tetrahydrofuran wurde unter Argon bei -78°C tropfenweise mit 6.80 ml (1.0M in THF, 2.5 eq.) Bis-(trimethylsilyl)-lithiumamid versetzt und 15 min gerührt. Anschließend wurden 963 mg (3.81 mmol, 1.4 eq.) 2-(Brommethyl)pyridin-Hydrobromid hinzugegeben. Die resultierende Reaktionsmischung wurde 30 min bei -78°C und 1.5 h bei RT nachgerührt. Das Reaktionsgemisch wurde mit gesättigter, wässriger Ammoniumchlorid-Lösung versetzt. Nach Phasentrennung wurde die wässrige Phase mit Essigsäureethylester extrahiert. Die vereinigten, organischen Phasen wurden mit gesättigter, wässriger Natriumchlorid-Lösung gewaschen. Die organische Phase wurde getrocknet (Natriumsulfat), filtriert und unter reduziertem Druck eingeengt. Das Rohprodukt wurde anschließend mittels Normalphasen-Chromatographie (Eluent: Cyclohexan-Essigsäureethylester (0-70%) Gemische) aufgereinigt. Ausbeute 1.04 g (82% d. Th.)
LC/MS [Methode 1]: Rₜ = 0.97 min; MS (ESIpos): m/z = 459 (M+H)⁺.
¹H-NMR (400 MHz, DMSO-d₆): δ [ppm] = 8.49 (dd, 1H), 7.78-7.62 (m, 1H), 7.53 (ddd, 1H), 7.44 (dd, 1H), 7.33 (t, 1H), 7.29-7.12 (m, 3H), 6.37 (s, 1H), 5.60 (dd, 1H), 3.67-3.51 (m, 2H), 3.50 (s, 3H), 1.36 (s, 9H).

### Beispiel 8.1E

### 2-[4-(5-Chlor-2-fluorphenyl)-5-methoxy-2-oxopyridin-1(2H)-yl]-3-(pyridin-2-yl)propansäure-Hydrochlorid (Racemat)

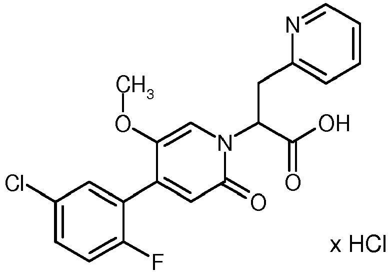

Gemäß allgemeiner Methode 6B wurden 1.04 g (2.22 mmol) tert-Butyl-2-[4-(5-chlor-2-cyanophenyl)-5-methoxy-2-oxopyridin-1(2H)-yl]-3-(pyridin-4-yl)propanoat (Racemat) und 22.4 ml einer Chlorwasserstoff in Dioxan (4M) Lösung umgesetzt. Ausbeute: 1.15 g (75% Reinheit, 88% d. Th.)
LC/MS [Methode 1]: Rₜ = 0.70 min; MS (ESIpos): m/z = 403 (M+H)⁺.
¹H-NMR (400 MHz, DMSO-d₆): δ [ppm] = 8.78 (d, 1H), 8.41-8.32 (m, 1H), 7.92-7.74 (m, 2H), 7.54 (ddd, 1H), 7.50-7.41 (m, 2H), 7.38-7.31 (m, 1H), 6.40 (s, 1H), 5.65-5.57 (m, 1H), 3.98 (dd, 1H), 3.74 (dd, 1H), 3.55 (s, 3H).

### Beispiel 9.1A

### (5-Chlor-2-methoxypyridin-4-yl)boronsäure

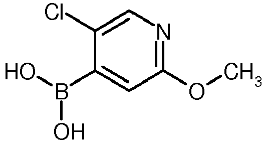

Nach der allgemeinen Methode 1A wurden 10.0 g (69.65 mmol) 5-Chlor-2-methoxypyridin umgesetzt. Das gewünschte Produkt fiel als Niederschlag beim Ansäuern mit Salzsäure (2N) aus.
Ausbeute: 10.44 g (91% Reinheit, 73% d. Th.)
LC/MS [Methode 1]: Rₜ = 0.50 min; MS (ESIpos): m/z = 188 (M+H)⁺
¹H-NMR (400 MHz, DMSO-d₆): δ [ppm] = 8.64 (bs, 2H), 8.12 (s, 1H), 6.81 (s, 1H), 3.82 (s, 3H).

### Beispiel 9.1B

### 4-Chlor-2-(5-chlor-2-methoxypyridin-4-yl)benzonitril

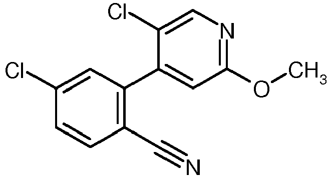

Nach der allgemeinen Methode 2A wurden 5.36 g (91% Reinheit, 26.03 mmol) 5-Chlor-2-methoxypyridin-4-ylboronsäure mit 5.12 g (23.66 mmol) 2-Brom-4-chlorbenzonitril in Gegenwart von [1,1-Bis-(diphenylphosphino)-ferrocen]-palladium(II)chlorid-dichlormethan-Monoaddukt umgesetzt. Nach Aufarbeitung wurde das Rohprodukt anschließend mittels Flash-Chromatographie (Kieselgel-60, Cyclohexan-Dichlormethan-Gemische) aufgereinigt. Ausbeute: 4.11 g (91% Reinheit, 52% d. Th.)
LC/MS [Methode 1]: Rₜ = 1.17 min; MS (ESIpos): m/z = 279 (M+H)⁺.

### Beispiel 9.1C

### 4-Chlor-2-(5-chlor-2-oxo-1,2-dihydropyridin-4-yl)benzonitril

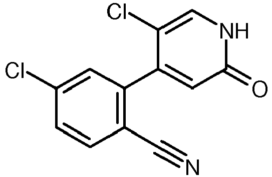

Nach der allgemeinen Methode 3A wurden 6.34 g (93% Reinheit, 21.12 mmol) 4-Chlor-2-(5-chlor-2-methoxypyridin-4-yl)benzonitril mit Pyridinium-Hydrochlorid umgesetzt. Ausbeute: 4.23 g (76% d. Th.)
LC/MS [Methode 1]: Rₜ = 0.82 min; MS (ESIpos): m/z = 265 (M+H)⁺.

### Beispiel 9.1D

### [5-Chlor-4-(5-chlor-2-cyanophenyl)-2-oxopyridin-1(2H)-yl]essigsäure-tert.-butylester

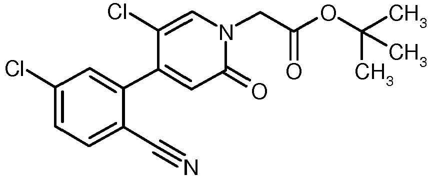

Nach der allgemeinen Methode 4A wurden 3.1 g (11.46 mmol) 4-Chlor-2-(5-chlor-2-oxo-1,2-dihydropyridin-4-yl)benzonitril mit 1.2 eq. Bromessigsäure-*tert*.-butylester bei 100°C umgesetzt.
Ausbeute: 3.65 g (84% d. Th.)
LC/MS [Methode 8]: Rₜ = 1.34 min, MS (ESIneg): m/z = 377 (M-H)⁻,
¹H-NMR (400 MHz, DMSO-d₆): δ [ppm] = 8.20 (s, 1H), 8.09-8.20 (m, 1H), 7.85-7.72 (m, 2H), 6.67 (s, 1H), 4.65 (s, 2H), 1.44 (s, 9H).

### Beispiel 10.1A

### 2-[5-Chlor-4-(5-chlor-2-cyanophenyl)-2-oxopyridin-1(2H)-yl]-4-methoxybutansäure-tert.-butylester (Racemat)

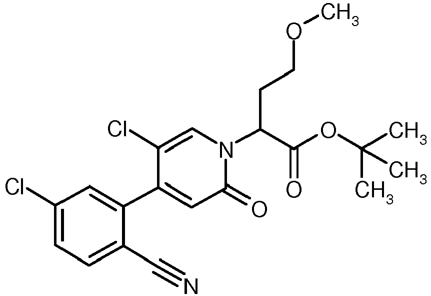

Nach der allgemeinen Methode 8B wurden 2.0 g (5.27 mmol) [5-Chlor-4-(5-chlor-2-cyanophenyl)-2-oxopyridin-1(2*H*)-yl]essigsäure-*tert*.-butylester in Gegenwart von 7.12 ml (7.12 mmol, 1.35 eq.) Bis-(trimethylsilyl)-lithiumamid (1M in THF) mit 1.33 g (95% Reinheit, 6.06 mmol, 1.15 eq.) 2-Methoxyethyl-trifluormethansulfonat umgesetzt. Ausbeute: 2.10 g (94% Reinheit, 86% d. Th.)
LC/MS [Methode 1]: Rₜ = 1.14 min; MS (ESIpos): m/z = 437 (M+H)⁺,
¹H-NMR (400 MHz, DMSO-d₆): δ [ppm] = 8.16-8.10 (m, 1H), 8.09-8.02 (m, 1H), 7.73-7.84 (m, 2H), 6.64 (s, 1H), 5.25-5.07 (m, 1H), 3.44-3.36 (m, 1H), 3.22-3.12 (m, 4H), 2.41-2.27 (m, 2H).

### Beispiel 10.1B

### 2-[5-Chlor-4-(5-chlor-2-cyanophenyl)-2-oxopyridin-1(2H)-yl]-4-methoxybutansäure (Racemat)

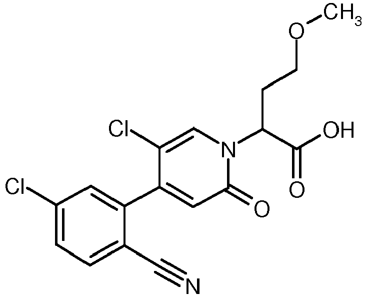

Nach der allgemeinen Methode 6A wurden 2.1 g (94% Reinheit, 4.51 mmol) 2-[5-Chlor-4-(5-chlor-2-cyanophenyl)-2-oxopyridin-1(*2H*)-yl]-4-methoxybutansäure-*tert*.-butylester (Racemat) umgesetzt. Ausbeute: 1.89 g (quant.)
LC/MS [Methode 1]: Rₜ = 0.85 min; MS (ESIpos): m/z = 381 (M+H)⁺,
¹H-NMR (400 MHz, DMSO-d₆): δ [ppm] = 13.19 (br. s, 1H), 8.15 (s, 1H), 8.05 (d, 1H), 7.82 (d, 1H), 7.81-7.76 (m, 1H), 6.63 (s, 1H), 5.31-5.13 (m, 1H), 3.46-3.35 (m, 1H), 3.22-3.08 (m, 4H), 2.43-2.27 (m, 2H).

### Beispiel 10.1C

### Methyl-5-({2-[5-chlor-4-(5-chlor-2-cyanphenyl)-2-oxopyridin-1(2H)-yl]-4-methoxybutanoyl}-amino)thiophen-2-carboxylat (Racemat)

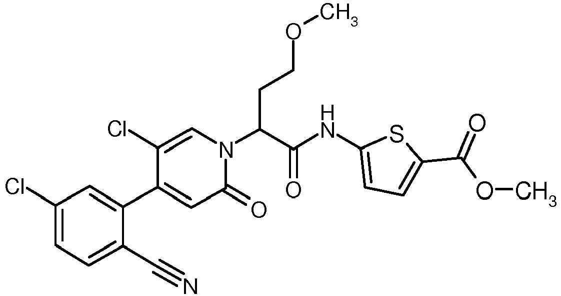

Gemäß allgemeiner Methode 5B wurden 80 mg (0.20 mmol) 2-[5-Chlor-4-(5-chlor-2-cyanphenyl)-2-oxopyridin-1(2H)-yl]-4-methoxybutansäure (Racemat) und 48 mg (0.30 mmol) Methyl-5-aminothiophen-2-carboxylat in 1 ml Pyridin umgesetzt. Ausbeute: 92 mg (87% d. Th.).
LC/MS [Methode 1]: Rₜ = 1.08 min; MS (ESIpos): m/z = 520 (M+H)⁺,
¹H-NMR (400 MHz, DMSO-d₆): δ [ppm] = 12.15 (bs, 1H), 8.24 (s, 1H), 8.07 (d, 1H), 7.85-7.76 (m, 2H), 7.62 (d, 1H), 6.83 (d, 1H), 6.68 (s, 1H), 5.70 (bs, 1H), 3.78 (s, 3H), 3.45-3.37 (m, 1H), 3.27-3.14 (m, 4H), 2.47-2.38 (m, 2H).

### Beispiel 11.1A

### Methyl-5-({2-[4-(5-chlor-2-cyanophenyl)-5-methoxy-2-oxopyridin-1(2H)-yl]-4-methoxybutanoyl}-amino)thiophen-2-carboxylat (Racemat)

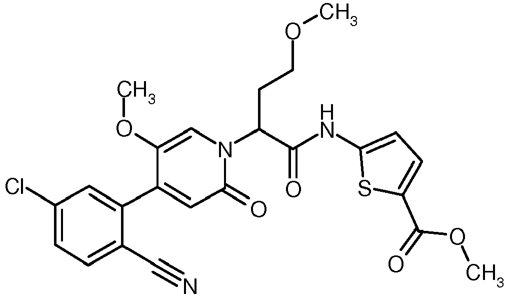

Nach der allgemeinen Methode 5B wurden 80 mg (95% Reinheit, 0.20 mmol) 2-[4-(5-Chlor-2-cyanphenyl)-5-methoxy-2-oxopyridin-1(2H)-yl]-4-methoxybutansäure (Racemat) und 47.6 mg Methyl-5-aminothiophen-2-carboxylat (0.30 mmol, 1.5eq.) umgesetzt. Ausbeute: 104 mg (97% d. Th.).
LC/MS [Methode 1]: Rₜ = 1.00 min; MS (ESIpos): m/z = 516 (M+H)⁺,
¹H-NMR (400 MHz, DMSO-d₆): δ [ppm] = 12.07 (s, 1H), 8.05-7.95 (m, 1H), 7.76-7.71 (m, 2H), 7.62 (d, 1H), 7.50 (s, 1H), 6.84 (d, 1H), 6.53 (s, 1H), 5.68 (dd, 1H), 3.77 (s, 3H), 3.69 (s, 3H), 3.40 (dt, 1H), 3.28-3.22 (m, 1H), 3.20 (s, 3 H), 2.47-2.38 (m, 2H).

### Beispiel 12.1A

### tert-Butyl-2-[4-(5-chlor-2-cyanphenyl)-5-methoxy-2-oxopyridin-1(2H)-yl]-3-(1-methyl-1H-pyrazol-3-yl)propanoat (Racemat)

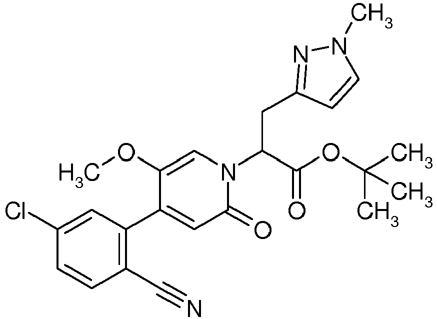

Eine Lösung von 750 mg (2.00 mmol) tert-Butyl-[4-(5-chlor-2-cyanphenyl)-5-methoxy-2-oxopyridin-1(2H)-yl]acetat in 15 ml THF wurde unter Argon bei -70°C mit 2.50 ml (2.50 mmol, 1.25 eq.) 1N Lithium-bis(trimethylsilyl)amid in THF versetzt und für 30 min gerührt. Anschließend wurden 725 mg (1.91 mmol, 46% Reinheit, 0.95 eq.) 3-(Brommethyl)-1-methyl-1H-pyrazol hinzugegeben, 30 min bei -70°C und anschließend auf RT kommend 2 h gerührt. Das Reaktionsgemisch wurde mit 10 ml gesättigter, wässriger Ammoniumchlorid-Lösung, 10 ml Wasser und 80 ml Essigsäureethylester versetzt. Die wässrige Phase wurde einmal mit Essigsäureethylester extrahiert und die vereinigten organischen Phasen wurden mit gesättigter, wässriger Natriumchlorid-Lösung gewaschen, anschließend getrocknet und eingeengt. Das Rohprodukt wurde mittels Biotage-Isolera (Eluent: Dichlormethan/Methanol, 0-10%) gereinigt. Ausbeute: 1.01 g (85% Reinheit, 92% d. Th.).
LC/MS [Methode 1]: Rₜ = 0.99 min; MS (ESIpos): m/z = 469 (M+H)⁺,
¹H-NMR (400 MHz, DMSO-d₆): δ [ppm] = 7.97 (d, 1H), 7.75-7.66 (m, 2H), 7.50 (d, 1H), 7.30 (s, 1H), 6.44 (s, 1H), 5.96 (d, 1H), 5.29 (dd, 1H), 3.73 (s, 3H), 3.55 (s, 3H), 3.49-3.37 (m, 1H), 3.36-3.27 (m, 1H), 1.41 (s, 9H).

### Beispiel 12.1B

### 2-[4-(5-Chlor-2-cyanphenyl)-5-methoxy-2-oxopyridin-1(2H)-yl]-3-(1-methyl-1H-pyrazol-3-yl)propansäure (Racemat)

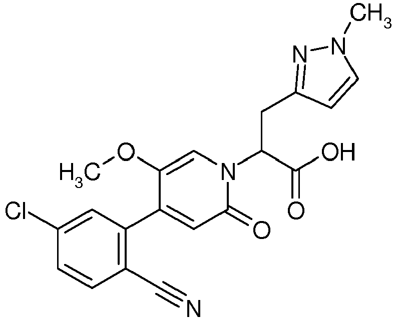

1.02 g (1.85 mmol, 85% Reinheit) tert-Butyl-2-[4-(5-chlor-2-cyanphenyl)-5-methoxy-2-oxopyridin-1(2H)-yl]-3-(1-methyl-1H-pyrazol-3-yl)propanoat (Racemat) wurden mit 18 ml 4N Chlorwasserstoff in Dioxan versetzt und über Nacht bei RT gerührt. Das Reaktionsgemisch wurde eingeengt und lyophilisiert. Der Rückstand wurde mittels präparativer HPLC (RP18 Säule, Laufmittel: Acetonitril/Wasser-Gradient unter Zusatz von 0.1% Ameisensäure) gereinigt. Ausbeute: 620 mg (81% d. Th.).
LC/MS [Methode 1]: Rₜ = 0.77 min; MS (ESIpos): m/z = 413 (M+H)⁺,
¹H-NMR (400 MHz, DMSO-d₆): δ [ppm] = 13.05 (bs, 1H), 8.02-7.92 (m, 1H), 7.74-7.66 (m, 2H), 7.48 (d, 1H), 7.32 (s, 1H), 6.42 (s, 1H), 5.91 (d, 1H), 5.30 (dd, 1H), 3.72 (s, 3H), 3.54 (s, 3H), 3.47 (dd, 1H), 3.38-3.26 (m, 1H).

### Beispiel 13.1A

### 5-(Brommethyl)-1,3-oxazol

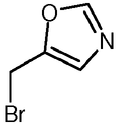

Eine Lösung von 1.83 ml (13.12 mmol, 1.3 eq.) Triethylamin und 1.0 g (10.09 mmol, 1 eq.) 1,3-Oxazol-5-ylmethanol in 14 ml *N,N*-Dimethylformamid wurde unter Argon bei 0°C tropfenweise mit 1.02 ml (13.12 mmol, 1.3 eq.) Methansulfonsäurechlorid versetzt und 1 h bei 0°C gerührt. Anschließend wurden 2.45 g (28.26 mmol, 2.8 eq.) Lithiumbromid zugegeben, und diese Reaktionsmischung wurde 1 h bei 0°C gerührt. Nach Zugabe von Wasser wurde die Mischung mit Essigsäureethylester extrahiert. Die vereinigten organischen Phasen wurden mit gesättigter, wässriger Natriumchlorid-Lösung gewaschen, über Natriumsulfat getrocknet und im Vakuum eingeengt. Das Rohprodukt wurde anschließend mittels Normalphasen-Chromatographie (Eluent: Dichlormethan) aufgereinigt. Ausbeute 1.23 g (80% Reinheit, 60% d. Th.)
¹H-NMR (400 MHz, DMSO-d₆): δ [ppm] = 8.42 (s, 1H), 7.26 (s, 1H), 4.93 (s, 2H).

### Beispiel 13.1B

### 2-[4-(5-Chlor-2-cyanophenyl)-5-methoxy-2-oxopyridin-1(2H)-yl]-3-(l,3-oxazol-5-yl)propansäure-tert.-butylester (Racemat)

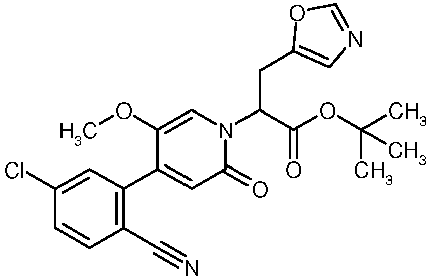

Nach der allgemeinen Methode 8B wurden 1.5 g (4.00 mmol) [4-(5-Chlor-2-cyanophenyl)-5-methoxy-2-oxopyridin-1(2*H*)-yl]essigsäure-*tert*.-butylester mit 1.78 g (51% Reinheit, 5.60 mmol, 1.4 eq.) 5-(Brommethyl)-1,3-oxazol umgesetzt. Ausbeute: 1.89 g (60% Reinheit, 62% d. Th.)
LC/MS [Methode 1]: Rₜ = 0.98 min; MS (ESIpos): m/z = 456 (M+H)⁺.

### Beispiel 13.1C

### 2-[4-(5-Chlor-2-cyanophenyl)-5-methoxy-2-oxopyridin-1(2H)-yl]-3-(1,3-oxazol-5-yl)propansäure (Racemat)

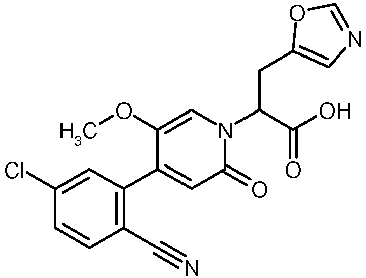

Gemäß allgemeiner Methode 6A wurden 1.89 g (60% Reinheit, 2.48 mmol) 2-[4-(5-Chlor-2-cyanophenyl)-5-methoxy-2-oxopyridin-1(2*H*)-yl]-3-(1,3-oxazol-5-yl)propansäure-*tert*.-butylester (Racemat) in 28 ml Dichlormethan mit 14 ml (435 mmol) TFA umgesetzt. Ausbeute: 597 mg (80% Reinheit, 48% d. Th.)
LC/MS [Methode 1]: Rₜ = 0.70 min; MS (ESIpos): m/z = 400 (M+H)⁺,
¹H-NMR (400 MHz, DMSO-d₆): δ [ppm] = 13.24 (br. s, 1H), 8.17 (s, 1H), 8.02-7.93 (m, 1H), 7.77-7.66 (m, 2H), 7.35 (s, 1H), 6.85 (s, 1H), 6.47 (s, 1H), 5.32 (dd, 1H), 3.63-3.72 (m, 1H), 3.58-3.47 (m, 4H).

### Ausführungsbeispiele

### Allgemeine Methode 1: Amid-Kupplung mit T3P/Pyridin

Eine Lösung der entsprechenden Carbonsäure oder Carbonsäurehydrochlorid (1 eq.) und des entsprechenden Amins oder Aminhydrochlorid (1.1-1.9 eq.) in Pyridin (ca. 0.1M) wurde auf 60°C erwärmt und tropfenweise mit T3P (50%ig in Essigsäureethylester, 1.5-15 eq.) versetzt. Alternativ wurde T3P bei RT hinzugefügt und danach bei RT gerührt oder auf 50 bis 90°C erhitzt. Nach 1 bis 20 h wurde das Reaktionsgemisch auf RT gekühlt und entweder direkt mittels präparativer RP-HPLC (Wasser-Acetonitril-Gradient oder Wasser-Methanol-Gradient) aufgereinigt, oder mit Wasser und Essigsäureethylester versetzt. Die wässrige Phase wurde mit Essigsäureethylester extrahiert. Die vereinigten organischen Phasen wurden mit wässriger Puffer-Lösung (pH=5), mit gesättigter, wässriger Natriumhydrogencarbonat-Lösung und mit gesättigter, wässriger Natriumchlorid-Lösung gewaschen, über Natriumsulfat getrocknet und im Vakuum eingeengt. Das Rohprodukt wurde gegebenenfalls anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 2: Verseifung eines Methyl-/Ethyl- oder Benzylesters mit Lithiumhydroxid

Eine Lösung von 1.0 eq. des entsprechenden Methyl- oder Ethylesters in Tetrahydrofuran/Wasser (3:1, ca. 7-15 ml/mmol) wurde bei RT mit Lithiumhydroxid (2-4 eq.) versetzt. Das Reaktionsgemisch wurde bei RT bis 60°C gerührt und anschließend mit wässriger Salzsäure (1N) auf pH1 gestellt. Nach Zugabe von Wasser/Essigsäureethylester und Phasentrennung wurde die wässrige Phase dreimal mit Essigsäureethylester extrahiert. Die vereinigten, organischen Phasen wurden getrocknet (Natriumsulfat oder Magnesiumsulfat), filtriert und im Vakuum eingeengt. Das Rohprodukt wurde anschließend entweder mittels Normalphasen-Chromatographie (Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

Die folgenden Beispiele 1 bis 6 wurden gemäß der allgemeinen Methode 1 hergestellt.

| Bsp. | IUPAC-Name / Struktur | Ausbeute | Analytik |
|---|---|---|---|
| **1** | 5-({2-[4-(5-Chlor-2-cyanphenyl)-5-methoxy-2-oxopyridin-1(2H)-yl]-4-methoxybutanoyl}amino)-thiophen-2-carboxamid (Racemat) | 41 mg, 96% Reinheit 62% d. Th. | MS (ESI): m/z = 501 [M+H]⁺ |
| | | | LC/MS (Methode 1): Rₜ = 0.83 min. |
| | 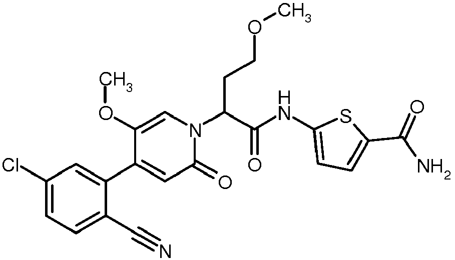 | | ¹H-NMR (400 MHz, DMSO-*d*₆) δ ppm 11.83 (s, 1H), 8.04-7.94 (m, 1H), 7.85-7.66 (m, 3H), 7.56-7.44 (m, 2H), 7.15 (bs, 1H), 6.75 (d, 1H), 6.53 (s, 1H), 5.70 (dd, 1H), 3.68 (s, 3H), 3.41-3.36 (m, 1H), 3.26-3.22 (m, 1H), 3.20 (s, 3H), 2.44-2.37 (m, 2H) |
| | Hergestellt aus: 50 mg (0.13 mmol) 2-[4-(5-Chlor-2-cyanophenyl)-5-methoxy-2-oxopyridin-1(2H)-yl]-4-methoxybutansäure (Racemat), 31 mg (87% Reinheit, 0.19 mmol) 5-Aminothiophen-2-carboxamid und äquimolaren Mengen der übrigen Reagenzien | | |
| **2** | 5-({2-[5-Chlor-4-(5-chlor-2-cyanophenyl)-2-oxopyridin-1(2H)-yl]-4-methoxybutanoyl}amino)-thiophen-2-carboxamid (Racemat) | 14 mg 22% d. Th. | MS (ESI): m/z = 505 [M+H]⁺ |
| | | | LC/MS (Methode 1): Rₜ = 0.88 min. |
| | 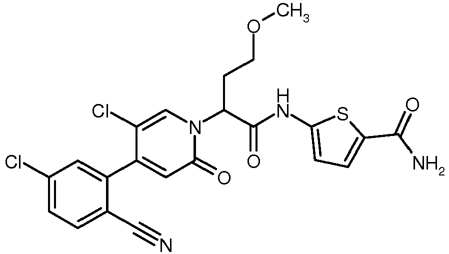 | | ¹H-NMR (400 MHz, DMSO-*d*₆) δ ppm 11.91 (bs, 1H), 8.24 (s, 1H), 8.06 (d, 1H), 7.89-7.65 (m, 3H), 7.53 (d, 1H), 7.17 (bs, 1H), 6.75 (d, 1H), 6.67 (s, 1H), 5.78-5.63 (m, 1H), 3.40 (dt, 1H), 3.26-3.21 (m, 1H), 3.19 (s, 3H), 2.45-2.38 (m, 2H) |
| | Hergestellt aus: 50 mg (0.13 mmol) 2-[5-Chlor-4-(5-chlor-2-cyanophenyl)-2-oxopyridin-1(2H)-yl]-4-methoxybutansäure (Racemat), 31 mg (87% Reinheit, 0.19 mmol) 5-Aminothiophen-2-carboxamid und äquimolaren Mengen der übrigen Reagenzien | | |
| **3** | 5-({2-[4-(5-Chlor-2-cyanophenyl)-5-methoxy-2-oxopyridin-1(2H)-yl]-3-(pyridin-2-yl)propanoyl}-amino)-3-fluorthiophen-2-carboxamid (Racemat) | 13 mg 14% d. Th. | MS (ESI): m/z = 552 [M+H]⁺ |
| | | | LC/MS (Methode 1): Rₜ = 0.77 min. |
| | 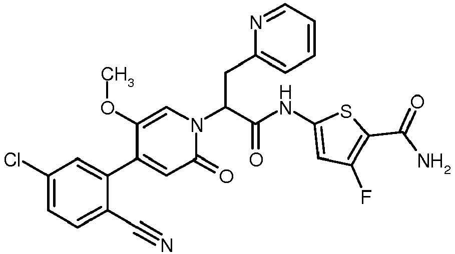 | | ¹H-NMR (400 MHz, DMSO-*d*₆) δ ppm 10.99 (s, 1H), 8.48 (d, 1H), 7.96 (d, 1H), 7.86 (d, 1H), 7.79-7.68 (m, 3H), 7.65 (d, 1H), 7.54 (s, 1H), 7.45-7.34 (m, 2H), 7.23 (dd, 1H), 6.42 (s, 1H), 6.18 (dd, 1H), 3.75-3.64 (m, 2H), 3.62 (s, 3H) |
| | Hergestellt aus: 75 mg (0.17 mmol) 2-[4-(5-Chlor-2-cyanophenyl)-5-methoxy-2-oxopyridin-1(2H)-yl]-3-(pyridin-2-yl)propansäure-Hydrochlorid (Racemat), 49 mg (83% Reinheit, 0.25 mmol) 5-Amino-3-fluorthiophen-2-carboxamid und äquimolaren Mengen der übrigen Reagenzien | | |
| **4** | 5-({2-[4-(5-Chlor-2-cyanophenyl)-5-methoxy-2-oxopyridin-1(2H)-yl]-3-(pyridin-4-yl)propanoyl}-amino)-3-fluorthiophen-2-carboxamid (Racemat) | 14 mg 24% d. Th. | MS (ESI): m/z = 552 [M+H]⁺ |
| | | | LC/MS (Methode 1): Rₜ = 0.63 min. |
| | 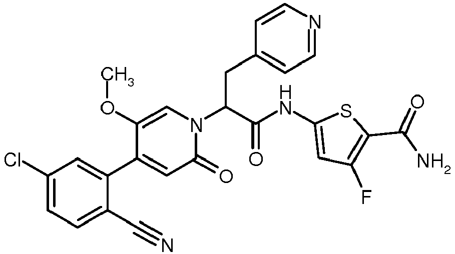 | | ¹H-NMR (400 MHz, DMSO-*d*₆) δ ppm 10.86 (s, 1H), 8.51-8.38 (m, 2H), 7.96 (d, 1H), 7.91 (d, 1H), 7.83-7.67 (m, 2H), 7.67-7.57 (m, 2H), 7.43 (bs, 1H), 7.29 (d, 2H), 6.39 (s, 1H), 6.22 (dd, 1H), 3.73-3.58 (m, 4H), 3.47 (dd, 1H) |
| | Hergestellt aus: 50 mg (93% Reinheit, 0.10 mmol) 2-[4-(5-Chlor-2-cyanophenyl)-5-methoxy-2-oxopyridin-1(2H)-yl]-3-(pyridin-4-yl)propansäure-Hydrochlorid (Racemat), 31 mg (83% Reinheit, 0.16 mmol) 5-Amino-3-fluorthiophen-2-carboxamid und äquimolaren Mengen der übrigen Reagenzien | | |
| **5** | 5-({2-[4-(5-Chlor-2-cyanphenyl)-5-methoxy-2-oxopyridin-1(2H)-yl]-3-(pyridin-3-yl)propanoyl}-amino)-3-fluorthiophen-2-carboxamid (Racemat) | 26 mg 40% d. Th. | MS (ESI): m/z = 552 [M+H]⁺ |
| | | | LC/MS (Methode 1): Rₜ = 0.67 min. |
| | 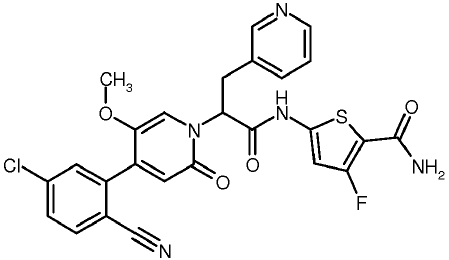 | | ¹H-NMR (400 MHz, DMSO-*d*₆) δ ppm 10.85 (s, 1H), 8.54 (d, 1H), 8.39 (dd, 1H), 7.96 (d, 1H), 7.91 (d, 1H), 7.84-7.68 (m, 2H), 7.68-7.58 (m, 3H), 7.43 (bs, 1H), 7.27 (dd, 1H), 6.40 (s, 1H), 6.28-6.11 (m, 1H), 3.69 (s, 3H), 3.66-3.55 (m, 1H), 3.51-3.44 (m, 1H) |
| | Hergestellt aus: 60 mg (88% Reinheit, 0.12 mmol) 2-[4-(5-Chlor-2-cyanophenyl)-5-methoxy-2-oxopyridin-1(2H)-yl]-3-(pyridin-3-yl)propansäure-Hydrochlorid (Racemat), 34 mg (83% Reinheit, 0.18 mmol) 5-Amino-3-fluorthiophen-2-carboxamid und äquimolaren Mengen der übrigen Reagenzien | | |
| **6** | 5-({2-[4-(5-Chlor-2-fluorphenyl)-5-methoxy-2-oxopyridin-1(2H)-yl]-3-(pyridin-2-yl)propanoyl}-amino)-3-fluorthiophen-2-carboxamid (Racemat) | 17 mg, 94% Reinheit 21% d. Th. | MS (ESI): m/z = 545 [M+H]⁺ |
| | | | LC/MS (Methode 1): Rₜ = 0.82 min. |
| | 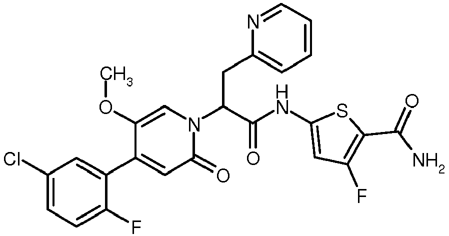 | | ¹H-NMR (400 MHz, DMSO-*d*₆) δ ppm 10.98 (s, 1H), 8.49 (d, 1H), 7.85 (d, 1H), 7.81-7.63 (m, 2H), 7.53 (ddd, 1H), 7.49-7.38 (m, 4H), 7.33 (t, 1H), 7.25 (dd, 1H), 6.36 (s, 1H), 6.26-6.08 (m, 1H), 3.76-3.62 (m, 2H), 3.59 (s, 3H) |
| | Hergestellt aus: 80 mg (75% Reinheit, 0.14 mmol) 2-[4-(5-Chlor-2-fluorphenyl)-5-methoxy-2-oxopyridin-1(2H)-yl]-3-(pyridin-2-yl)propansäure-Hydrochlorid (Racemat), 40 mg (83% Reinheit, 0.20 mmol) 5-Amino-3-fluorthiophen-2-carboxamid und äquimolaren Mengen der übrigen Reagenzien | | |

### Beispiel 7

### 5-({2-[4-(5-Chlor-2-cyanphenyl)-5-methoxy-2-oxopyridin-1(2H)-yl]-4-methoxybutanoyl}-amino)thiophen-2-carbonsäure (Racemat)

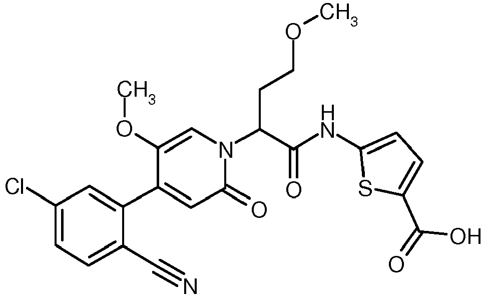

Eine Lösung von 92.5 mg (0.18 mmol, 1 eq.) Methyl-5-({2-[4-(5-chlor-2-cyanophenyl)-5-methoxy-2-oxopyridin-1(2H)-yl] -4-methoxybutanoyl} amino)thiophen-2-carboxylat (Racemat) in 3 ml Tetrahydrofuran und 1 ml Wasser wurde mit 15 mg (0.36 mmol, 2 eq.) Lithiumhydroxid-Monohydrat versetzt und bei Raumtemperatur 60 min gerührt. Das Reaktiongemisch wurde bei -20°C über 16 h gelagert. Anschließend wurde das Reaktiongemisch auf 50°C erwärmt und 24 h gerührt. Danach wurde das Reaktiongemisch auf 80°C erwärmt und 24 h gerührt. Das Reaktiongemisch wurde mit 1M Salzäure neutralisiert und der Rückstand wurde anschließend mittels präparativer RP-HPLC (Wasser-Acetonitril-0.1% Ameisensäure-Gradient) aufgereinigt. Ausbeute: 34 mg (34% d. Th.).
LC/MS [Methode 1]: Rₜ = 0.88 min; MS (ESIpos): m/z = 502 (M+H)⁺,
¹H-NMR (400 MHz, DMSO-d₆): δ [ppm] = 12.66 (bs, 1H), 11.99 (s, 1H), 8.05-7.94 (m, 1H), 7.78-7.69 (m, 2H), 7.58-7.46 (m, 2H), 6.81 (d, 1H), 6.53 (s, 1H), 5.69 (dd, 1H), 3.69 (s, 3H), 3.39 (dt, 1H), 3.27-3.22 (m, 1H), 3.20 (s, 3H), 2.46-2.38 (m, 2H).

### Beispiel 8

### 5-({2-[4-(5-Chlor-2-cyanphenyl)-5-methoxy-2-oxopyridin-1(2H)-yl]-4-methoxybutanoyl}-amino)thiophen-2-carboxamid (Enantiomer 1)

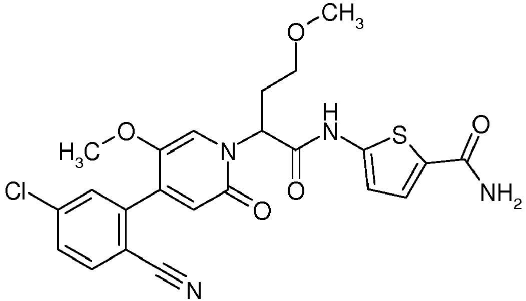

Enantiomerentrennung von 120 mg 5-({2-[4-(5-Chlor-2-cyanphenyl)-5-methoxy-2-oxopyridin-1(2H)-yl]-4-methoxybutanoyl}amino)thiophen-2-carboxamid (Racemat) (Beispiel 1) ergab 30 mg Enantiomer 2 (chirale HPLC: Rₜ = 9.45 min) und 34 mg der Titelverbindung Beispiel 8 (Enantiomer 1): Chirale HPLC: Rₜ = 6.96 min; 100% ee.
Trennmethode: Säule: Daicel Chiralpak OX-H 5µm 250 mm x 20 mm; Eluent: iso-Hexan 10% / Ethanol 90%; Temperatur: 20°C; Fluss: 15 ml/min; UV-Detektion: 220 nm.
Analytik: Säule: Daicel Chiralpak OX-3 3µm 50 mm x 4.6 mm; Eluent: 50% iso-Hexan, 50% Ethanol; Fluss: 1 ml/min; UV-Detektion: 210 nm.
LC/MS [Methode 1]: Rₜ = 0.80 min; MS (ESIpos): m/z = 501 (M+H)⁺,
¹H-NMR (400 MHz, DMSO-*d*₆): δ [ppm] = 11.83 (s, 1H), 8.04-7.94 (m, 1H), 7.85-7.66 (m, 3H), 7.56-7.44 (m, 2H), 7.15 (bs, 1H), 6.75 (d, 1H), 6.53 (s, 1H), 5.70 (dd, 1H), 3.68 (s, 3H), 3.41-3.36 (m, 1H), 3.26-3.22 (m, 1H), 3.20 (s, 3H), 2.44-2.37 (m, 2H).

### Beispiel 9

### 5-({2-[4-(5-Chlor-2-cyanphenyl)-5-methoxy-2-oxopyridin-1(2H)-yl]-4-methoxybutanoyl}amino)-3-fluorthiophen-2-carboxamid (Racemat)

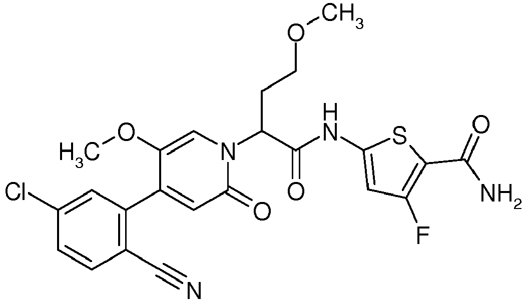

Gemäß allgemeiner Methode 1 wurden 100 mg (0.26 mmol) 2-[4-(5-Chlor-2-cyanphenyl)-5-methoxy-2-oxopyridin-1(2H)-yl]-4-methoxybutansäure (Racemat) und 77 mg (0.40 mmol, 83% Reinheit) 5-Amino-3-fluorthiophen-2-carboxamid in 2 ml Pyridin umgesetzt. Ausbeute: 123 mg (89% d. Th.).
LC/MS [Methode 10]: Rₜ = 1.49 min; MS (ESIpos): m/z = 519 (M+H)⁺,
¹H-NMR (400 MHz, DMSO-d₆): δ [ppm] = 10.68 (s, 1H), 8.02-7.97 (m, 1H), 7.85 (d, 1H), 7.78-7.67 (m, 3H), 7.49 (s, 1H), 7.41 (bs, 1H), 6.52 (s, 1H), 5.83 (t, 1H), 3.67 (s, 3H), 3.42-3.34 (m, 1H), 3.34-3.25 (m, 1H), 3.21 (s, 3H), 2.43-2.34 (m, 2H).

### Beispiel 10

### 5-({2-[4-(5-Chlor-2-cyanphenyl)-5-methoxy-2-oxopyridin-1(2H)-yl]-4-methoxybutanoyl}-amino)-3-fluorthiophen-2-carboxamid (Enantiomer 1)

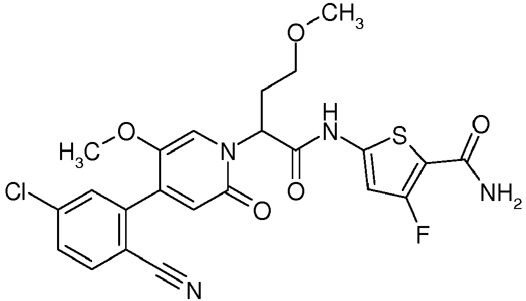

Enantiomerentrennung von 120 mg 5-({2-[4-(5-Chlor-2-cyanphenyl)-5-methoxy-2-oxopyridin-1(2H)-yl]-4-methoxybutanoyl}amino)-3-fluorthiophen-2-carboxamid (Racemat) (Beispiel 9) ergab 45 mg Enantiomer 2 (chirale HPLC: Rₜ = 24 min) und 43 mg der Titelverbindung Beispiel 10 (Enantiomer 1): Chirale HPLC: Rₜ = 17 min; 100% ee.
Trennmethode: Säule: Daicel Chiralcel OX-H 5µm, 250 mm x 30 mm; Eluent: Kohlendioxid 70% / Ethanol 30%; Temperatur: 40°C; Fluss: 80 ml/min; Druck: 100 bar; UV-Detektion: 210 nm.
Analytik: Säule: Daicel OX 5 µm 250 mm x 4.6 mm; Eluent: 60% Kohlendioxid, 40% Ethanol; Fluss: 3 ml/min; UV-Detektion: 210 nm.
LC/MS [Methode 10]: Rₜ = 1.49 min; MS (ESIpos): m/z = 519 (M+H)⁺,
¹H-NMR (400 MHz, DMSO-d₆): δ [ppm] = 10.68 (s, 1H), 8.02-7.97 (m, 1H), 7.85 (d, 1H), 7.78-7.67 (m, 3H), 7.49 (s, 1H), 7.41 (bs, 1H), 6.52 (s, 1H), 5.83 (t, 1H), 3.67 (s, 3H), 3.42-3.34 (m, 1H), 3.34-3.25 (m, 1H), 3.21 (s, 3H), 2.43-2.34 (m, 2H).

### Beispiel 11

### 5-({2-[4-(5-Chlor-2-cyanphenyl)-5-methoxy-2-oxopyridin-1(2H)-yl]-4-methoxybutanoyl}amino)-N-methylthiophen-2-carboxamid (Racemat)

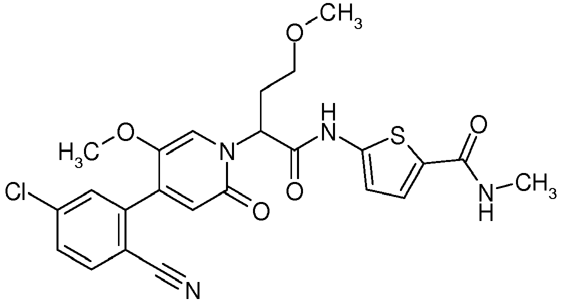

Gemäß allgemeiner Methode 1 wurden 150 mg (0.36 mmol, 91% Reinheit) 2-[4-(5-Chlor-2-cyanphenyl)-5-methoxy-2-oxopyridin-1(2H)-yl]-4-methoxybutansäure (Racemat) und 87 mg (0.54 mmol) 5-Amino-N-methylthiophen-2-carboxamid in 3 ml Pyridin umgesetzt. Ausbeute: 135 mg (70% d. Th.).
LC/MS [Methode 1]: Rₜ = 0.86 min; MS (ESIpos): m/z = 515 (M+H)⁺,
¹H-NMR (400 MHz, DMSO-d₆): δ [ppm] = 11.84 (s, 1H), 8.23 (q, 1H), 8.02-7.97 (m, 1H), 7.76-7.71 (m, 2H), 7.51 (s, 1H), 7.47 (d, 1H), 6.76 (d, 1H), 6.53 (s, 1H), 5.75-5.66 (m, 1H), 3.68 (s, 3H), 3.42-3.35 (m, 1H), 3.28-3.22 (m, 1H), 3.20 (s, 3H), 2.73 (d, 3H), 2.46-2.35 (m, 2H).

### Beispiel 12

### 5-({2-[4-(5-Chlor-2-cyanphenyl)-5-methoxy-2-oxopyridin-1(2H)-yl]-4-methoxybutanoyl}-amino)-N-methylthiophen-2-carboxamid (Enantiomer 1)

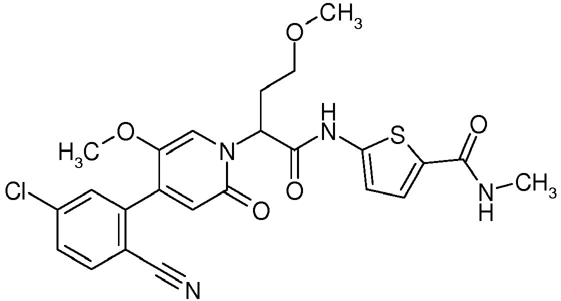

Enantiomerentrennung von 126 mg 5-({2-[4-(5-Chlor-2-cyanphenyl)-5-methoxy-2-oxopyridin-1(2H)-yl]-4-methoxybutanoyl}amino)-N-methylthiophen-2-carboxamid (Racemat) (Beispiel 11) ergab 62 mg Enantiomer 2 (chirale HPLC: Rₜ = 8.3 min) und 53 mg der Titelverbindung Beispiel 12 (Enantiomer 1): Chirale HPLC: Rₜ = 6.2 min; 100% ee.
Trennmethode: Säule: Daicel Chiralcel OX-H 5 µm, 250 mm x 20 mm; Eluent: iso-Hexan 75% / Ethanol 25%; Temperatur: 40°C; Fluss: 15 ml/min; UV-Detektion: 210 nm.
Analytik: Säule: Chiralcel OX-H 5µm 250 mm x 4.6 mm; Eluent: 100% Ethanol; Fluss: 1 ml/min; UV-Detektion: 220 nm.
LC/MS [Methode 1]: Rₜ = 0.86 min; MS (ESIpos): m/z = 515 (M+H)⁺,
¹H-NMR (400 MHz, DMSO-d₆): δ [ppm] = 11.84 (s, 1H), 8.23 (q, 1H), 8.02-7.97 (m, 1H), 7.76-7.71 (m, 2H), 7.51 (s, 1H), 7.47 (d, 1H), 6.76 (d, 1H), 6.53 (s, 1H), 5.75-5.66 (m, 1H), 3.68 (s, 3H), 3.42-3.35 (m, 1H), 3.28-3.22 (m, 1H), 3.20 (s, 3H), 2.73 (d, 3H), 2.46-2.35 (m, 2H).

### Beispiel 13

### 5-({2-[4-(5-Chlor-2-cyanphenyl)-5-methoxy-2-oxopyridin-1(2H)-yl]-3-(1-methyl-1H-pyrazol-3-yl)propanoyl}amino)thiophen-2-carboxamid (Racemat)

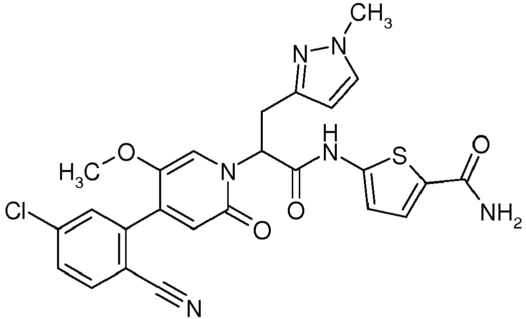

Gemäß allgemeiner Methode 1 wurden 120 mg (0.27 mmol) 2-[4-(5-Chlor-2-cyanphenyl)-5-methoxy-2-oxopyridin-1(2H)-yl]-3-(1-methyl-1H-pyrazol-3-yl)propansäure (Racemat) und 61 mg (0.41 mmol) 5-Aminothiophen-2-carboxamid in 2.25 ml Pyridin umgesetzt. Ausbeute: 92 mg (61% d. Th.).
LC/MS [Methode 1]: Rₜ = 0.76 min; MS (ESIpos): m/z = 537 (M+H)⁺,
¹H-NMR (400 MHz, DMSO-d₆): δ [ppm] = 11.90 (s, 1H), 7.98 (d, 1H), 7.86-7.67 (m, 3 H), 7.57 (s, 1H), 7.54-7.49 (m, 2H), 7.16 (bs, 1H), 6.74 (d, 1H), 6.46 (s, 1H), 5.96 (d, 1H), 5.84 (dd, 1H), 3.73 (s, 3H), 3.66 (s, 3H), 3.57-3.38 (m, 2H).

### Beispiel 14

### 5-({2-[4-(5-Chlor-2-cyanphenyl)-5-methoxy-2-oxopyridin-1(2H)-yl]-3-(1-methyl-1H-pyrazol-3-yl)propanoyl}amino)thiophen-2-carboxamid (Enantiomer 1)

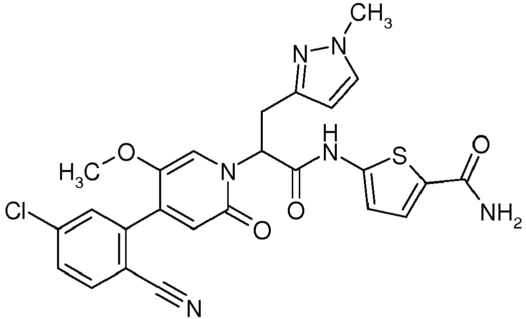

Enantiomerentrennung von 87 mg 5-({2-[4-(5-Chlor-2-cyanphenyl)-5-methoxy-2-oxopyridin-1(2H)-yl]-3-(1-methyl-1H-pyrazol-3-yl)propanoyl}amino)thiophen-2-carboxamid (Racemat) (Beispiel 13) ergab 27 mg Enantiomer 2 (chirale HPLC: Rₜ = 8.70 min) und 29 mg der Titelverbindung Beispiel 14 (Enantiomer 1): Chirale HPLC: Rₜ = 4.25 min; 100% ee.
Trennmethode: Säule: Daicel Chiralpak IC 5 µm, 250 mm x 20 mm; Eluent: Kohlendioxid 50% / Ethanol 50%; Temperatur: 30°C; Fluss: 80 ml/min; Druck: 140 bar; UV-Detektion: 210 nm.
Analytik: Säule: Daicel Chiralpak IC 5µm 250 mm x 4.6 mm; Eluent: 60% Kohlendioxid, 40% Ethanol; Fluss: 3 ml/min; UV-Detektion: 210 nm.
LC/MS [Methode 1]: Rₜ = 0.76 min; MS (ESIpos): m/z = 537 (M+H)⁺,
¹H-NMR (400 MHz, DMSO-d₆): δ [ppm] = 11.90 (s, 1H), 7.98 (d, 1H), 7.86-7.67 (m, 3 H), 7.57 (s, 1H), 7.54-7.49 (m, 2H), 7.16 (bs, 1H), 6.74 (d, 1H), 6.46 (s, 1H), 5.96 (d, 1H), 5.84 (dd, 1H), 3.73 (s, 3H), 3.66 (s, 3H), 3.57-3.38 (m, 2H).

### Beispiel 15

### 5-({2-[4-(5-Chlor-2-cyanphenyl)-5-methoxy-2-oxopyridin-1(2H)-yl]-3-(1-methyl-1H-pyrazol-3-yl)propanoyl}amino)-N-methylthiophen-2-carboxamid (Racemat)

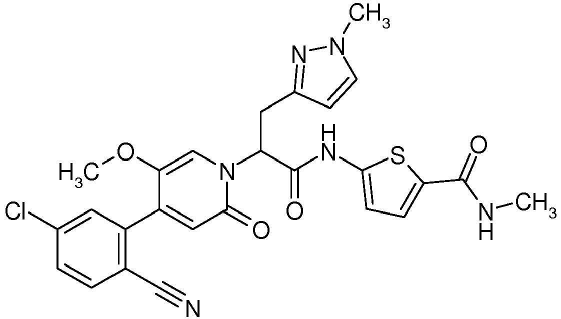

Gemäß allgemeiner Methode 1 wurden 100 mg (0.24 mmol) 2-[4-(5-Chlor-2-cyanphenyl)-5-methoxy-2-oxopyridin-1(2H)-yl]-3-(1-methyl-1H-pyrazol-3-yl)propansäure (Racemat) und 58 mg (0.36 mmol) 5-Amino-N-methylthiophen-2-carboxamid in 2 ml Pyridin umgesetzt. Ausbeute: 100 mg (72% d. Th.).
LC/MS [Methode 1]: Rₜ = 0.79 min; MS (ESIpos): m/z = 551 (M+H)⁺,
¹H-NMR (400 MHz, DMSO-d₆): δ [ppm] = 11.91 (s, 1H), 8.24 (q, 1H), 7.98 (d, 1H), 7.75-7.67 (m, 2H), 7.57 (s, 1H), 7.51 (d, 1H), 7.48 (d, 1H), 6.75 (d, 1H), 6.46 (s, 1H), 5.96 (d, 1H), 5.85 (dd, 1H), 3.73 (s, 3H), 3.66 (s, 3H), 3.54-3.38 (m, 2H), 2.73 (d, 3H).

### Beispiel 16

### 5-({2-[4-(5-Chlor-2-cyanphenyl)-5-methoxy-2-oxopyridin-1(2H)-yl]-3-(1-methyl-1H-pyrazol-3-yl)propanoyl}amino)-N-methylthiophen-2-carboxamid (Enantiomer 1)

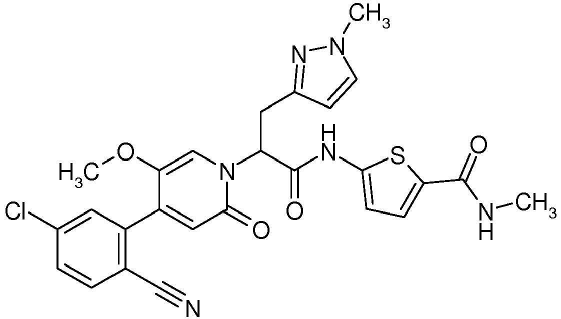

Enantiomerentrennung von 94 mg 5-({2-[4-(5-Chlor-2-cyanphenyl)-5-methoxy-2-oxopyridin-1(2H)-yl]-3-(1-methyl-1H-pyrazol-3-yl)propanoyl}amino)-N-methylthiophen-2-carboxamid (Racemat) (Beispiel 15) ergab 44 mg Enantiomer 2 (chirale HPLC: Rₜ = 10.7 min) und 37 mg der Titelverbindung Beispiel 16 (Enantiomer 1): Chirale HPLC: Rₜ = 7.1 min; 100% ee.
Trennmethode: Säule: Chiralcel OX-H 5 µm, 250 mm x 20 mm; Eluent: iso-Hexan 25% / Ethanol 75%; Temperatur: 40°C; Fluss: 15 ml/min; UV-Detektion: 220 nm.
Analytik: Säule: Chiralcel OX-H 5µm 250 mm x 4.6 mm; Eluent: 100 % Ethanol; Fluss: 1 ml/min; UV-Detektion: 220 nm.
LC/MS [Methode 1]: Rₜ = 0.79 min; MS (ESIpos): m/z = 551 (M+H)⁺,
¹H-NMR (400 MHz, DMSO-d₆): δ [ppm] = 11.91 (s, 1H), 8.24 (q, 1H), 7.98 (d, 1H), 7.75-7.67 (m, 2H), 7.57 (s, 1H), 7.51 (d, 1H), 7.48 (d, 1H), 6.75 (d, 1H), 6.46 (s, 1H), 5.96 (d, 1H), 5.85 (dd, 1H), 3.73 (s, 3H), 3.66 (s, 3H), 3.54-3.38 (m, 2H), 2.73 (d, 3H).

### Beispiel 17

### 5-({2-[4-(5-Chlor-2-cyanphenyl)-5-methoxy-2-oxopyridin-1(2H)-yl]-3-(1,3-oxazol-5-yl)-propanoyl}amino)-N-methylthiophen-2-carboxamid (Racemat)

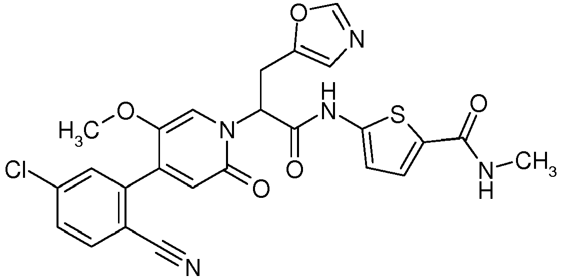

Gemäß allgemeiner Methode 1 wurden 50 mg (0.12 mmol) 2-[4-(5-Chlor-2-cyanphenyl)-5-methoxy-2-oxopyridin-1(2H)-yl]-3-(1,3-oxazol-5-yl)propansäure (Racemat) und 27 mg (0.17 mmol) 5-Amino-N-methylthiophen-2-carboxamid in 1 ml Pyridin umgesetzt. Ausbeute: 56 mg (90% d. Th.).
LC/MS [Methode 1]: Rₜ = 0.81 min; MS (ESIpos): m/z = 538 (M+H)⁺,
¹H-NMR (400 MHz, DMSO-d₆): δ [ppm] = 11.89 (s, 1H), 8.25 (q, 1H), 8.22 (s, 1H), 7.99 (d, 1H), 7.75-7.68 (m, 2H), 7.54 (s, 1H), 7.48 (d, 1H), 6.88 (s, 1H), 6.75 (d, 1H), 6.50 (s, 1H), 5.89 (dd, 1H), 3.81-3.70 (m, 1H), 3.68-3.59 (m, 4H), 2.73 (d, 3H).

### Beispiel 18

### 5-({2-[4-(5-Chlor-2-cyanphenyl)-5-methoxy-2-oxopyridin-1(2H)-yl]-3-(pyridin-2-yl)propanoyl}-amino)thiophen-2-carbonsäure (Racemat)

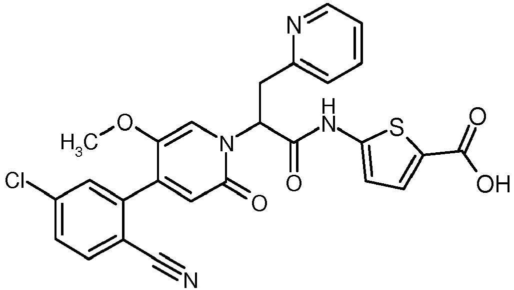

Gemäß allgemeiner Methode 2 wurden 40 mg (0.07 mmol) Methyl-5-({2-[4-(5-chlor-2-cyanphenyl)-5-methoxy-2-oxopyridin-1(2H)-yl]-3-(pyridin-2-yl)propanoyl}amino)thiophen-2-carboxylat (Racemat) umgesetzt. Ausbeute: 11 mg (29% d. Th.).
LC/MS [Methode 1]: Rₜ = 0.81 min; MS (ESIpos): m/z = 535 (M+H)⁺,
¹H-NMR (400 MHz, DMSO-d₆): δ [ppm] = 12.65 (bs, 1H), 12.11 (s, 1H), 8.50-8.46 (m, 1H), 7.96 (d, 1H), 7.73-7.63 (m, 3H), 7.53 (d, 1H), 7.50 (s, 1H), 7.31 (d, 1H), 7.24-7.19 (m, 1H), 6.80 (d, 1H), 6.43 (s, 1H), 6.07 (dd, 1H), 3.76-3.64 (m, 2H), 3.61 (s, 3H).

### Beispiel 19

### 5-({2-[5-Chlor-4-(5-chlor-2-cyanphenyl)-2-oxopyridin-1(2H)-yl]-4-methoxybutanoyl}amino)-thiophen-2-carbonsäure (Racemat)

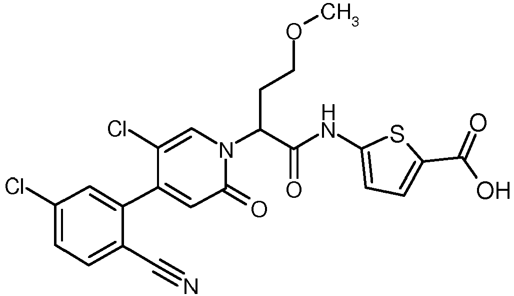

Gemäß allgemeiner Methode 2 wurden 92.4 mg (0.18 mmol) Methyl-5-({2-[5-chlor-4-(5-chlor-2-cyanphenyl)-2-oxopyridin-1(2H)-yl]-4-methoxybutanoyl}amino)thiophen-2-carboxylat (Racemat) umgesetzt. Ausbeute: 31.5 mg (35% d. Th.).
LC/MS [Methode 1]: Rₜ = 0.89 min; MS (ESIpos): m/z = 506 (M+H)⁺,
¹H-NMR (400 MHz, DMSO-d₆): δ [ppm] = 12.68 (bs, 1H), 12.07 (bs, 1H), 8.24 (s, 1H), 8.07 (d, 1H), 7.86-7.76 (m, 2H), 7.53 (d, 1H), 6.80 (d, 1H), 6.68 (s, 1H), 5.71 (bs, 1H), 3.44-3.37 (m, 1H), 3.27-3.21 (m, 1H), 3.19 (s, 3H), 2.47-2.38 (m, 2H).

### B) Bewertung der physiologischen Wirksamkeit

Die Eignung der erfindungsgemäßen Verbindungen zur Behandlung von thromboembolischen Erkrankungen kann in folgenden Assaysystemen gezeigt werden:

### a) Testbeschreibungen (in vitro)

### a.1) Messung der FXIa-Hemmung

Zur Bestimmung der Faktor XIa-Hemmung der erfindungsgemäßen Substanzen wird ein biochemisches Testsystem verwendet, in dem die Umsetzung eines peptidischen Faktor XIa-Substrates zur Ermittlung der enzymatischen Aktivität von humanem Faktor XIa benutzt wird. Dabei spaltet Faktor XIa von dem peptischen Faktor XIa-Substrat das C-terminale Aminomethylcoumarin (AMC) ab, dessen Fluoreszenz gemessen wird. Die Bestimmungen werden in Mikrotiterplatten durchgeführt.

Prüfsubstanzen werden in Dimethylsulfoxid aufgelöst und seriell in Dimethylsulfoxid verdünnt (3000 µM bis 0.0078 µM; resultierende Endkonzentrationen im Test: 50 µM bis 0.00013 µM). Jeweils 1 µl der verdünnten Substanzlösungen werden in die Vertiefungen von weißen Mikrotiterplatten der Firma Greiner (384 Vertiefungen) vorgelegt. Anschließend werden nacheinander 20 µl Assaypuffer (50 mM Tris/HCl pH 7.4; 100 mM Natriumchlorid; 5 mM Calciumchlorid; 0,1% bovines Serumalbumin) und 20 µl Faktor XIa der Firma Kordia (0.45 nM in Assaypuffer) hinzugefügt. Nach 15 min Inkubation wird die Enzymreaktion durch Zugabe von 20 µl des in Assaypuffer gelösten Faktor XIa Substrates Boc-Glu(OBzl)-Ala-Arg-AMC (10 µM in Assaypuffer) der Firma Bachem gestartet, für 30 min bei Raumtemperatur (22°C) inkubiert und anschließend eine Fluoreszensmessung durchgeführt (Anregung: 360 nM, Emission: 460 nM). Die gemessenen Emissionen der Testansätze mit Prüfsubstanz werden mit denen von Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet. Wirkdaten aus diesem Test sind in der folgenden Tabelle A aufgeführt:

**Tabelle A**

| **Beispiel-Nr.** | **IC₅₀[nM]** | | **Beispiel-Nr.** | **IC₅₀[nM]** |
|---|---|---|---|---|
| 1 | 7 | | 2 | 26 |
| 3 | 34 | | 4 | 29 |
| 5 | 42 | | 6 | 1200 |
| 7 | 3 | | 8 | 3 |
| 9 | 54 | | 10 | 27 |
| 11 | 4.6 | | 12 | 2.5 |
| 13 | 4.1 | | 14 | 2.3 |
| 15 | 5.4 | | 16 | 2.8 |
| 17 | 6.2 | | 18 | 2.6 |
| 19 | 6.9 | | | |

### a.2) Bestimmung der Selektivität

Zum Nachweis der Selektivität der Substanzen bezüglich FXIa-Hemmung werden die Prüfsubstanzen auf ihre Hemmung anderer humaner Serinproteasen wie Faktor Xa, Trypsin und Plasmin hin untersucht. Zur Bestimmung der enzymatischen Aktivität von Faktor Xa (1.3 nmol/l von Kordia), Trypsin (83 mU/ml von Sigma) und Plasmin (0.1 µg/ml von Kordia) werden diese Enzyme gelöst (50 mmol/l Tris-Puffer [C,C,C-Tris(hydroxymethyl)-aminomethan], 100 mmol/l NaCl, 0.1% BSA [bovines Serumalbumin], 5 mmol/l Calciumchlorid, pH 7.4) und für 15 min mit Prüfsubstanz in verschiedenen Konzentrationen in Dimethylsulfoxid sowie mit Dimethylsulfoxid ohne Prüfsubstanz inkubiert. Anschließend wird die enzymatische Reaktion durch Zugabe der entsprechenden Substrate gestartet (5 µmol/l Boc-Ile-Glu-Gly-Arg-AMC von Bachem für Faktor Xa und Trypsin, 5 50 µmol/l MeOSuc-Ala-Phe-Lys-AMC von Bachem für Plasmin). Nach einer Inkubationszeit von 30 min bei 22°C wird die Fluoreszenz gemessen (Anregung: 360 nm, Emission: 460 nm). Die gemessenen Emissionen der Testansätze mit Prüfsubstanz werden mit den Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet.

### a.3) Thrombin Generation Assay (Thrombogram)

Die Wirkung der Prüfsubstanzen auf das Thrombogram (Thrombin Generation Assay nach Hemker) wird in vitro in Humanplasma (Octaplas® von der Firma Octapharma) bestimmt.

Im Thrombin Generation Assay nach Hemker wird die Aktivität von Thrombin in gerinnendem Plasma durch die Messung der fluoreszenten Spaltprodukte des Substrats I-1140 (Z-Gly-Gly-Arg-AMC, Bachem) bestimmt. Die Reaktionen werden in Gegenwart variierender Konzentrationen an Prüfsubstanz oder dem entsprechenden Lösungsmittel durchgeführt. Um die Reaktion zu starten werden Reagenzien der Firma Thrombinoscope verwendet (30 pM or 0.1 pM recombinant tissue factor, 24 µM phospholipids in HEPES). Außerdem wird ein Thrombin Calibrator der Firma Thrombinoscope verwendet, dessen amidolytische Aktivität zur Berechnung der Thrombinaktivität in einer Probe mit unbekannter Menge an Thrombin benötigt wird. Die Testdurchführung erfolgt nach Herstellerangaben (Thrombionsocpe BV): 4 µl der Prüfsubstanz oder des Lösungsmittels, 76 µl Plasma und 20 µl PPP-Reagenz oder Thrombin Calibrator werden 5 min bei 37°C inkubiert. Nach Zugabe von 20 µl 2.5 mM Thrombinsubstrat in 20 mM Hepes, 60 mg/ml BSA, 102 mM Calciumchlorid wird die Thrombin Generierung 120 min alle 20 s gemessen. Die Messung wird mit einem Fluorometer (Fluoroskan Ascent) der Firma Thermo Electron durchgeführt, der mit einem 390/460 nM Filterpaar und einem Dispenser ausgestattet ist.

Durch die Verwendung der "thrombinoscope Software" wird das Thrombogramm berechnet und graphisch dargestellt. Die folgenden Parameter werden berechnet: lag time, time to Peak, Peak, ETP (endogenous thrombin potential) und start tail.

### a.4) Bestimmung der antikoagulatorischen Wirkung

Die antikoagulatorische Wirkung der Prüfsubstanzen wird in vitro in Human- und Rattenplasma bestimmt. Dazu wird Blut unter Verwendung einer 0.11 molaren Natriumcitrat-Lösung als Vorlage in einem Mischungsverhältnis Natriumcitrat/Blut 1/9 abgenommen. Das Blut wird unmittelbar nach der Abnahme gut gemischt und 15 Minuten bei ca. 4000 g zentrifugiert. Der Überstand wird abpipettiert.

Die Prothrombinzeit (PT, Synonyme: Thromboplastinzeit, Quick-Test) wird in Gegenwart variierender Konzentrationen an Prüfsubstanz oder dem entsprechenden Lösungsmittel mit einem handelsüblichen Testkit (Neoplastin® von der Firma Boehringer Mannheim oder Hemoliance® RecombiPlastin von der Firma Instrumentation Laboratory) bestimmt. Die Testverbindungen werden 3 Minuten bei 37°C mit dem Plasma inkubiert. Anschließend wird durch Zugabe von Thromboplastin die Gerinnung ausgelöst und der Zeitpunkt des Gerinnungseintritts bestimmt. Es wird die Konzentration an Prüfsubstanz ermittelt, die eine Verdoppelung der Prothrombinzeit bewirkt.

Die aktivierte partielle Thromboplastinzeit (APTT) wird in Gegenwart variierender Konzentrationen an Prüfsubstanz oder dem entsprechenden Lösungsmittel mit einem handelsüblichen Testkit (PTT Reagent von der Firma Roche) bestimmt. Die Testverbindungen werden 3 Minuten bei 37°C mit dem Plasma und dem PTT Reagenz (Cephalin, Kaolin) inkubiert.

Anschließend wird durch Zugabe von 25 mM Calciumchlorid die Gerinnung ausgelöst und der Zeitpunkt des Gerinnungseintritts bestimmt. Es wird die Konzentration an Prüfsubstanz ermittelt, die eine 50%ige Verlängerung beziehungsweise ein Verdoppelung der APTT bewirkt.

### a.5) Bestimmung der Plasma-Kallikrein Aktivität

Zur Bestimmung der Plasma Kallikrein-Hemmung der erfindungsgemäßen Substanzen wird ein biochemisches Testsystem verwendet, in dem die Umsetzung eines peptidischen Plasma Kallikrein-Substrates zur Ermittlung der enzymatischen Aktivität von humanem Plasma Kallikrein benutzt wird. Dabei spaltet Plasma Kallikrein von dem peptischen Plasma Kallikrein-Substrat das C-terminale Aminomethylcoumarin (AMC) ab, dessen Fluoreszenz gemessen wird. Die Bestimmungen werden in Mikrotiterplatten durchgeführt.

Prüfsubstanzen werden in Dimethylsulfoxid aufgelöst und seriell in Dimethylsulfoxid verdünnt (3000 µM bis 0.0078 µM; resultierende Endkonzentrationen im Test: 50 µM bis 0.00013 µM). Jeweils 1 µl der verdünnten Substanzlösungen werden in die Vertiefungen von weißen Mikrotiterplatten der Firma Greiner (384 Vertiefungen) vorgelegt. Anschließend werden nacheinander 20 µl Assaypuffer (50 mM Tris/HCl pH 7,4; 100 mM Natriumchlorid-Lösung; 5 mM Calciumchlorid-Lösung; 0.1% bovines Serumalbumin) und 20 µl Plasma-Kallikrein der Firma Kordia (0.6 nM in Assaypuffer) hinzugefügt. Nach 15 min Inkubation wird die Enzymreaktion durch Zugabe von 20 µl des in Assaypuffer gelösten Substrates H-Pro-Phe-Arg-AMC (10 µM in Assaypuffer) der Firma Bachem gestartet, für 30 min bei Raumtemperatur (22°C) inkubiert und anschließend eine Fluoreszensmessung durchgeführt (Anregung: 360 nM, Emission: 460 nM). Die gemessenen Emissionen der Testansätze mit Prüfsubstanz werden mit denen von Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet.

**Tabelle B**

| **Beispiel-Nr.** | **IC₅₀ [nM]** | | **Beispiel-Nr.** | **IC₅₀[nM]** |
|---|---|---|---|---|
| 1 | 3 | | 2 | 28 |
| 3 | 68 | | 4 | 36 |
| 5 | 38 | | 6 | >10000 |
| 7 | 7.6 | | 8 | 0.94 |
| 9 | 100 | | 10 | 47 |
| 11 | 2.7 | | 12 | 1.7 |
| 13 | 2.2 | | 14 | 0.49 |
| 15 | 1.6 | | 16 | 1.5 |
| 17 | 2.7 | | 18 | 6.0 |
| 19 | 47 | | | |

### a.6) Bestimmung der Endothel Integrität

Die Aktivität der erfindungsgemäßen Verbindungen werden mittels eines in-vitro Permeabilitäts-Assays auf "human umbilical venous cells" (HUVEC) charakterisiert. Mittels des EOS Apparatus (EC IS: Electric Cell-substrate Impedance Sensing; Applied Biophysics Inc; Troy, NY) können Unterschiede des transendothelialen elektrischen Widerstandes (TEER) über einer endothelialen Zell-Monoschicht, die über Gold-Elektroden plattiert wurde, kontinuierlich gemessen werden. HUVECs werden auf einer 96-well sensor Elektrodenplatte (96W1 E, Ibidi GmbH, Martinsried) ausgesäht. Eine Hyperpermeabilität der entstandenen konfluenten Zell-Monoschicht wird mittels Stimulation mit Kininogen, Prekallikrein und Faktor XII (je 100 nM) induziert. Die erfindungsgemäßen Verbindungen werden vor der Zugabe der oben angegebenen Substanzen zugegeben. Die üblichen Konzentrationen der Verbindungen sind 1 x 10⁻¹⁰ bis 1 x 10⁻⁶ M.

### a.7) Bestimmung der in-vitro Permeabilität von Endothelzellen

In einem weiteren Hyperpermeabilitäts-Modell wird die Aktivität der Substanzen auf die Modulation der makromolekularen Permeabilität bestimmt. HUVECs werden auf einer Fibronektin-überzogenen Transwell Filter Membran (24-well plates, 6.5 mm-Einsatz mit 0.4 µM Polykarbonat Membran; Costar #3413) ausgesäht. Die Filtermembran trennt den oberen von dem unteren Zellkulturraum mit der konfluenten Endothelzellschicht auf dem Boden des oberen Zellkulturraumes. Dem Medium des oberen Raumes wird 250 g/ml 40 kDa FITC-Dextan (Invitrogen, D1844) zugesetzt. Die Hyperpermeabilität der Monolayer-Schicht wird mittels Stimulation mit Kininogen, Prekallikrein und Faktor XII (je 100 nM) induziert. Medium Proben werden alle 30 min aus der unteren Kammer entnommen und die relative Fluoreszenz, als Parameter für die Veränderungen der makromolekularen Permeabilität in Abhängigkeit von der Zeit, mit einem Fluorimeter bestimmt. Die erfindungsgemäßen Verbindungen werden vor der Zugabe der oben angegebenen Substanzen zugegeben. Die üblichen Konzentationen der Verbindungen sind 1 x 10⁻¹⁰ bis 1 x 10⁻⁶ M.

### b) Bestimmung der antithrombotischen Wirkung (in vivo)

### b.1) Arterielles Thrombose-Modell (Eisen(II)chlorid-induzierte Thrombose) in Kombination mit Ohrblutungszeit im Kaninchen

Die antithrombotische Aktivität der FXIa-Inhibitoren wird in einem arteriellen Thrombose-Modell getestet. Dabei wird die Thrombusbildung durch chemische Beschädigung eines Bereichs der Arteria carotis im Kaninchen ausgelöst. Simultan wird die Ohrblutungszeit bestimmt.

Männliche Kaninchen (Crl:KBL (NZW)BR, Charles River) unter normaler Diät mit einem Gewicht von 2.2 - 2.5 kg Körpergewicht werden durch intramuskuläre Applikation von Xylazin und Ketamin (Rompun, Bayer, 5 mg/kg und Ketavet, Pharmacia & Upjohn GmbH, 40 mg/kg Körpergewicht) anästhesiert. Die Anästhesie wird weiterhin durch intravenöse Gabe derselben Präparate (bolus: Dauerinfusion) über die rechte Ohrvene unterstützt.

Nach Freipräparation der rechten Arteria carotis wird der Gefäßschaden dadurch erzeugt, dass ein Stück Filterpapier (10 mm x 10 mm) auf einem Streifen Parafilm® (25 mm x 12 mm) um die A. carotis gewickelt wird, ohne den Blutfluß dadurch zu beeinträchtigen. Das Filterpapier enthält 100 µL einer 13%igen Lösung von Eisen(II)chlorid (Sigma) in Wasser. Nach 5 min wird das Filterpapier entfernt und das Gefäß zweimal mit wässriger 0.9%iger Natriumchlorid-Lösung gespült. 30 min nach der Verletzung wird die Arteria carotis im Bereich der Schädigung herauspräpariert und eventuell vorhandenes thrombotisches Material entnommen und gewogen.

Die Prüfsubstanzen werden entweder intravenös über die Vena femoralis den anästhesierten oder oral mittels Schlundsonde den wachen Tieren jeweils 5 min beziehungsweise 2 h vor Schädigung verabreicht.

Die Ohrblutungszeit wird 2 min nach der Schädigung der Arteria carotis bestimmt. Hierzu wird das linke Ohr rasiert und ein definierter Schnitt von 3 mm Länge (Klinge Art.Nummer 10-150-10, Martin, Tuttlingen, Germany) parallel zur Ohrlängsachse gesetzt. Dabei wird darauf geachtet, kein sichtbares Gefäß zu verletzen. Eventuell austretendes Blut wird in 15 Sekunden-Intervallen mit genau gewogenen Filterpapierstücken aufgenommen, ohne die Wunde direkt zu berühren. Die Blutungszeit wird berechnet als die Zeitspanne vom Setzen des Schnitts bis zu dem Zeitpunkt, an dem am Filterpapier kein Blut mehr nachweisbar ist. Das ausgetretene Blutvolumen wird nach Wiegen der Filterpapierstücke berechnet.

### c) Bestimmung der Wirkung auf die Extravasation/Ödembildung und/oder Neovaskularisierung im Auge (in vivo)

### c.1) Testung der Wirksamkeit von Substanzen im Laser-induzierten choroidalen Neovaskularisierungs-Modell

Diese Studie dient dem Zweck, die Wirksamkeit einer Testsubstanz auf die Reduktion der Extravasation/Ödembildung und/oder der choroidalen Neovaskularisierung im Rattenmodell der Laser-induzierten choroidalen Neovaskularisierung zu untersuchen.

Dafür werden pigmentierte Ratten vom Stamm Brown-Norway, die keine Anzeichen ophthalmologischer Erkrankungen aufweisen, ausgewählt und nach dem Zufallsprinzip Behandlungsgruppen zugeordnet. Am Tag 0 werden die Tiere mittels intraperitonealer Injektion narkotisiert (15 mg/kg Xylazin und 80 mg/kg Ketamin). Nach Instillation eines Tropfens einer 0.5%igen Tropicamid-Lösung zur Weitstellung der Pupillen wird die choroidale Neovaskularisierung mittels eines 532 nm Argon Laser Photokoagulators an sechs definierten Stellen um den Nervus opticus herum ausgelöst (50-75 µm Durchmesser, 150 mW Intensität, 100 ms Dauer). Die Testsubstanz und das entsprechende Vehikel (z.B. PBS, isotone Kochsalzlösung) werden entweder systemisch oral beziehungsweise intraperitonal appliziert oder lokal am Auge durch mehrfache Gabe als Augentropfen beziehungsweise intravitreale Injektion verabreicht. Das Körpergewicht aller Tiere wird vor Beginn der Studie, und anschließend täglich während der Studie bestimmt.

An Tag 21 wird eine Angiographie mittels einer Fluoreszenz-Funduskammera ( z.B. Kowe, HRA) durchgeführt. In Narkose und nach erneuter Pupillenerweiterung wird ein 10%iger Natrium-Fluorescein-Farbstoff subkutan (s.c.) injiziert. 2-10 min später werden Bilder des Augenhintergrundes aufgenommen. Die Stärke der Extravasation/des Ödems, dargestellt durch die Leckage von Fluorescein, wird von zwei bis drei verblindeten Beobachtern beurteilt und in Schweregrade von 0 (keine Extravasation) bis 3 (starke Einfärbung über die eigentliche Läsion hinaus) eingeteilt.

Nach Abtöten der Tiere an Tag 23 werden die Augen entnommen und in 4%iger Paraformaldehyd-Lösung für eine Stunde bei Raumtemperatur fixiert. Nach einem Waschdurchgang wird die Retina vorsichtig herausgeschält, und der Sklera-Choroidea-Komplex wird mit einem FITC-Isolektin B4 Antikörper angefärbt und anschließend flach auf einen Objetträger aufgebracht. Die so erhaltenen Präparate werden mittels eines Fluoreszenz-Mikroskops (Apotom, Zeiss) bei einer Anregungs-Wellenlänge von 488 nm ausgewertet. Die Fläche beziehungsweise das Volumen der choroidalen Neovaskularisierung (in µm² bzw. µm³) wird durch morphometrische Analyse mittels Axiovision 4.6 Software berechnet.

### c.2) Testung der Wirksamkeit von Substanzen im Sauerstoff-induzierten Retinopathie Modell

Es wurde gezeigt, dass eine durch Sauerstoff induzierte Retinopathie ein wertvolles Tiermodell für die Untersuchung der pathologischen retinalen Angiogenese darstellt. Dieses Modell basiert auf der Beobachtung, dass eine Hyperoxie während der frühen postnatalen Entwicklung in der Retina zum Anhalten oder zur Verlangsamung des Wachstums von normalen retinalen Blutgefäßen führt. Sobald die Tiere nach einer 7-tägigen Hyperoxiephase zur normoxischen Raumluft zurückkehren, ist dieses gleichbedeutend einer relativen Hypoxie, da in der Retina die normalen Gefäße fehlen, welche erforderlich sind, um eine ausreichende Versorgung des neuralen Gewebes unter normoxischen Bedingungen zu gewährleisten. Die dadurch erzeugte ischämische Situation führt zur abnormen Neovaskularisation, die einige Ähnlichkeiten mit der pathophysiologischen Neovaskularisation in Augenerkrankungen wie der feuchten AMD aufzeigt. Darüber hinaus ist die hervorgerufene Neovaskularisierung sehr reproduzierbar, quantifizierbar und ein wichtiger Parameter für die Untersuchung der Krankheitsmechanismen und möglichen Behandlungen für verschiedenste Formen von Netzhauterkrankungen.

Das Ziel dieser Studie ist es, die Wirksamkeit von täglich systemisch verabreichten Dosen der Testverbindung auf das Wachstum der retinalen Gefäße im Sauerstoff-induzierten Retinopathie-Modell zu untersuchen. Neugeborene von C57Bl / 6 Mäusen und ihre Mütter werden am postnatalen Tag 7 (PD7) einer Hyperoxie (70% Sauerstoff) für 5 Tage ausgesetzt. Ab PD12, werden die Mäuse unter normoxischen Bedingungen (Raumluft, 21% Sauerstoff) bis zum PD17 gehalten. Von Tag 12 bis Tag 17 werden die Mäuse täglich mit der Testsubstanz oder dem entsprechenden Vehikel behandelt. Am Tag 17 werden alle Mäuse mit Isofluran narkotisiert und anschließend durch Genickbruch abgetötet. Die Augen werden entnommen und in 4% Formalin fixiert. Nach dem Waschen in Phosphat-gepufferter Kochsalzlösung wird die Netzhaut präpariert, ein Flachpräperat davon erzeugt und dieses mit Isolectin B4 Antikörper gefärbt. Die Quantifizierung der neugewachsenen Gefäße wird unter Verwendung eines Zeiss ApoTome durchgeführt.

### C) Ausführungsbeispiele für pharmazeutische Zusammensetzungen

Die erfindungsgemäßen Substanzen können folgendermaßen in pharmazeutische Zubereitungen überführt werden:

### Tablette:

### Zusammensetzung:

100 mg der Verbindung des Beispiels 1, 50 mg Lactose (Monohydrat), 50 mg Maisstärke, 10 mg Polyvinylpyrolidon (PVP 25) (Fa. BASF, Deutschland) und 2 mg Magnesiumstearat.

Tablettengewicht 212 mg. Durchmesser 8 mm, Wölbungsradius 12 mm.

### Herstellung:

Die Mischung aus der Verbindung des Beispiels 1, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben).

### Orale Suspension:

### Zusammensetzung:

1000 mg der Verbindung des Beispiels 1, 1000 mg Ethanol (96%), 400 mg Rhodigel (Xanthan gum) (Fa. FMC, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die Verbindung des Beispiels 1 wird der Suspension zugefügt. Unter Rühren erfolgt die Zugabe des Wassers. Bis zum Abschluss der Quellung des Rhodigels wird ca. 6 h gerührt.

### Lösung oder Suspension zur topischen Anwendung am Auge (Augentropfen):

Eine sterile pharmazeutische Zubereitung zur topischen Anwendung am Auge kann durch Rekonstitution eines Lyophilisats der erfindungsgemäßen Verbindung in steriler Kochsalz-Lösung hergestellt werden. Als Konservierungsmittel für eine solche Lösung oder Suspension ist beispielsweise Benzalkoniumchlorid, Thiomersal oder Phenylquecksilbernitrat in einem Konzentrationsbereich von 0.001 bis 1 Gewichtsprozent geeignet.

### Lösung oder Suspension zur topischen Anwendung am Auge (Augentropfen):

Eine sterile pharmazeutische Zubereitung zur topischen Anwendung am Auge kann durch Rekonstitution eines Lyophilisats der erfindungsgemäßen Verbindung in steriler Kochsalz-Lösung hergestellt werden. Als Konservierungsmittel für eine solche Lösung oder Suspension ist beispielsweise Benzalkoniumchlorid, Thiomersal oder Phenylquecksilbernitrat in einem Konzentrationsbereich von 0.001 bis 1 Gewichtsprozent geeignet.

## Patentansprüche

1. Verbindung der Formel 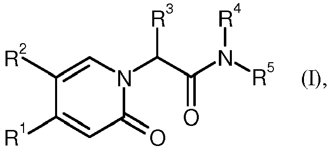 in welcher
R¹ für eine Gruppe der Formel 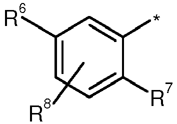 steht,
wobei * die Anknüpfstelle an den Oxopyridinring ist,
R⁶ für Brom, Chlor, Fluor, Methyl, Difluormethyl, Trifluormethyl, Methoxy, Difluormethoxy oder Trifluormethoxy steht,
R⁷ für Brom, Chlor, Fluor, Cyano, Nitro, Hydroxy, Methyl, Difluormethyl, Trifluormethyl, Methoxy, Ethoxy, Difluormethoxy, Trifluormethoxy, Ethinyl, 3,3,3-Trifluorprop-1-in-1-yl oder Cyclopropyl steht,
R⁸ für Wasserstoff, Chlor oder Fluor steht,
R² für Wasserstoff, Brom, Chlor, Fluor, Cyano, C₁-C₃-Alkyl, Difluormethyl, Trifluormethyl, 1,1-Difluorethyl, 2,2-Difluorethyl, 2,2,2-Trifluorethyl, C₁-C₃-Alkoxy, Difluormethoxy, Trifluormethoxy, 1,1-Difluorethoxy, 2,2-Difluorethoxy, 2,2,2-Trifluorethoxy, Methylcarbonyl oder Cyclopropyl steht,
R³ für Wasserstoff, C₁-C₅-Alkyl, C₁-C₄-Alkoxy, Difluormethyl, Trifluormethyl, 1,1-Difluorethyl, 1,1,2,2,2-Pentadeuteroethyl, 3,3,3-Trifluor-2-hydroxyprop-1-yl, 3,3,3-Trifluor-2-methoxyprop-1-yl, 3,3,3-Trifluor-2-ethoxyprop-1-yl, Prop-2-in-1-yl, Cyclopropyloxy oder Cyclobutyloxy steht,
wobei Alkyl substituiert sein kann mit einem Substituenten ausgewählt aus der Gruppe bestehend aus Fluor, Cyano, Hydroxy, Difluormethyl, Trifluormethyl, Methoxy, Ethoxy, Difluormethoxy, Trifluormethoxy, C₃-C₆-Cycloalkyl, 4- bis 6-gliedriges Oxo- Heterocyclyl, 1,4-Dioxanyl, Oxazolyl, Pyrazolyl, Phenyl und Pyridyl,
worin Cycloalkyl substituiert sein kann mit 1 bis 2 Substituenten unabhängig voneinander ausgewählt aus der Gruppe bestehend aus Fluor, Hydroxy, Methyl, Ethyl, Methoxy, Ethoxy, Difluormethyl, Trifluormethyl, Difluormethoxy und Trifluormethoxy,
und
worin Oxo-Heterocyclyl substituiert sein kann mit 1 bis 2 Substituenten unabhängig voneinander ausgewählt aus der Gruppe bestehend aus Oxo, Fluor, Methyl, Ethyl, Difluormethyl und Trifluormethyl,
und
worin Oxazolyl und Pyrazolyl substituiert sein können mit 1 bis 2 Substituenten unabhängig voneinander ausgewählt aus der Gruppe bestehend aus Methyl und Ethyl,
R⁴ für Wasserstoff steht,
R⁵ für eine Gruppe der Formel 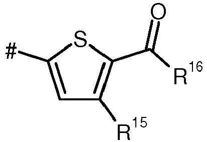 steht,
wobei # die Anknüpfstelle an das Stickstoffatom ist,
R¹⁵ für Wasserstoff oder Fluor steht,
R¹⁶ für Hydroxy oder -NHR¹⁷ steht,
worin
R¹⁷ für Wasserstoff oder C₁-C₄-Alkyl steht,
oder eines ihrer Salze, ihrer Solvate oder der Solvate ihrer Salze.

2. Verbindung nach Anspruch 1, **dadurch gekennzeichnet, dass**
R¹ für eine Gruppe der Formel 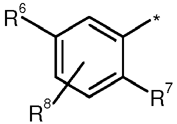 steht,
wobei * die Anknüpfstelle an den Oxopyridinring ist,
R⁶ für Chlor steht,
R⁷ für Fluor, Cyano, Difluormethyl oder Difluormethoxy steht,
R⁸ für Wasserstoff steht,
R² für Chlor, Cyano, Methoxy oder Difluormethoxy steht,
R³ für Methyl, Ethyl, n-Propyl oder n-Butyl steht,
wobei Methyl substituiert sein kann mit einem Substituenten ausgewählt aus der Gruppe bestehend aus Cyclopropyl, Cyclobutyl, Cyclohexyl, Tetrahydro-2H-pyranyl, Oxazolyl, Pyrazolyl und Pyridyl,
worin Cyclobutyl und Cyclohexyl substituiert sein können mit 1 bis 2 Substituenten unabhängig voneinander ausgewählt aus der Gruppe bestehend aus Hydroxy und Methoxy,
und
worin Oxazolyl und Pyrazolyl substituiert sein können mit einem Substituenten Methyl,
und
wobei Ethyl, n-Propyl und n-Butyl substituiert sein können mit einem Substituenten ausgewählt aus der Gruppe bestehend aus Methoxy und Trifluormethoxy,
R⁴ für Wasserstoff steht,
R⁵ für eine Gruppe der Formel 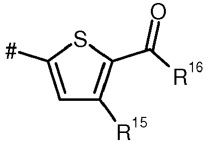 steht,
wobei # die Anknüpfstelle an das Stickstoffatom ist,
R¹⁵ für Wasserstoff oder Fluor steht,
R¹⁶ für Hydroxy oder -NHR¹⁷ steht,
worin
R¹⁷ für Wasserstoff, Methyl oder Ethyl steht,
oder eines ihrer Salze, ihrer Solvate oder der Solvate ihrer Salze.

3. Verbindung nach einem der Ansprüche 1 oder 2, **dadurch gekennzeichnet, dass**
R¹ für eine Gruppe der Formel 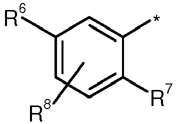 steht,
wobei * die Anknüpfstelle an den Oxopyridinring ist,
R⁶ für Chlor steht,
R⁷ für Cyano steht,
R⁸ für Wasserstoff steht,
R² für Chlor oder Methoxy steht,
R³ für Methyl oder Ethyl steht,
wobei Methyl substituiert ist mit einem Substituenten ausgewählt aus der Gruppe bestehend aus Tetrahydro-2H-pyranyl, Oxazolyl und Pyridyl,
worin Oxazolyl substituiert sein kann mit einem Substituenten Methyl,
und
wobei Ethyl substituiert sein kann mit einem Substituenten Methoxy,
R⁴ für Wasserstoff steht,
R⁵ für eine Gruppe der Formel 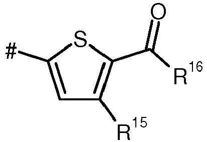 steht,
wobei # die Anknüpfstelle an das Stickstoffatom ist,
R¹⁵ für Wasserstoff oder Fluor steht,
R¹⁶ für Hydroxy oder -NHR¹⁷ steht,
worin
R¹⁷ für Wasserstoff oder Methyl steht,
oder eines ihrer Salze, ihrer Solvate oder der Solvate ihrer Salze.

4. Verfahren zur Herstellung einer Verbindung der Formel (I) oder eines ihrer Salze, ihrer Solvate oder der Solvate ihrer Salze nach Anspruch 1, **dadurch gekennzeichnet, dass** entweder
[A] eine Verbindung der Formel 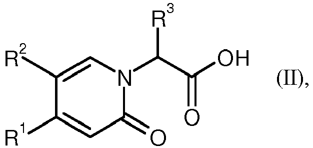 in welcher
R¹, R² und R³ die in Anspruch 1 angegebene Bedeutung haben,
in der ersten Stufe mit einer Verbindung der Formel 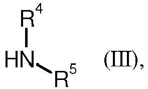 in welcher
R⁴ und R⁵ die in Anspruch 1 angegebene Bedeutung haben,
in Gegenwart eines Dehydratisierungsreagenzes umgesetzt wird, und
gegebenfalls in einer zweiten Stufe durch saure oder basische Esterspaltung zu einer Verbindung der Formel (I) umgesetzt wird,
oder
[B] eine Verbindung der Formel 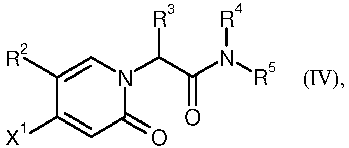 in welcher
R², R³, R⁴ und R⁵ die in Anspruch 1 angegebene Bedeutung haben, und
X¹ für Chlor, Brom oder Iod steht,
mit einer Verbindung der Formel 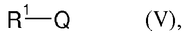 in welcher
R¹ die in Anspruch 1 angegebene Bedeutung hat, und
Q für -B(OH)₂, einen Boronsäure-Ester, bevorzugt Boronsäurepinakolester, oder-BF₃⁻K⁺ steht,
unter Suzuki-Kupplungsbedingungen zu einer Verbindung der Formel (I) umgesetzt wird.

5. Verbindung nach einem der Ansprüche 1 bis 3 zur Verwendung in der Behandlung und/oder Prophylaxe von Krankheiten.

6. Verwendung einer Verbindung nach einem der Ansprüche 1 bis 3 zur Herstellung eines Arzneimittels zur Behandlung und/oder Prophylaxe von Krankheiten.

7. Verwendung einer Verbindung nach einem der Ansprüche 1 bis 3 zur Herstellung eines Arzneimittels zur Behandlung und/oder Prophylaxe von thrombotischen beziehungsweise thromboembolischen Erkrankungen.

8. Verwendung einer Verbindung nach einem der Ansprüche 1 bis 3 zur Herstellung eines Arzneimittels zur Behandlung und/oder Prophylaxe von ophthalmologischen Erkrankungen.

9. Verwendung einer Verbindung nach einem der Ansprüche 1 bis 3 zur Herstellung eines Arzneimittels zur Behandlung und/oder Prophylaxe von hereditärem Angioödem oder entzündlichen Erkrankungen des Darms, wie Morbus Crohn oder Colitis ulcerosa.

10. Arzneimittel enthaltend eine Verbindung nach einem der Ansprüche 1 bis 3 in Kombination mit einem inerten, nichttoxischen, pharmazeutisch geeigneten Hilfsstoff.

11. Arzneimittel nach Anspruch 10 zur Behandlung und/oder Prophylaxe von thrombotischen beziehungsweise thromboembolischen Erkrankungen.

12. Arzneimittel nach Anspruch 10 zur Behandlung und/oder Prophylaxe von ophthalmologischen Erkrankungen.

13. Arzneimittel nach Anspruch 10 zur Behandlung und/oder Prophylaxe von hereditärem Angioödem oder entzündlichen Erkrankungen des Darms, wie Morbus Crohn oder Colitis ulcerosa.

## Claims

1. Compound of the formula 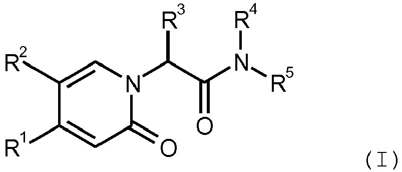 in which
R¹ is a group of the formula 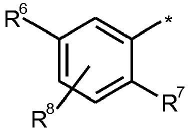 where * is the attachment point to the oxopyridine ring,
R⁶ is bromine, chlorine, fluorine, methyl, difluoromethyl, trifluoromethyl, methoxy, difluoromethoxy or trifluoromethoxy,
R⁷ is bromine, chlorine, fluorine, cyano, nitro, hydroxyl, methyl, difluoromethyl, trifluoromethyl, methoxy, ethoxy, difluoromethoxy, trifluoromethoxy, ethynyl, 3,3,3-trifluoroprop-1-yn-1-yl or cyclopropyl,
R⁸ is hydrogen, chlorine or fluorine,
R² is hydrogen, bromine, chlorine, fluorine, cyano, C₁-C₃-alkyl, difluoromethyl, trifluoromethyl, 1,1-difluoroethyl, 2,2-difluoroethyl, 2,2,2-trifluoroethyl, C₁-C₃-alkoxy, difluoromethoxy, trifluoromethoxy, 1,1-difluoroethoxy, 2,2-difluoroethoxy, 2,2,2-trifluoroethoxy, methylcarbonyl or cyclopropyl,
R³ is hydrogen, C₁-C₅-alkyl, C₁-C₄-alkoxy, difluoromethyl, trifluoromethyl, 1,1-difluoroethyl, 1,1,2,2,2-pentadeuteroethyl, 3,3,3-trifluoro-2-hydroxyprop-1-yl, 3,3,3-trifluoro-2-methoxyprop-1-yl, 3,3,3-trifluoro-2-ethoxyprop-1-yl, prop-2-yn-1-yl, cyclopropyloxy or cyclobutyloxy, where alkyl may be substituted by a substituent selected from the group consisting of fluorine, cyano, hydroxyl, difluoromethyl, trifluoromethyl, methoxy, ethoxy, difluoromethoxy, trifluoromethoxy, C₃-C₆-cycloalkyl, 4- to 6-membered oxoheterocyclyl, 1,4-dioxanyl, oxazolyl, pyrazolyl, phenyl and pyridyl,
in which cycloalkyl may be substituted by 1 to 2 substituents selected independently from the group consisting of fluorine, hydroxyl, methyl, ethyl, methoxy, ethoxy, difluoromethyl, trifluoromethyl, difluoromethoxy and trifluoromethoxy,
and
in which oxoheterocyclyl may be substituted by 1 to 2 substituents selected independently from the group consisting of oxo, fluorine, methyl, ethyl, difluoromethyl and trifluoromethyl,
and
in which oxazolyl and pyrazolyl may be substituted by 1 to 2 substituents selected independently from the group consisting of methyl and ethyl,
R⁴ is hydrogen,
R⁵ is a group of the formula 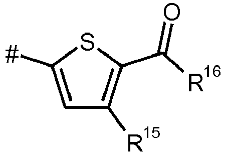 where # is the attachment point to the nitrogen atom,
R¹⁵ is hydrogen or fluorine,
R¹⁶ is hydroxyl or -NHR¹⁷,
in which
R¹⁷ is hydrogen or C₁-C₄-alkyl,
or one of the salts thereof, solvates thereof or solvates of the salts thereof.

2. Compound according to Claim 1, **characterized in that**
R¹ is a group of the formula 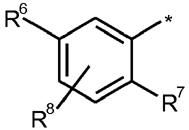 where * is the attachment point to the oxopyridine ring,
R⁶ is chlorine,
R⁷ is fluorine, cyano, difluoromethyl or difluoromethoxy,
R⁸ is hydrogen,
R² is chlorine, cyano, methoxy or difluoromethoxy,
R³ is methyl, ethyl, n-propyl or n-butyl, where methyl may be substituted by a substituent selected from the group consisting of cyclopropyl, cyclobutyl, cyclohexyl, tetrahydro-2H-pyranyl, oxazolyl, pyrazolyl and pyridyl,
in which cyclobutyl and cyclohexyl may be substituted by 1 to 2 substituents selected independently from the group consisting of hydroxyl and methoxy,
and
in which oxazolyl and pyrazolyl may be substituted by a methyl substituent,
and
where ethyl, n-propyl and n-butyl may be substituted by a substituent selected from the group consisting of methoxy and trifluoromethoxy,
R⁴
is hydrogen,
R⁵ is a group of the formula 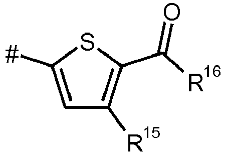 where # is the attachment point to the nitrogen atom,
R¹⁵ is hydrogen or fluorine,
R¹⁶ is hydroxyl or -NHR¹⁷,
in which
R¹⁷ is hydrogen, methyl or ethyl,
or one of the salts thereof, solvates thereof or solvates of the salts thereof.

3. Compound according to either of Claims 1 and 2,
**characterized in that**
R¹ is a group of the formula 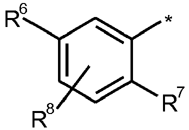 where * is the attachment point to the oxopyridine ring,
R⁶ is chlorine,
R⁷ is cyano,
R⁸ is hydrogen,
R² is chlorine or methoxy,
R³ is methyl or ethyl, where methyl is substituted by a substituent selected from the group consisting of tetrahydro-2H-pyranyl, oxazolyl and pyridyl,
in which oxazolyl may be substituted by a methyl substituent,
and
where ethyl may be substituted by a methoxy substituent,
R⁴ is hydrogen,
R⁵ is a group of the formula 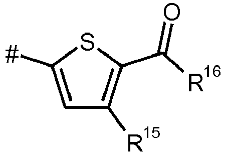 where # is the attachment point to the nitrogen atom,
R¹⁵ is hydrogen or fluorine,
R¹⁶ is hydroxyl or -NHR¹⁷,
in which
R¹⁷ is hydrogen or methyl,
or one of the salts thereof, solvates thereof or solvates of the salts thereof.

4. Process for preparing a compound of the formula (I) or one of the salts thereof, solvates thereof or solvates of the salts thereof according to Claim 1, **characterized in that** either
[A] a compound of the formula 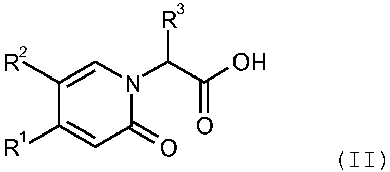 in which
R¹, R² and R³ have the definition given in Claim 1 is reacted in the first stage with a compound of the formula 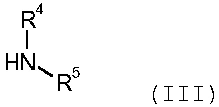 in which
R⁴ and R⁵ have the definition given in Claim 1 in the presence of a dehydrating reagent, and optionally converted in a second stage by acidic or basic ester hydrolysis to a compound of the formula (I),
or
[B] a compound of the formula 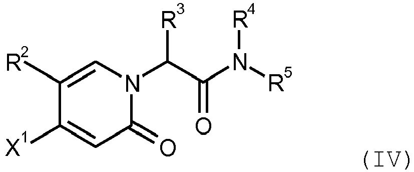 in which
R², R³, R⁴ and R⁵ have the definition given in Claim 1, and
X¹ is chlorine, bromine or iodine is reacted with a compound of the formula
R¹-Q (V)
in which
R¹ has the definition given in Claim 1, and
Q is -B(OH)₂, a boronic ester, preferably boronic acid pinacol ester, or -BF₃⁻K⁺,
under Suzuki coupling conditions to give a compound of the formula (I).

5. Compound according to any of Claims 1 to 3 for use in the treatment and/or prophylaxis of diseases.

6. Use of a compound according to any of Claims 1 to 3 for production of a medicament for treatment and/or prophylaxis of diseases.

7. Use of a compound according to any of Claims 1 to 3 for production of a medicament for treatment and/or prophylaxis of thrombotic or thromboembolic disorders.

8. Use of a compound according to any of Claims 1 to 3 for production of a medicament for treatment and/or prophylaxis of ophthalmic disorders.

9. Use of a compound according to any of Claims 1 to 3 for production of a medicament for treatment and/or prophylaxis of hereditary angiooedema or inflammatory disorders of the intestine, such as Crohn's disease or ulcerative colitis.

10. Medicament comprising a compound according to any of Claims 1 to 3 in combination with an inert, nontoxic, pharmaceutically suitable excipient.

11. Medicament according to Claim 10 for treatment and/or prophylaxis of thrombotic or thromboembolic disorders.

12. Medicament according to Claim 10 for treatment and/or prophylaxis of ophthalmic disorders.

13. Medicament according to Claim 10 for treatment and/or prophylaxis of hereditary angiooedema or inflammatory disorders of the intestine, such as Crohn's disease or ulcerative colitis.

## Revendications

1. Composé de formule 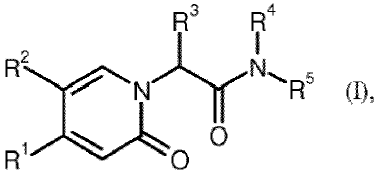 dans laquelle
R¹ représente un groupe de formule 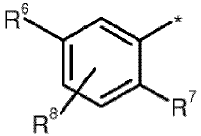 dans laquelle * est le point d'attachement au cycle oxopyridine,
R⁶ représente un atome de brome, chlore, fluor, un groupe méthyle, difluorométhyle, trifluorométhyle, méthoxy, difluorométhoxy ou trifluorométhoxy,
R⁷ représente un atome de brome, chlore, fluor, un groupe cyano, nitro, hydroxy, méthyle, difluorométhyle, trifluorométhyle, méthoxy, éthoxy, difluorométhoxy, trifluorométhoxy, éthynyle, 3,3,3-trifluoroprop-1-yn-1-yle ou cyclopropyle,
R⁸ représente un atome d'hydrogène, de chlore ou de fluor,
R² représente un atome d'hydrogène, de brome, chlore, fluor, un groupe cyano, alkyle en C₁-C₃, difluorométhyle, trifluorométhyle, 1,1-difluoro-éthyle, 2,2-difluoroéthyle, 2,2,2-trifluoroéthyle, alcoxy en C₁-C₃, difluorométhoxy, trifluorométhoxy, 1,1-difluoroéthoxy, 2,2-difluoroéthoxy, 2,2,2-trifluoroéthoxy, méthylcarbonyle ou cyclopropyle,
R³ représente un atome d'hydrogène, un groupe alkyle en C₁-C₅, alcoxy en C₁-C₄, difluorométhyle, trifluorométhyle, 1,1-difluoroéthyle, 1,1,2,2,2-pentadeuteroéthyle, 3,3,3-trifluoro-2-hydroxyprop-1-yle, 3,3,3-trifluoro-2-méthoxyprop-1-yle, 3,3,3-trifluoro-2-éthoxyprop-1-yle, prop-2-yn-1-yle, cyclopropyloxy ou cyclobutyloxy,
le groupe alkyle pouvant être substitué par un substituant choisi dans le groupe constitué par fluoro, cyano, hydroxy, difluorométhyle, trifluorométhyle, méthoxy, éthoxy, difluoro-méthoxy, trifluorométhoxy, cycloalkyle en C₃-C₆, oxo- hétérocyclyle à 4 à 6 chaînons, 1,4-dioxanyle, oxazolyle, pyrazolyle, phényle et pyridyle,
le groupe cycloalkyle pouvant être substitué par 1 ou 2 substituants choisis indépendamment l'un de l'autre dans le groupe constitué par fluoro, hydroxy, méthyle, éthyle, méthoxy, éthoxy, difluorométhyle, trifluorométhyle, difluorométhoxy et trifluorométhoxy,
et
le groupe oxo-hétérocyclyle pouvant être substitué par 1 ou 2 substituants choisis indépendamment l'un de l'autre dans le groupe constitué par oxo, fluoro, méthyle, éthyle, difluorométhyle et trifluorométhyle,
et
les groupes oxazolyle et pyrazolyle pouvant être substitués par 1 ou 2 substituants choisis indépendamment l'un de l'autre dans le groupe constitué par méthyle est éthyle,
R⁴ représente un atome d'hydrogène,
R⁵ représente un groupe de formule 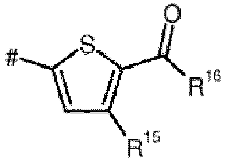 dans laquelle # est le point d'attachement à l'atome d'azote,
R¹⁵ représente un atome d'hydrogène ou de fluor,
R¹⁶ représente un groupe hydroxy ou -NHR¹⁷, où
R¹⁷ représente un atome d'hydrogène ou un groupe alkyle en C₁-C₄,
ou un de ses sels, de ses produits de solvatation ou des produits de solvatation de ses sels.

2. Composé selon la revendication 1, **caractérisé en ce que**
R¹ représente un groupe de formule 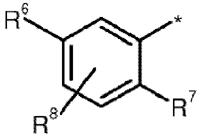
* le point d'attachement au cycle oxopyridine,
R⁶ représente un atome de chlore,
R⁷ représente un groupe fluoro, cyano, difluorométhyle ou difluorométhoxy,
R⁸ représente un atome d'hydrogène,
R² représente un atome de chlore, un groupe cyano, méthoxy ou difluorométhoxy,
R³ représente un groupe méthyle, éthyle, n-propyle ou n-butyle,
le groupe méthyle pouvant être substitué par un substituant choisi dans le groupe constitué par cyclopropyle, cyclobutyle, cyclohexyle, tétrahydro-2H-pyranyle, oxazolyle, pyrazolyle et pyridyle,
les groupes cyclobutyle et cyclohexyle pouvant être substitués par 1 ou 2 substituants choisis indépendamment l'un de l'autre dans le groupe constitué par hydroxy et méthoxy,
et
les groupes oxazolyle et pyrazolyle pouvant être substitués par un substituant méthyle,
et
les groupes éthyle, n-propyle et n-butyle pouvant être substitués par un substituant choisi dans le groupe constitué par méthoxy et trifluorométhoxy,
R⁴ représente un atome d'hydrogène,
R⁵ représente un groupe de formule 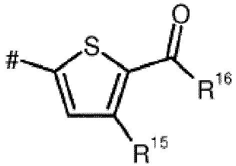 dans laquelle # est le point d'attachement à l'atome d'azote,
R¹⁵ représente un atome d'hydrogène ou de fluor,
R¹⁶ représente un groupe hydroxy ou -NHR¹⁷,
où
R¹⁷ représente un atome d'hydrogène ou un groupe méthyle ou éthyle,
ou un de ses sels, de ses produits de solvatation ou des produits de solvatation de ses sels.

3. Composé selon l'une quelconque des revendications 1 et 2, **caractérisé en ce que**
R¹ représente un groupe de formule 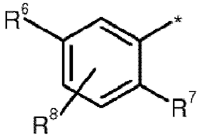 dans laquelle * est le point d'attachement au cycle oxopyridine,
R⁶ représente un atome de chlore,
R⁷ représente un groupe cyano,
R⁸ représente un atome d'hydrogène,
R² représente un atome de chlore ou un groupe méthoxy,
R³ représente un groupe méthyle ou éthyle,
le groupe méthyle étant substitué par un substituant choisi dans le groupe constitué par tétrahydro-2H-pyranyle, oxazolyle et pyridyle,
le groupe oxazolyle pouvant être substitué par un substituant méthyle,
et
le groupe éthyle pouvant être substitué par un substituant méthoxy,
R⁴ représente un atome d'hydrogène,
R⁵ représente un groupe de formule 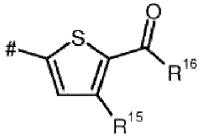 dans laquelle # est le point d'attachement à l'atome d'azote,
R¹⁵ représente un atome d'hydrogène ou de fluor,
R¹⁶ représente un groupe hydroxy ou -NHR¹⁷,
où
R¹⁷ représente un atome d'hydrogène ou un groupe méthyle,
ou un de ses sels, de ses produits de solvatation ou des produits de solvatation de ses sels.

4. Procédé pour la préparation d'un composé de formule (I) ou d'un de ses sels, de ses produits de solvatation ou des produits de solvatation de ses sels selon la revendication 1, **caractérisé en ce que** soit
[A] on fait réagir un composé de formule 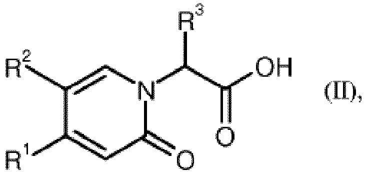 dans laquelle
R¹, R² et R³ ont la signification indiquée dans la revendication 1,
dans la première étape avec un composé de formule 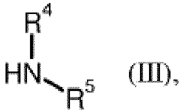 dans laquelle
R⁴ et R⁵ ont la signification indiquée dans la revendication 1,
en présence d'un réactif de déshydratation, et
éventuellement dans une deuxième étape on le convertit en un composé de formule (I) par coupure acide ou basique d'ester,
ou
[B] on fait réagir un composé de formule 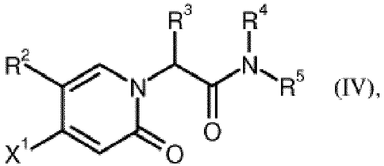 dans laquelle
R², R³, R⁴ et R⁵ ont la signification indiquée dans la revendication 1 et
X¹ représente un atome de chlore, de brome ou d'iode,
avec un composé de formule
R¹-Q (V)
dans laquelle
R¹ a la signification indiquée dans la revendication 1, et
Q représente -B(OH)₂, un ester d'acide boronique, de préférence le boronate de pinacol, ou -BF₃⁻K⁺,
dans des conditions de couplage de Suzuki, pour aboutir à un composé de formule (I).

5. Composé selon l'une quelconque des revendications 1 à 3, destiné à l'utilisation dans le traitement et/ou la prophylaxie de maladies.

6. Utilisation d'un composé selon l'une quelconque des revendications 1 à 3, pour la fabrication d'un médicament destiné au traitement et/ou à la prophylaxie de maladies.

7. Utilisation d'un composé selon l'une quelconque des revendications 1 à 3, pour la fabrication d'un médicament destiné au traitement et/ou à la prophylaxie de maladies thrombotiques ou thromboemboliques.

8. Utilisation d'un composé selon l'une quelconque des revendications 1 à 3, pour la fabrication d'un médicament destiné au traitement et/ou à la prophylaxie de maladies ophtalmologiques.

9. Utilisation d'un composé selon l'une quelconque des revendications 1 à 3, pour la fabrication d'un médicament destiné au traitement et/ou à la prophylaxie d'angio-oedèmes héréditaires ou de maladies inflammatoires de l'intestin, telles que la maladie de Crohn ou la colite ulcéreuse.

10. Médicament contenant un composé selon l'une quelconque des revendications 1 à 3 en association avec un adjuvant inerte, non toxique, pharmaceutiquement convenable.

11. Médicament selon la revendication 10, destiné au traitement et/ou à la prophylaxie de maladies thrombotiques ou thromboemboliques.

12. Médicament selon la revendication 10, destiné au traitement et/ou à la prophylaxie de maladies ophtalmologiques.

13. Médicament selon la revendication 10, destiné au traitement et/ou à la prophylaxie d'angio-oedémes héréditaires ou de maladies inflammatoires de l'intestin, telles que la maladie de Crohn ou la colite ulcéreuse.
